# EUROPEAN PATENT SPECIFICATION

(11) **EP 4 058 035 B1**
(45) Date of publication and mention of the grant of the patent: **23.09.2026**
(21) Application number: 20888482.5
(22) Date of filing: 13.11.2020
(51) Int. Cl.: A61K 35/17, C12N 15/86, C07K 14/705, C07K 14/485, A61K 48/00, A61K 45/06, A61K 38/20, A61K 38/17, A61K 40/11, A61K 40/32, A61K 40/36, A61K 40/42, C07K 14/725, C12N 5/0783, A61P 37/02, A61P 35/00

(54) **COMPOSITIONS AND METHODS FOR IMMUNOTHERAPY**
ZUSAMMENSETZUNGEN UND VERFAHREN FÜR DIE IMMUNTHERAPIE
COMPOSITIONS ET PROCÉDÉS D'IMMUNOTHÉRAPIE

(30) Priority: 14.11.2019 US 201962935308 P
(43) Date of publication of application: 21.09.2022
(73) Proprietor: Ludwig Institute for Cancer Research Ltd, 8001 Zürich (CH)
(72) Inventor: COUKOS, George, 1066 Epalinges (CH); IRVING, Melita, 1066 Epalinges (CH); OSORIO, Angel, De Jesus Corria, 1066 Epalinges (CH); ZOETE, Vincent, 1066 Epalinges (CH)
(74) Representative: HGF
(86) International application number: PCT/US2020/060505
(87) International publication number: WO 2021/097278

(56) References cited:
- WO-A1-2006/086823
- WO-A1-2017/120997
- WO-A1-2018/096361
- WO-A1-2018/170390
- WO-A1-2018/183921
- WO-A1-2019/070755
- WO-A1-2021/097278
- WO-A1-2022/104043
- LANITIS EVRIPIDIS ET AL: "All systems go: converging synthetic biology and combinatorial treatment for CAR-T cell therapy", CURRENT OPINION IN BIOTECHNOLOGY, LONDON, GB, vol. 65, 25 February 2020 (2020-02-25), pages 75 - 87, XP086318305, ISSN: 0958-1669, [retrieved on 20200225], DOI: 10.1016/J.COPBIO.2020.01.009
- TANG X ET AL: "The advantages of PD1 activating chimeric receptor (PD1-ACR) engineered lymphocytes for PDL1(+) cancer therapy", vol. 7, no. 3, 15 March 2015 (2015-03-15), pages 460 - 473, XP002743491, ISSN: 1943-8141, Retrieved from the Internet <URL:www.ajtr.org /ISSN:1943-8141/AJTR0002962> [retrieved on 20150315]
- HUANG BAOZHU ET AL: "B7-H3 specific T cells with chimeric antigen receptor and decoy PD-1 receptors eradicate established solid human tumors in mouse models", vol. 9, no. 1, 1 January 2020 (2020-01-01), XP055895680, Retrieved from the Internet <URL:https://www.ncbi.nlm.nih.gov/pmc/articles/PMC6959446/pdf/koni-09-01-1684127.pdf> DOI: 10.1080/2162402X.2019.1684127
- STROHL ET AL: "Bispecific T-Cell Redirection versus Chimeric Antigen Receptor (CAR)-T Cells as Approaches to Kill Cancer Cells", vol. 8, no. 3, 1 January 2019 (2019-01-01), pages 41, XP009516302, ISSN: 2073-4468, Retrieved from the Internet <URL:https://www.ncbi.nlm.nih.gov/pmc/articles/doi/10.3390/antib8030041> [retrieved on 20190703], DOI: 10.3390/ANTIB8030041
- BIANCA HEEMSKERK ET AL: "Adoptive Cell Therapy for Patients with Melanoma, Using Tumor-Infiltrating Lymphocytes Genetically Engineered to Secrete Interleukin-2", HUMAN GENE THERAPY, vol. 19, no. 5, 1 May 2008 (2008-05-01), GB, pages 496 - 510, XP055528657, ISSN: 1043-0342, DOI: 10.1089/hum.2007.0171
- BIAGI ETTORE ET AL: "Immunotherapy of Chronic Lymphocytic Leukemia using CD40L and IL2 Expressing Autologous Tumor Cells", BLOOD, AMERICAN SOCIETY OF HEMATOLOGY, US, vol. 104, no. 11, 16 November 2004 (2004-11-16), pages 768, XP086655600, ISSN: 0006-4971, DOI: 10.1182/BLOOD.V104.11.768.768
- TAKAHASHI SATOSHI ET AL: "Autologous Antileukemic Immune Response Induced by Chronic Lymphocytic Leukemia B Cells Expressing the CD40 Ligand and Interleukin 2 Transgenes", HUMAN GENE THERAPY, vol. 12, no. 6, 10 April 2001 (2001-04-10), GB, pages 659 - 670, XP055972032, ISSN: 1043-0342, DOI: 10.1089/104303401300057360
- ROUSSEAU RAPHAËL F. ET AL: "Immunotherapy of high-risk acute leukemia with a recipient (autologous) vaccine expressing transgenic human CD40L and IL-2 after chemotherapy and allogeneic stem cell transplantation", vol. 107, no. 4, 15 February 2006 (2006-02-15), US, pages 1332 - 1341, XP055972037, ISSN: 0006-4971, Retrieved from the Internet <URL:http://ashpublications.org/blood/article-pdf/107/4/1332/468986/zh800406001332.pdf> DOI: 10.1182/blood-2005-03-1259
- KUHN NICHOLAS F ET AL: "CD40 Ligand-Modified Chimeric Antigen Receptor T Cells Enhance Antitumor Function by Eliciting an Endogenous Antitumor Response", CANCER CELL, vol. 35, no. 3, 2019, pages 473, XP085638453, ISSN: 1535-6108, DOI: 10.1016/J.CCELL.2019.02.006
- LIEBERMAN J ET AL: "Avoiding the kiss of death: how HIV and other chronic viruses survive", CURRENT OPINION IN IMMUNOLOGY, ELSEVIER, OXFORD, GB, vol. 14, no. 4, 1 August 2002 (2002-08-01), pages 478 - 486, XP004367715, ISSN: 0952-7915, DOI: 10.1016/S0952-7915(02)00366-7
- COUKOS: "International Conference on Lymphocyte Engineering 31 March-2 April 2022 Munich, Germany", HUMAN GENE THERAPY, 1 April 2022 (2022-04-01), pages A - 1, XP093072859, Retrieved from the Internet <URL:https://www.liebertpub.com/doi/10.1089/hum.2022.29200.abstracts> [retrieved on 20230811], DOI: 10.1089/hum.2022.29200.abstracts

## Description

### FIELD OF THE INVENTION

The present invention relates generally to compositions and their use for treating cancer or a tumor in a subject and more specifically to compositions and their use for treating cancer or a tumor in a subject by modulating the immune system of the subject.

### BACKGROUND OF THE INVENTION

Adoptive cell transfer or adoptive cell therapy (ACT) represents a promising therapeutic approach for the treatment of cancer patients. However, it faces two major obstacles: the short-term survival of the transferred cells in the cancer patients and the hostile immunosuppressive tumor microenvironment.

To overcome these limitations, several options have been proposed. For example, some trials tested the administration of interleukin 2 (IL-2) concurrently with the ACT. IL-2 is a potent immunostimulant; therefore it boosts the immune response and increases the survival of the transferred cells. However, this approach was unsuccessful due to the toxicities associated with IL-2. US Patent 7,381,405 describes methods for preparing IL-2-transduced lymphocytes for ACT that secrete IL-2. This approach is based on the hypothesis that the lymphocytes will secrete their own growth factor (*e.g.,* IL-2) and thus depend less on other exogenous factors for survival *in vivo.* Despite the successful results in *in vitro* settings, clinical trials determined that this approach was ineffective. IL-2-transduced lymphocytes were not more effective than non-transduced lymphocytes in treating cancer (Heemskerk et al., Human Gene Therapy, 2008).

The advent of chimeric antigen receptor (CAR) T cells has provided a useful tool to improve ACT. TRUCKs (International Publication WO 2017/108805) and Armored CARs (US Patent 10,124,023) are representative examples of CAR T cells that have been further engineered for the secretion of a recombinant interleukin-12 (IL-12) and CD40L, respectively. However, these strategies have disadvantages. For example, in TRUCKs, high transgenic IL-12 production limited T cell expansion and increased apoptosis, showing limited therapeutic efficacy. Also, the clinical application of armored CAR T cells has been limited to liquid tumors so far.

The solid tumors and their microenvironment have given a series of challenges for the success of ACT therapy. These challenges include efficient trafficking and infiltration of the tumor, as well as overcoming tumor-mediated immunosuppression. Despite numerous efforts, the state-of-the-art ACT therapies do not provide functional persistence within the immunosuppressive solid tumor microenvironment for long-term efficacy.

Therefore, there is a pressing need for identifying novel ACT therapies that provide cells with functional persistence and/or that can change the cytokine milieu to overcome the immunosuppressive tumor microenvironment.

WO 2017/120997 A1 discloses transgenic lymphocytes co-expressing EGFRVIII chimeric antigen receptor and non-functional EGFR and uses of the lymphocytes.

Lanitis Evripidis et al., (Curr Opin Biotechnol. 2020 Oct:65:75-87) discloses a study on converging synthetic biology and combinatorial treatment for CAR-T cell therapy.

WO 2018/096361 A1 discloses a signal transduction modifying protein which comprises a domain which binds a phosphorylated immunoreceptor tyrosine-based inhibition motif (pITIM).

Tang X et al.,( Am J Transl Res. 2015 Mar 15;7(3):460-73) discloses the advantages of PD1 activating chimeric receptor (PD1-ACR) engineered lymphocytes for PDL1(+) cancer therapy.

Huang Baozhu et al., (Oncoimmunology. 2019 Nov 4;9(1):1684127) discloses that B7-H3 specific T cells with chimeric antigen receptor and decoy PD-1 receptors eradicate established solid human tumors in mouse model.

Strohl et al., (Antibodies (Basel). 2019 Jul 3;8(3):41) discloses a review on bispecific T-Cell redirection versus Chimeric Antigen Receptor (CAR)-T cells as approaches to kill cancer cells.

Bianca Heemskerk et al., (Hum Gene Ther. 2008 May;19(5):496-510) discloses a study on adoptive cell therapy for patients with melanoma, using tumor-infiltrating lymphocytes genetically engineered to secrete interleukin-2.

WO 2018/170390 A1 discloses methods and compositions for dynamically controlling and targeting multiple arms of the immune system. Some aspects provide mesenchymal stem cells (MSCs) engineered to produce multiple effector molecules.

WO 2021/097278 A1 discloses methods and compositions to confer and/or increase immune responses mediated by cellular immunotherapy, such as by adoptively transferring tumor-specific genetically-modified subsets of lymphocytes.

WO 2022/104043 A1 discloses PD-1 decoy variants, compositions, and methods to confer and/or increase immune responses mediated by cellular immunotherapy, such as by adoptively transferring tumor-specific genetically-modified subsets of lymphocytes.

Biagi Ettore et al., (Blood (2004) 104 (11): 768) discloses an immunotherapy of chronic lymphocytic leukemia using CD40L and IL2 expressing autologous tumor cells.

Takahashi Satoshi et al., (Hum Gene Ther. 2001 Apr 10;12(6):659-70) discloses a study on autologous antileukemic immune response induced by chronic lymphocytic leukemia B cells expressing the CD40 ligand and interleukin 2 transgenes.

Rousseau Raphael F. et al., (Blood. 2006 Feb 15;107(4):1332-41) discloses an immunotherapy of high-risk acute leukemia with a recipient (autologous) vaccine expressing transgenic human CD40L and IL-2 after chemotherapy and allogeneic stem cell transplantation.

Kuhn Nicholas F. et al., (Cancer Cell. 2019 Mar 18;35(3):473-488.e6) discloses that CD40 ligand-modified chimeric antigen receptor T cells enhances antitumor function by eliciting an endogenous antitumor response.

Lieberman J. et al., (Curr Opin Immunol. 2002 Aug;14(4):478-86) discloses how HIV and other chronic viruses survive.

Coukos et al., discloses in the International Conference on Lymphocyte Engineering 31 March-2 April 2022 Munich, Germany (Human Gene Therapy, 1 April 2022) that orthogonal gene engineering enables CD8+ T cells to control tumors through a novel tox-indifferent synthetic effector state.

WO 2018/183921 A1 and WO 2019/070755 A1 disclose methods and compositions for detecting and modulating an immunotherapy resistance gene signature in cancer.

### SUMMARY OF THE INVENTION

The invention is defined by the claims. Further aspects and preferred embodiments are defined in the dependent claims. Any aspects, embodiments and examples of the present disclosure which do not fall under the scope of the appended claims do not form part of the invention and are merely provided for illustrative purposes. Any references in the description to methods of treatments refer to the compositions of the present invention for use in a method for treatment of the human (or animal) body by therapy (or for diagnosis).

Accordingly, the present invention relates to a composition comprising a plurality of genetically-modified lymphocytes for targeting tumor cells in a subject, wherein the plurality of lymphocytes express at least three transgenes for modulating the immune system of the subject, and wherein the at least three transgenes comprise a PD-1-Fc decoy, an IL-2 variant having an amino acid sequence of any one of SEQ ID NOs: 21-23, and a CD40L having an amino acid of any one of SEQ ID NOs: 32-34, 36, and 38.

The present invention further relates to a pharmaceutical composition comprising an effective amount of the composition of the invention and a pharmaceutically acceptable carrier.

The present invention also relates to a composition according to the invention for use in treating a cancer/tumor or chronic infection in a subject, preferably wherein the cancer is selected from the group consisting of melanoma, sarcoma, ovarian cancer, prostate cancer, lung cancer, bladder cancer, MSI-high tumors, head and neck tumors, kidney cancer, and breast cancer, preferably wherein the composition is administered by intravenous infusion, preferably wherein the composition further comprises a second therapeutic agent, and preferably wherein the second therapeutic agent is an anti-cancer or anti-tumor agent.

This disclosure addresses the need mentioned above in a number of aspects. Disclosed herein is a composition comprising a plurality of genetically-modified lymphocytes expressing at least two transgenes (*e.g*., therapeutic transgenes) for modulating the immune system of a subject.

Transgenes may be selected from the group consisting of antibodies, antibody fragments, receptors, decoys, checkpoint blockade modulators, cytokines, chemokines, hormones, cellular elimination tags, and combinations thereof.

The decoy may be selected from the group consisting of PD1, CTLA4, LAG3, VEGFR1, TIM3, TIGIT, and SIRPalpha decoy. The decoy may be a PD1 decoy. The PD-1 decoy may be a PD-1.IgG4 (*e.g*., PD-1.IgG4Fc) decoy.

The cytokine may be selected from the group consisting of LIGHT or a variant/fragment thereof, IL-33 or a variant/fragment thereof, IL-2 or a variant/fragment thereof, IL-15 or a variant/fragment thereof, IL-12 or a variant/fragment thereof, and CD40L or a variant/fragment thereof. The cytokine may be a mutant cytokine.

In some embodiments, the cellular elimination tag is selected from the group consisting of tEGFR, Her2, CD20, and CD19.

The at least two transgenes may comprise two or more of a PD-1 decoy or a variant/fragment thereof, an IL-2 variant/fragment, LIGHT or a variant/fragment thereof, IL-33 or a variant/fragment thereof, and CD40L or a variant/fragment thereof. The at least two transgenes may further comprise a truncated EGFR (tEGFR) or a variant/fragment thereof, a truncated HER2 (tHER2) or a variant/fragment thereof, or CD20 or a variant/fragment thereof. The PD-1 decoy or a variant/fragment thereof and the tEGFR or the variant/fragment thereof (or the tHER2 or a variant/fragment thereof, CD20 or a variant/fragment thereof, CD19 or a variant/fragment thereof) may be harbored on the same vector.

The at least two transgenes may comprise: (a) the PD-1 decoy or the variant thereof and tEGFR or the variant thereof; (b) the PD-1 decoy or the variant thereof and the IL-2 variant; (c) the PD-1 decoy or the variant thereof and the LIGHT or the variant thereof; (d) the PD-1 decoy or the variant thereof and the IL-33 or the variant thereof; (e) the PD-1 decoy or the variant thereof and the CD40L or the variant thereof; (f) the PD-1 decoy or the variant thereof, the IL-2 variant, and the IL-33 or the variant thereof; (g) the PD-1 decoy or the variant thereof, the tEGFR or the variant thereof, and the IL-2 variant; (h) the PD-1 decoy or the variant thereof, the tEGFR or the variant thereof, and the LIGHT or the variant thereof; (i) the PD-1 decoy or the variant thereof, the tEGFR or the variant thereof, and the IL-33 or the variant thereof; (j) the PD-1 decoy or the variant thereof, the tEGFR or the variant thereof, and the CD40L or the variant thereof; (k) the PD-1 decoy or the variant thereof, the tEGFR or the variant thereof, the IL-2 variant, and the IL-33 or the variant thereof; (l) the PD-1 decoy or the variant thereof, the tEGFR or the variant thereof, the IL-2 variant, and the CD40L or the variant thereof; or (m) the PD-1 decoy or the variant thereof, the tEGFR or the variant thereof, the IL-33variant and the CD40L or the variant thereof.

The PD-1 decoy may comprise an amino acid sequence of any one of SEQ ID NOs: 1-4, 6-17, 42, 44, 47-48, and 51-52 or an amino acid sequence having at least 80% identity to any one of SEQ ID NOs: 1-4, 6-17, 42, 44, 47-48, and 51-52.

The IL-2 variant may comprise an amino acid sequence of any one of SEQ ID NOs: 21-23 or an amino acid sequence having at least 80% identity to any one of SEQ ID NOs: 21-23.

The IL-33 may comprise an amino acid sequence of any one of SEQ ID NOs: 25 and 27 or an amino acid sequence having at least 80% identity to any one of SEQ ID NOs: 25 and 27.

The LIGHT may comprise an amino acid sequence of any one of SEQ ID NOs: 28-29 and 31 or an amino acid sequence having at least 80% identity to any one of SEQ ID NOs: 28-29 and 31.

The CD40L may comprise an amino acid sequence of any one of SEQ ID NOs: SEQ ID NOs: 32-34, 36, and 38 or an amino acid sequence having at least 80% identity to any one of SEQ ID NOs: SEQ ID NOs: 32-34, 36, and 38.

The tEGFR may comprise an amino acid sequence having at least 80% identity to SEQ ID NO: 40 or the amino acid sequence of SEQ ID NO: 40. The HER2 may comprise an amino acid sequence having at least 80% identity to SEQ ID NO: 45 or the amino acid sequence of SEQ ID NO: 45. The CD20 may comprise an amino acid sequence having at least 80% identity to SEQ ID NO: 49 or the amino acid sequence of SEQ ID NO: 49.

The transgenes may comprise the antibodies or antibody fragments that are selected from the group consisting of VEGF, TGF-B, 4-1BB, CD28, CD27, NKG2D, PD1, PDL1, and CTLA4 antibodies. The antibody may be a PD1 antibody.

The plurality of lymphocytes may comprise at least two subsets of lymphocytes. The plurality of lymphocytes may consist of two subsets of lymphocytes. Each subset of the plurality of lymphocytes may express at least one transgene. The at least two transgenes may different from each other.

In some embodiments, the plurality of lymphocytes comprises: (i) a first subset expressing at least two transgenes; and (ii) a second subset expressing at least two transgenes, wherein at least one of the transgenes of the first subset is different from the transgenes of the second subset or wherein at least one of the transgenes of the first subset is in common with the transgenes of the second subset.

As disclosed herein, (i) the first subset may express at least a PD-1 decoy or a variant thereof and an IL-2 variant and the second subset may express at least a PD-1 decoy or a variant thereof and LIGHT or a variant thereof; (ii) the first subset may express at least a PD-1 decoy or a variant thereof and an IL-2 variant and the second subset may express at least a PD-1 decoy or a variant thereof and IL-33 or a variant thereof; (iii) the first subset may express at least a PD-1 decoy or a variant thereof and an IL-2 variant and the second subset may express at least a PD-1 decoy or a variant thereof and CD40L or a variant thereof; (iv) the first subset may express at least a PD-1 decoy or a variant thereof and LIGHT or a variant thereof and the second subset may express at least a PD-1 decoy or a variant thereof and IL-33 or a variant thereof; or (v) the first subset may express at least a PD-1 decoy or a variant thereof and LIGHT or a variant thereof and the second subset may express at least a PD-1 decoy or a variant thereof and CD40L or a variant thereof; or (vi) the first subset may express at least a PD-1 decoy or a variant thereof and IL-33 or a variant thereof and the second subset may express at least a PD-1 decoy or a variant thereof and CD40L or a variant thereof.

In some embodiments, the first subset or the second subset further expresses tEGFR or a variant thereof, a truncated HER2 (tHER2) or a variant thereof, CD20 or a variant thereof, or CD19 or a variant thereof.

As disclosed herein, (i) the first subset may express at least the PD-1 decoy or the variant thereof, tEGFR or the variant thereof and an IL-2 variant and the second subset may express at least the PD-1 decoy or the variant thereof, tEGFR or the variant thereof and LIGHT or the variant thereof; (ii) the first subset may express at least the PD-1 decoy or the variant thereof, tEGFR or the variant thereof and an IL-2 variant and the second subset may express at least the PD-1 decoy or the variant thereof, tEGFR or the variant thereof and IL-33 or the variant thereof; (iii) the first subset may express at least the PD-1 decoy or the variant thereof, tEGFR or the variant thereof and an IL-2 variant and the second subset may express at least the PD-1 decoy or the variant thereof, tEGFR or the variant thereof and CD40L or the variant thereof; (iv) the first subset may express at least the PD-1 decoy or the variant thereof, tEGFR or the variant thereof and LIGHT or the variant thereof and the second subset may express at least the PD-1 decoy or the variant thereof, tEGFR or the variant thereof and IL-33 or the variant thereof; (v) the first subset may express at least the PD-1 decoy or the variant thereof, tEGFR or the variant thereof and LIGHT or the variant thereof and the second subset may express at least the PD-1 decoy or the variant thereof, tEGFR or the variant thereof and CD40L or the variant thereof; or (vi) the first subset may express at least the PD-1 decoy or the variant thereof, tEGFR or the variant thereof and IL-33 or the variant thereof and the second subset may express at least the PD-1 decoy or the variant thereof, tEGFR or the variant thereof and CD40L or the variant thereof.

In some embodiments, the first subset or the second subset further expresses tEGFR or a variant thereof, tHER2 or a variant thereof, or CD20 or a variant thereof.

In some embodiments, the two subsets are combined at a ratio from about 1:1 to about 1:100. In some embodiments, the two subsets are combined at the ratio of about 1:1.

In some embodiments, the lymphocytes are autologous. In some embodiments, the lymphocytes are tumor-infiltrating lymphocytes. In some embodiments, the lymphocytes express a chimer antigen receptor (CAR). In some embodiments, the lymphocytes express a recombinant T cell receptor (TCR). In some embodiments, the recombinant T cell receptor (TCR) shows reactivity against NY-ESO1, MAGE-A1, MAGE-A3, MAGE A-10, MAGE-C2, SSX2, MAGE-A12, or a combination thereof.

Also within the scope of this disclosure is a pharmaceutical composition comprising an effective amount of a composition as described above and a pharmaceutically acceptable carrier. In some embodiments, the pharmaceutical composition further comprises a second therapeutic agent.

Additionally provided in this disclosure is a kit comprising an effective amount of a composition as described above.

Disclosed herein is a method of preparing a composition as described above. The method comprises: (a) providing a plurality of lymphocytes; (b) introducing to the plurality of lymphocytes a nucleic acid molecule encoding at least two transgenes to obtain a plurality of genetically-modified lymphocytes; and (c) expanding the plurality of genetically-modified in a cell culture medium.

Alternatively, the method comprises: (a) providing a plurality of lymphocytes; (b) introducing to the plurality of lymphocytes two or more nucleic acid molecules, each of the two or more nucleic acid molecules encoding at least one transgene, thereby obtaining a plurality of genetically-modified lymphocytes; and (c) expanding the plurality of genetically-modified in a cell culture medium.

The at least two transgenes may comprise two or more of a PD-1 decoy, an IL-2 variant/fragment, LIGHT or a variant/fragment thereof, IL-33 or a variant/fragment thereof, and CD40L or a variant/fragment thereof. The at least two transgenes may further comprise tEGFR or a variant/fragment thereof. The PD-1 decoy or the variant/fragment thereof and the tEGFR or the variant/fragment thereof (or the tHER2 or a variant/fragment thereof, CD20 or a variant/fragment thereof, or CD19 or a variant/fragment thereof) may be harbored on the same vector.

The method may comprise: (a) introducing to a first plurality of lymphocytes a first nucleic acid molecule encoding at least two transgenes to obtain a first plurality of genetically-modified lymphocytes; and (b) introducing to a second plurality of lymphocytes a second nucleic acid molecule encoding at least two transgenes to obtain a second plurality of genetically-modified lymphocytes.

The method may further comprise expanding the first plurality of lymphocytes in a cell culture medium following the step of introducing the first nucleic acid or expanding the second plurality of lymphocytes in a cell culture medium following the step of introducing the second nucleic acid.

The method may further comprise combining the first plurality of genetically-modified lymphocytes with the first plurality of genetically-modified lymphocytes at a predetermined ratio between about 1:1 and about 1:100 (*e.g.,* 1:1).

The cell culture medium may be a defined cell culture medium. The cell culture medium may comprise neoantigen peptides.

In yet another aspect, this disclosure further provides a method of treating a cancer/tumor or chronic infection in a subject. The method comprises administering to a subject in need thereof a therapeutically effective amount of a composition or a pharmaceutical composition, as described above.

In some embodiments, the cancer is selected from the group consisting of melanoma, sarcoma, ovarian cancer, prostate cancer, lung cancer, bladder cancer, MSI-high tumors, head and neck tumors, kidney cancer, and breast cancer.

In some embodiments, the composition is administered by intravenous infusion. In some embodiments, the method further comprises administering to the subject a second therapeutic agent. In some embodiments, the second therapeutic agent is an anti-cancer or anti-tumor agent. In some embodiments, the composition or the pharmaceutical composition is administered to the subject before, after, or concurrently with the second therapeutic agent.

The foregoing summary is not intended to define every aspect of the disclosure, and additional aspects are described in other sections, such as the following detailed description. Other features and advantages of the invention will become apparent from the following detailed description..

### BRIEF DESCRIPTION OF THE DRAWINGS

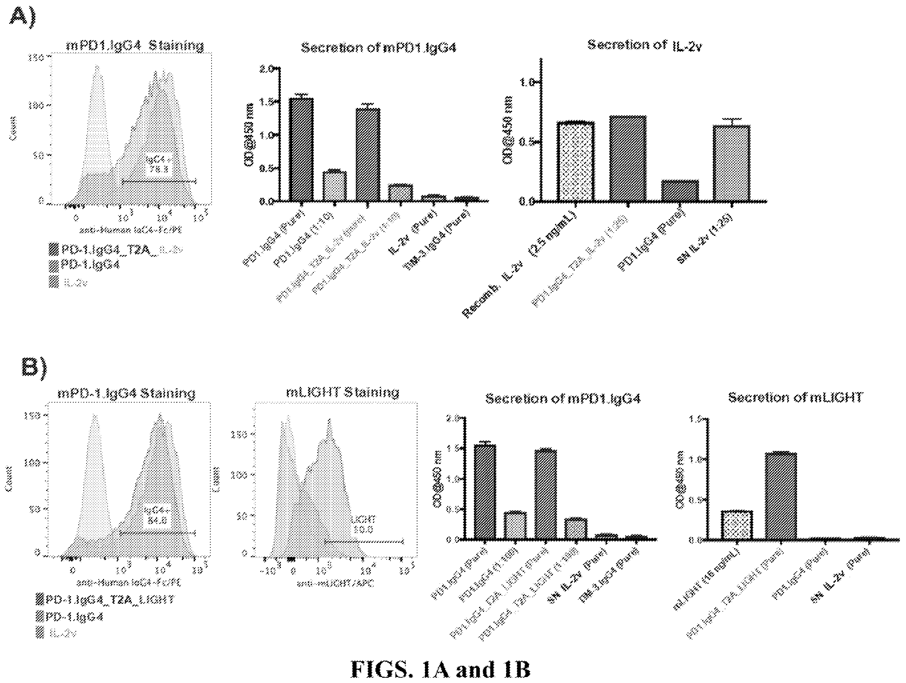

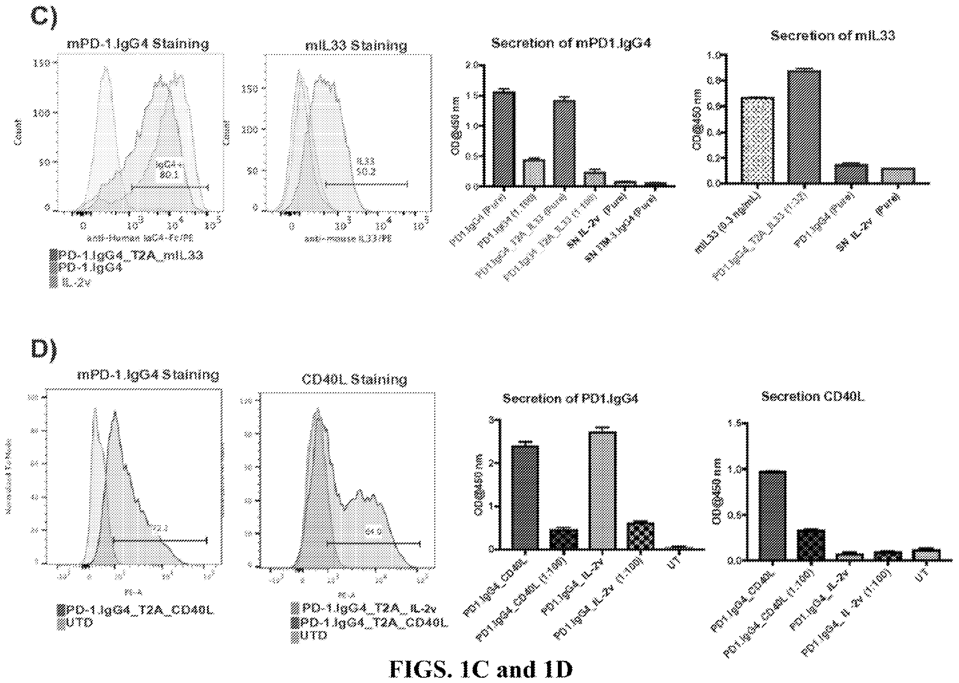

FIGS. 1A, 1B, 1C, and 1D (collectively "FIG. 1") are a set of diagrams showing that OT-1 CD8⁺-T cells can be gene-engineered to secrete PD1.IgG4 decoy in combination with either a IL-2 variant (referred to as IL-2^{V}), LIGHT, IL-33, or CD40L. FIG. 1A shows that OT-1 CD8⁺-T cells were genetically engineered to secrete both PD1.IgG4 and mutIL2. Transduction efficiency was determined by FACS (FIG. 1A; left panel), and secretion was assessed by ELISA (FIG. 1A; middle and right panels). FIG. 1B shows that OT-1 CD8⁺-T cells were genetically engineered to secrete both PD1.IgG4 and LIGHT. Transduction efficiency was determined by FACS (FIG. 1B; left and left-middle panels), and secretion was assessed by ELISA (FIG. 1B; middle-right and right panels). FIG. 1C shows that OT-1 CD8⁺-T cells were genetically engineered to secrete both PD1.IgG4 and IL-33. Transduction efficiency was determined by FACS (FIG. 1C; left and left-middle panels), and secretion was assessed by ELISA (FIG. 1C; middle-right and right panels). FIG. 1D shows that OT-1 CD8⁺-T cell was genetically engineered to secrete both PD1.IgG4 and CD40L. Transduction efficiency by FACS (FIG. 1C; left and left-middle panels), and secretion was assessed by ELISA (FIG. 1D; middle-right and right panels).

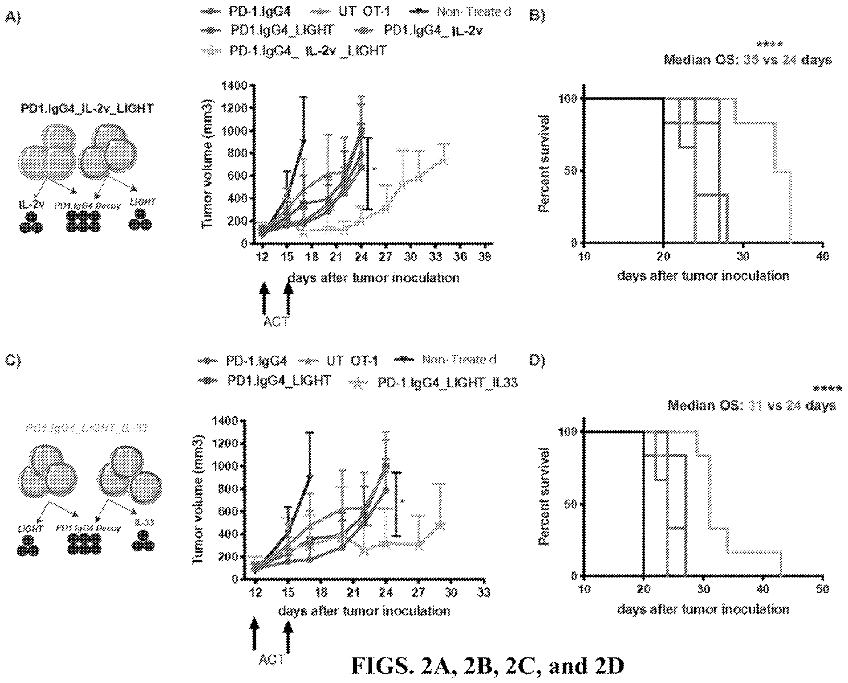

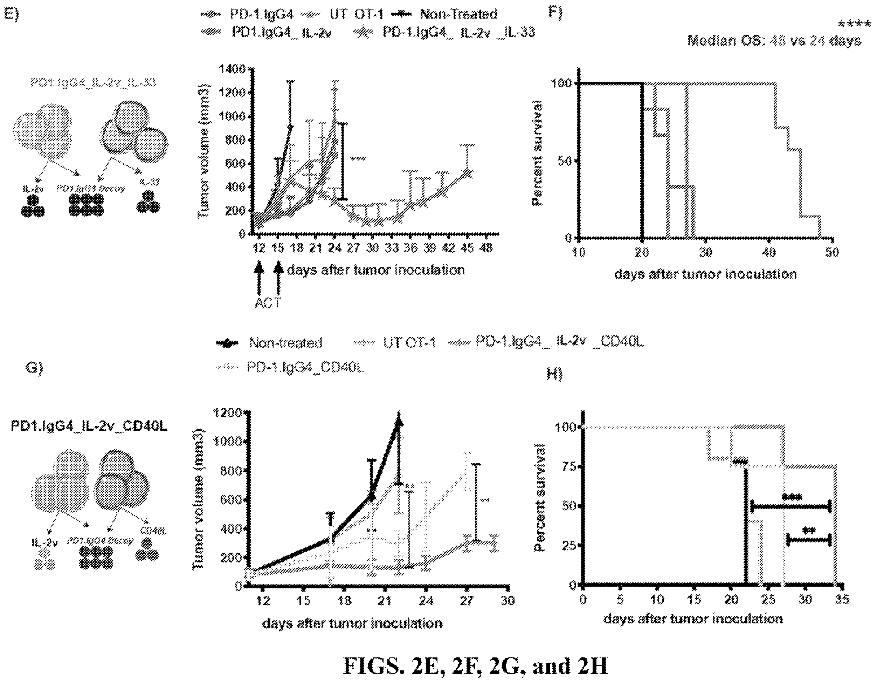

FIGS. 2A, 2B, 2C, 2D, 2E, 2F, 2G, and 2H (collectively "FIG. 2") are a set of diagrams showing that adoptive transfer of OT-1 CD8⁺-T cells genetically-modified to secrete combinations of three immunomodulatory factors significantly improved tumor control of large established B16-OVA tumors in the absence of pre-conditioning. FIGS. 2A and 2B show the tumor growth curve (FIG. 2A) and the overall survival curve (FIG. 2B) of mice receiving OT-1 CD8+-T cells secreting PD-1.IgG4, IL-2^{V}, and LIGHT. FIGS. 2C and 2D show the tumor growth curve (FIG. 2C) and the overall survival curve (FIG. 2D) of mice receiving OT-1 CD8⁺-T cells secreting PD-1.IgG4, IL-33, and LIGHT. FIGS. 2E and 2F show the tumor growth curve (FIG. 2E) and the overall survival curve (FIG. 2F) of mice receiving OT-1 CD8⁺-T cells secreting PD-1.IgG4, IL-33, and IL-2^{V}. The experiment was performed in a blinded fashion using six animals per group. FIGS. 2G and 2H show the tumor growth curve (FIG. 2G) and the overall survival curve (FIG. 2H) of mice receiving OT-1 CD8⁺-T cells secreting PD-1.IgG4, IL-2^{V}, and CD40L. Survival analysis was carried out using a log-rank mantel-cox model. Tumor growth comparison on day 27 was carried out using a Kruskal Wallis test comparing each group against mice that received UT OT-1 CD8⁺-T cells. Correction for multiple comparison was done using a Dunn's test * p<0.05, **p<0.001, ****p<0.0001.

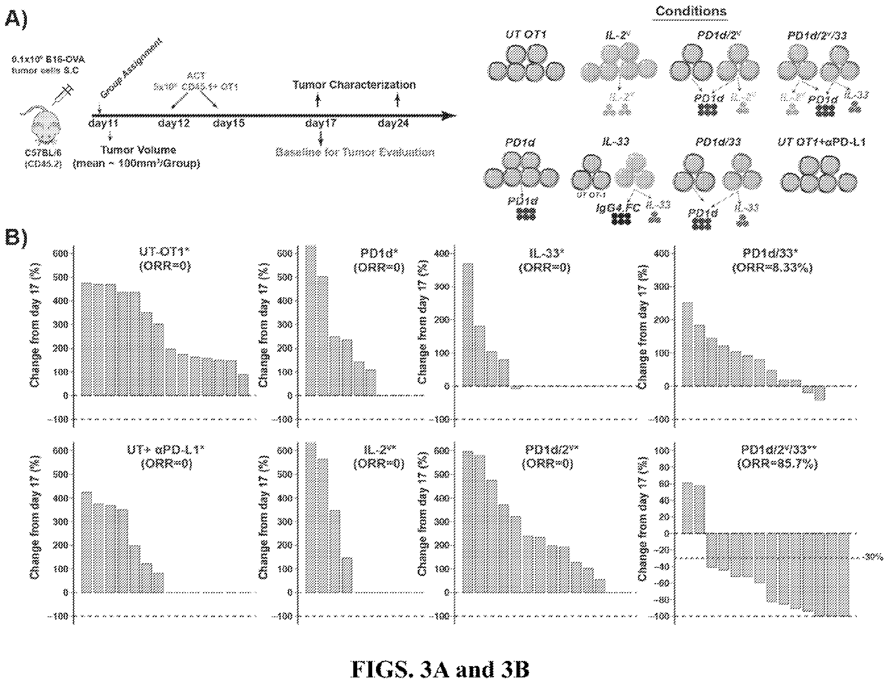

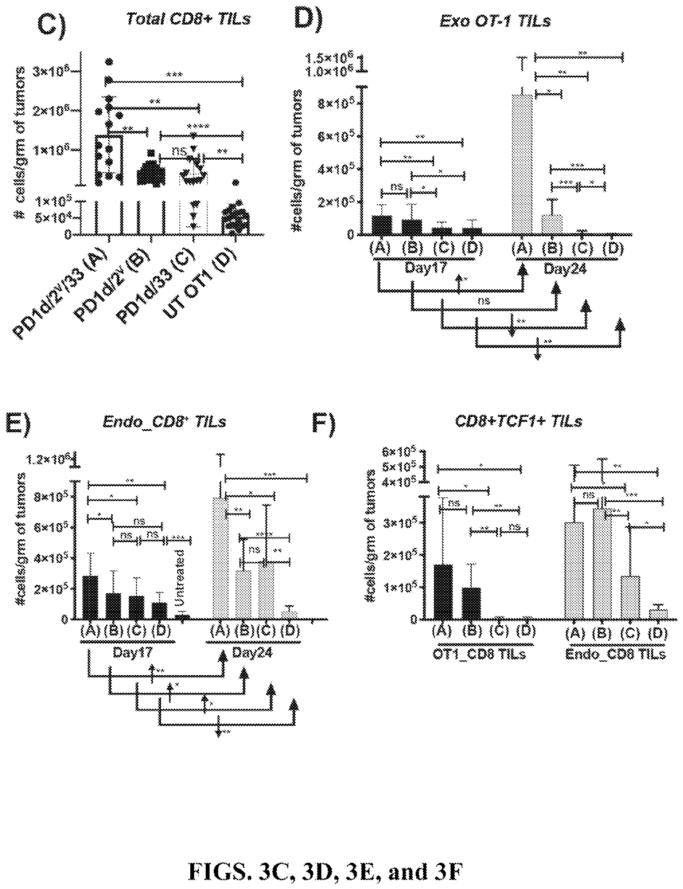

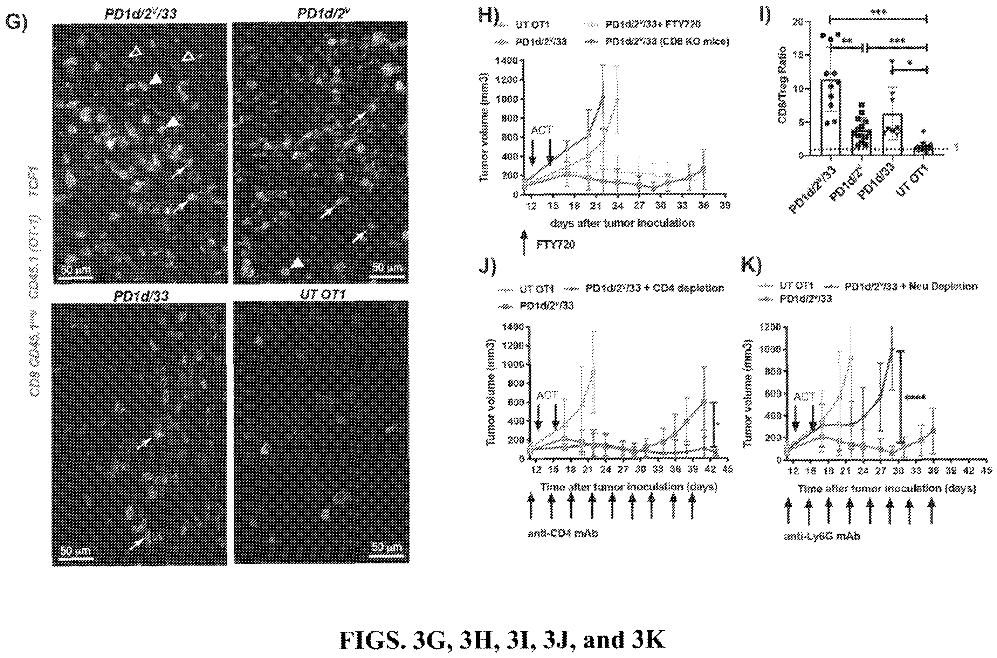

FIGS. 3A, 3B, 3C, 3D, 3E, 3F, 3G, 3H, 3I, 3J, and 3K are a set of diagrams showing that orthogonal T-cell engineering improves ACT efficacy in the immunocompetent host through expansion of adoptively transferred CD8⁺ T cells and mobilization of endogenous anti-tumor immunity. FIG. 3A shows the experimental design. FIG. 3B is a waterfall plot showing changes in tumor volumes from day 17. The best response (smallest tumor volume) observed for each animal after at least 12 days post-1^{st} ACT was taken for the calculation (* day 24 post tumor inoculation, ** day 31 post tumor inoculation). Objective Response rate (ORR) includes Complete Response (CR; 100% reduction in tumor volume) and Partial Response (PR; ≤-30% tumor change). FIGS. 3C, 3D, 3E, and 3F show that mice with B16-OVA tumors were treated with either engineering or untransduced OT1 cells as indicated; then tumors were harvested on days 17 and 24 and cell quantification was performed by flow cytometry. Data are from three independent experiments (n >= 5 animals/group). FIG. 3C shows the total numbers of CD8⁺ TILs at day 24. FIG. 3D shows the total number of CD45.1⁺ OT1 on days 17 and 24. FIG. 3E shows the total number of endogenous CD45.1^{neg} CD8 TILs on days 17 and 24. FIG. 3F shows the total numbers of endogenous and exogenous TCF1⁺ CD8⁺ TILs on day 24. FIG. 3G shows representative immunofluorescence micrographs of tumor sections from each experimental group on day 24 showing OT1 and endogenous TCF1⁺ CD8⁺ TILs. Filled triangle: TCF1⁺OT1, open triangle: TCF1^{neg}OT1, white arrows: TCF1⁺ Endogenous CD8⁺ TILs. FIG. 3H shows that PD1d/2^{V}/33+ OT-1 cells were administrated as previously indicated, to B16-OVA-tumor bearing CD8KO mice or wtC57BL6 mice that also were treated with 100µg/mouse of the drug FTY720 (administrated i.p. every three days beginning two days before 1^{st} cell transfer). FIG. 3I shows that mice with B16-OVA tumors were treated as indicated then tumors were harvested on day 24, and Treg quantification was performed by flow cytometry. Data are from three independent experiments (n >= 5 animals/group). Shown are bar plots for CD8⁺/Treg ratio. FIGS. 3J and 3K show tumor growth control over time of B16-OVA tumor-bearing mice treated with PD1d/2^{V}/33+ OT-1 cells in the presence or absence of 250 µg/mouse of depleting antibodies specific for the indicated surface markers administered i.p. beginning 1 day before 1^{st} cell transfer and maintained every three days; CD4 (maintained until day 55 post tumor inoculation) (FIG. 3J) and Ly6G (FIG. 3K). A representative experiment out of two independent experiments (n=6 animals/per group) is shown for (FIGS. 3H, 3J, and 3K). A Brown-Forsythe and Welch ANOVA test combined with Tukey Test to correct for multiple comparisons was used for comparing different groups in (FIGS. 3C, 3D, 3E, and 3F) and tumor volumes in (FIGS. 3H, 3J, and 3K). A two-tailed Student's t test with Welch's correction was used for comparing day17 and day 24 in (D and F). * p<0.05, ** p<0.01, *** p<0.001, ****p<0.0001.

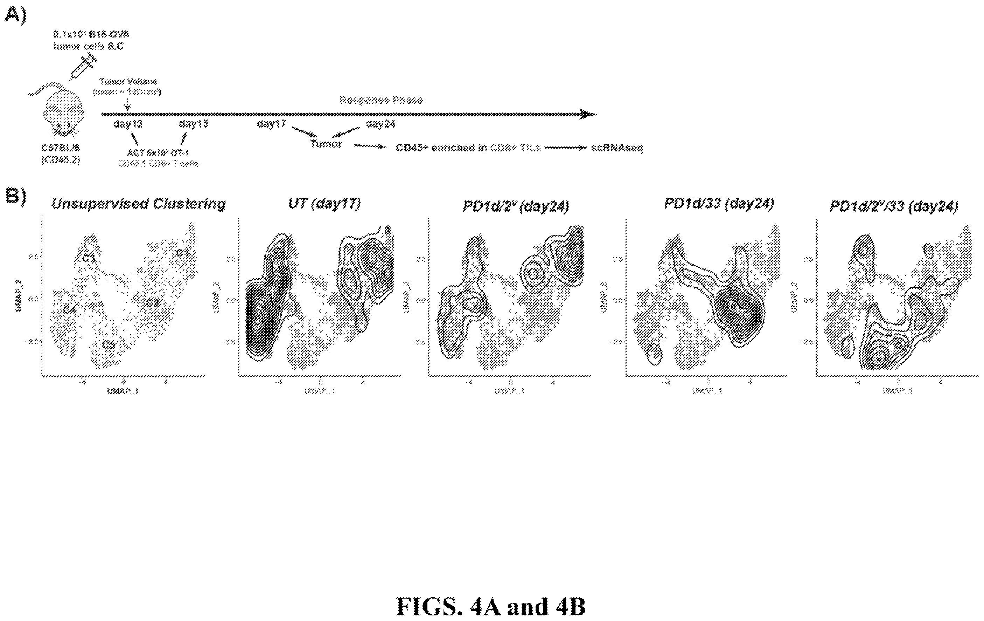

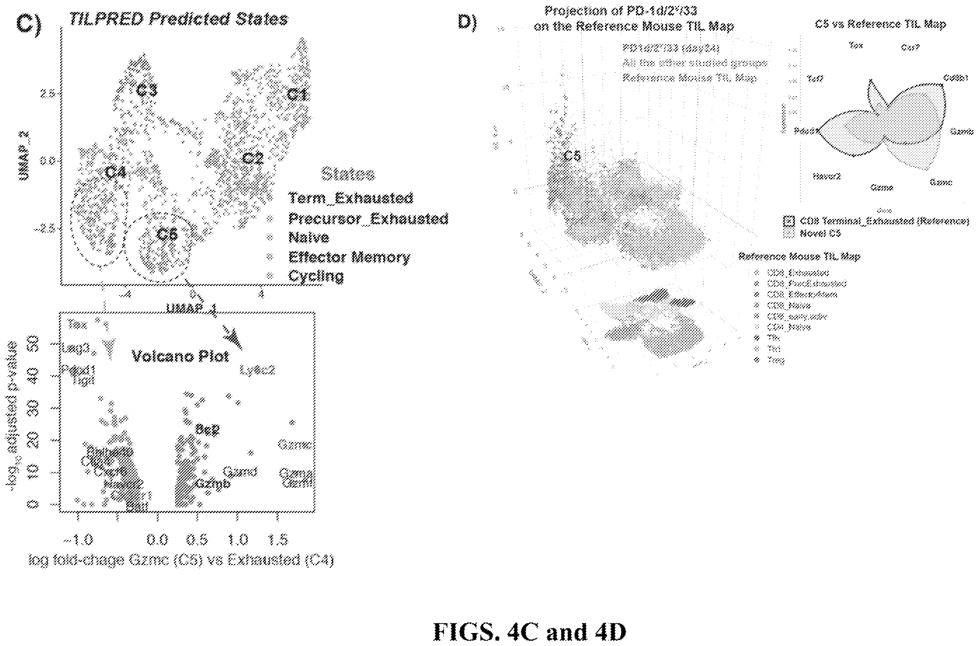

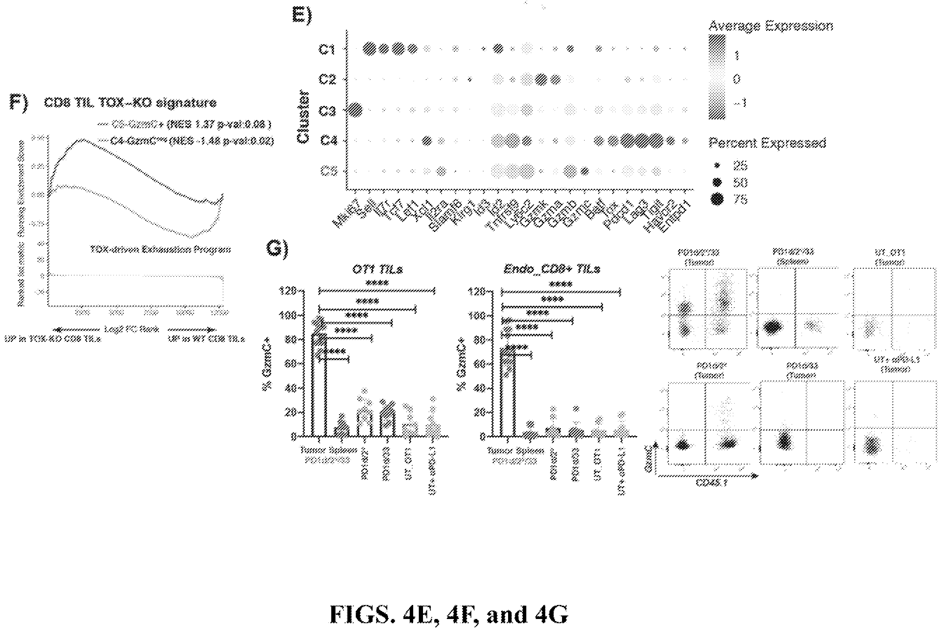

FIGS. 4A, 4B, 4C, 4D, 4E, 4F, and 4G are a set of diagrams showing that orthogonal engineering induces a novel subset of effector-like CD8 T cells different from the terminal-exhausted state and transitory CX3CR1⁺effector-like. FIG. 4A shows the experimental design. Mice with B16-OVA tumors were treated as indicated; then tumors were harvested on days 17 and 24, and a cell suspension of CD45+ enriched in CD8+TILs was obtained by FACS sorting and single cell sequenced using the 10X Genomics. FIG. 4B shows a UMAP plot depicting a low-dimensional representation of cell heterogeneity and unsupervised clustering results, where contour plots depict high cell density areas for each treatment. FIG. 4C shows TILPRED predicted CD8 TILs states (top) and Volcano Plot (bottom) depicting significant differentially expressed genes between GzmC+C5 and GzmC^{neg} Terminal-Exhausted cells. FIG. 4D shows projection of PD-1d/2V/33 (day 24) TILs onto the reference TIL map using ProjecTILs. On the right, radar plot showing expression levels of important T cell markers for projected vs. reference exhausted T cell state. FIG. 4E shows dot plots depicting clusters-specific markers. FIG. 4F shows CD8 TIL Tox Knock-out Gene Signature Enrichment Analysis (GSEA) of Gzmc+C5 vs. Gzmc^{neg} C4 cells. FIG. 4G shows that mice with B16-OVA tumors were treated with either engineering or untransduced OT1 cells on days 12 and 15 after tumor cell inoculation. Tumors or spleens (PD1d/2^{V}/33 OT1) were harvested on day 24, and intracellular expression of granzymeC was performed by flow cytometry. A summary of 2 independent experiments are shown, (n >= 5 animals/group) (UT: non-transduced). One-way ANOVA test in combination with a Dunnet Test to correct for multiple comparisons was used. * p<0.05, ** p<0.01, *** p<0.001, ****p<0.0001.

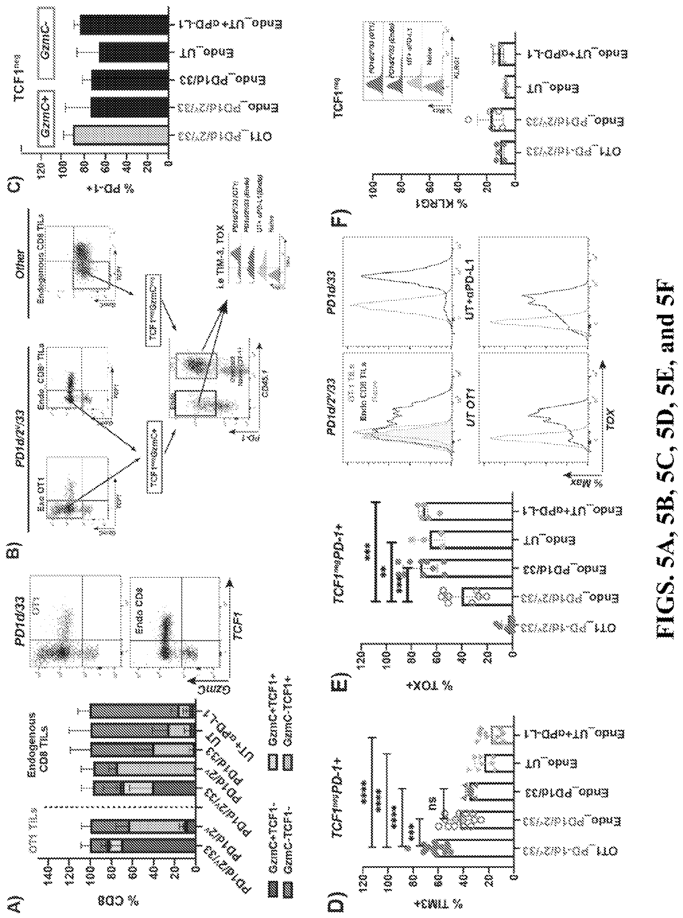

FIGS. 5A, 5B, 5C, 5D, 5E, and 5F are a set of diagrams showing that orthogonal engineering decouples the expression of TOX from that of coinhibitory receptors in GzmC⁺ TCF1^{neg} CD8⁺ TILs. FIG. 5A shows the analysis of exogenous and endogenous CD8⁺ T cell compartments based on granzyme C and TCF1 expression on day 24. No OT1 TILs were harvested from tumors post PD1d/33 ACT (UT: non-transduced). FIG. 5B shows the gating strategy for evaluation of TOX and phenotype markers. PD1d/2^{V} was not included in the statistical analysis because CD8 TILs were mostly TCF1⁺. FIG. 5C shows surface expression of PD-1 in TCF1^{neg} CD8⁺ TILs cells. FIG. 5D shows surface expression of TIM-3 in PD-1⁺ TCF1^{neg} CD8⁺ TILs. For these two surface markers, data obtained from either 4 independent experiments (PD1d/2^{V}/33) or 2 (other groups) are shown (n= 4 or 5 animals/per experiment, day24 post tumor inoculation). FIG. 5E shows TOX expression in PD-1⁺ TCF1^{neg} CD8⁺ TILs. Data obtained from either 2 independent experiments (PD1d/2^{V}/33, PD1d/33) or 1 (other groups) are shown (n= 4 or 5 animals/per experiment, day 24 post tumor inoculation). FIG. 5F shows KLRG1 surface expression in TCF1^{neg} CD8 TILs. A representative experiment out of two independent experiments (n=5 animals/per group) is shown. One-way ANOVA test in combination with a Dunnet Test to correct for multiple comparisons was used * p<0.05, ** p<0.01, *** p<0.001, ****p<0.0001. Naive OT-1 T cells isolated from the spleen of non-tumor bearing mice were used as internal negative control of the FACS staining.

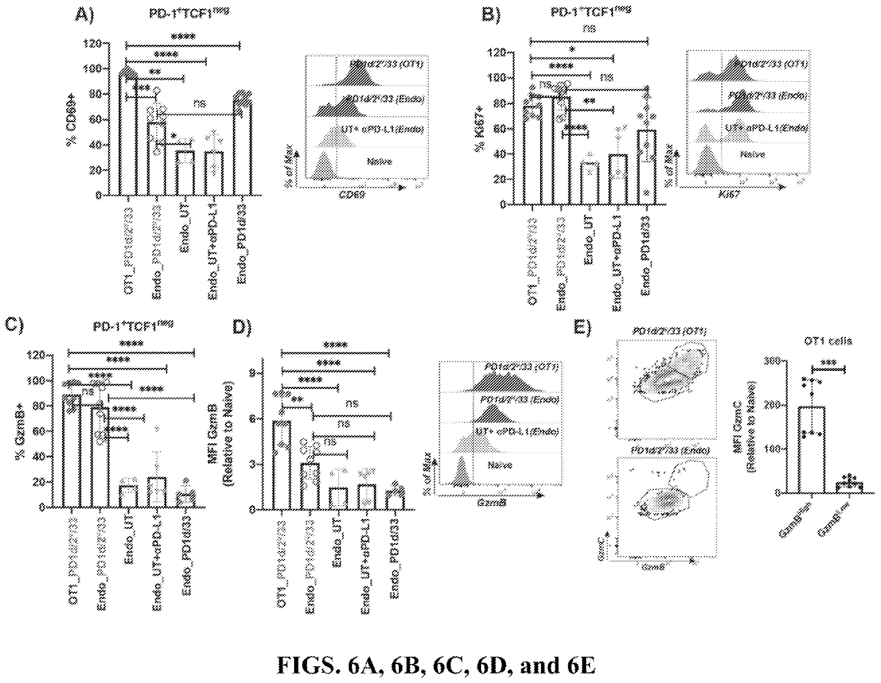

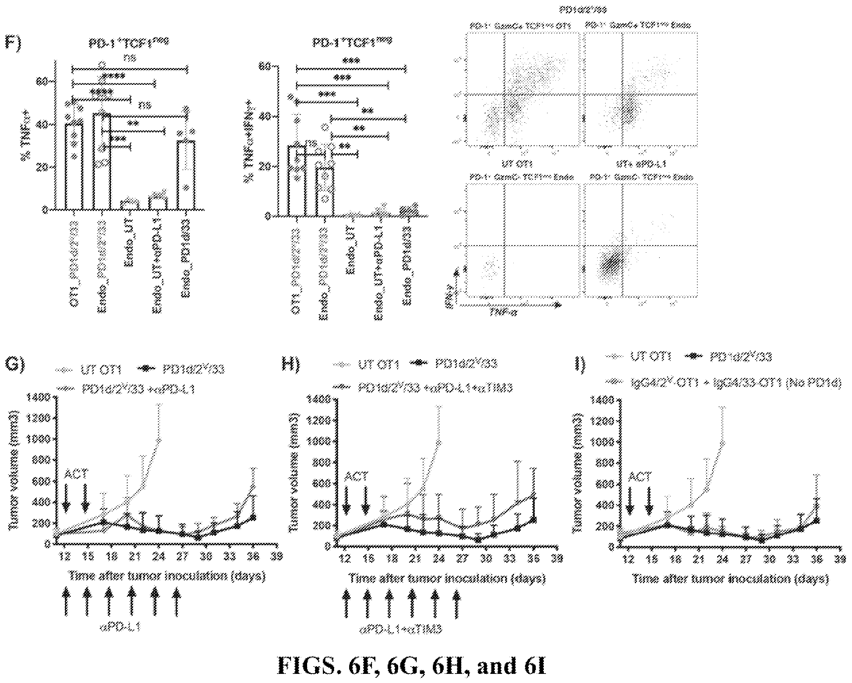

FIGS. 6A, 6B, 6C, 6D, 6E, 6F, 6G, 6H, and 6I are a set of diagrams showing that GzmC⁺ TCF1^{neg} CD8⁺ TILs are polyfunctional effector cells with inconsequential expression of coinhibitory receptors. OT1 and endogenous CD8 TILs from animals treated with gene-engineered or untransduced (UT) OT1 cells were analyzed on day 24 for the quantification of effector molecules in the PD-1⁺ TCF1^{neg} CD8⁺ TILs. No OT1 TILs were harvested from tumors post PD1d/33 ACT or UT. PD1d/2^{V} was not included in the statistical analysis because CD8 TILs were mostly TCF1⁺. FIG. 6A shows surface expression of CD69. FIG. 6B shows intracellular of Ki-67. FIG. 6C shows intracellular expression of Granzyme B. FIG. 6D shows the normalized MFI of Granzyme B expression relative to naive OT-1 T cells isolated from non-tumor bearing mice. FIG. 6E shows co-expression of Granzyme B, and FIG. 6F shows intracellular expression of TNFα and INFγ after 4 hours *ex vivo* stimulation with aCD3 and aCD28 antibodies. Data shown in FIGS. 6A and 6B were obtained from either 2 independent experiments (PD1d/2^{V}/33, PD1d/33) or 1 (other groups) (n= 4-6 animals/group). Data shown in FIGS. 6C, 6D, 6E, and 6F were obtained from either 2 independent experiments (PD1d/2^{V}/33) or 1 (other groups) (n= 4-6 animals/group). One-way ANOVA test in combination with a Dunnet Test to correct for multiple comparisons was used * p<0.05, ** p<0.01, *** p<0.001, ****p<0.0001. Naive OT-1 T cells isolated from the spleen of non-tumor bearing mice were used as an internal negative control of FACS staining. FIGS. 6G and 6H show tumor growth control over time of B16-OVA tumor-bearing mice treated with PD1d/2^{V}/33+ OT-1 cells in the presence or absence of 250µg/mouse of antibodies specific for the indicated surface markers administered i.p. beginning 1 day before 1^{st} cell transfer and maintained every three days maximum 6 doses; αPD-L1 (FIG. 6G) and αPD-L1+αTIM3 (FIG. 6H). FIG. 6I shows tumor growth control over time of B16-OVA tumor-bearing mice treated with PD1 d/2^{V}/33⁺ OT1 cells or with OT1 T cells gene-engineered for secreting IL-2^{V} and IL-33 (no PD-1 ectodomain). Similar to PD1d/2^{V}/33, this arm resulted from mixing IgG4/IL-33 expressing OT-1 (no PD-1 decoy production) with IgG4/IL-2^{V} expressing OT-1 cells in a 1:1 ratio. The expression of both IgG4 Fc and IL-33 was confirmed by FACS and ELISA and was not significantly different from the PD1d/33 (n=8 animals/group). A representative experiment out of two independent experiments (n=6 animals/group) is shown for experiments in FIGS. 6G, 6H, and 6I. Naive OT-1 T cells isolated from the spleen of non-tumor bearing mice were used as internal negative control of the FACS staining.

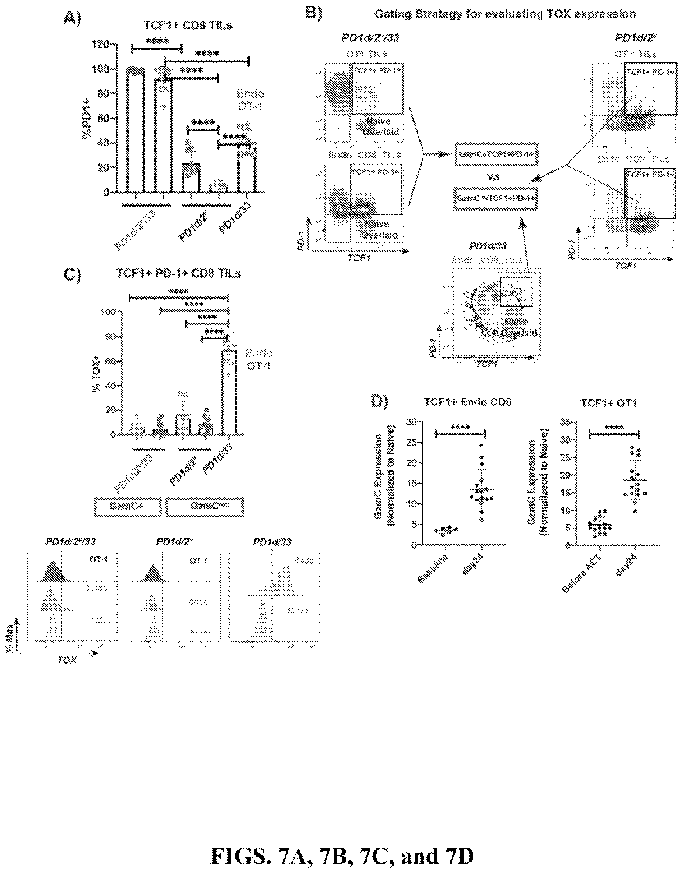

FIGS. 7A, 7B, 7C, and 7D are a set of diagrams showing that orthogonal engineering drives TOX^{neg/low} GzmC⁺ precursor differentiation. FIG. 7A shows the analysis of PD-1 expression in TCF1⁺ CD8⁺ TILs harvested on day 24. FIG. 7B shows the gating strategy for evaluating TOX expression in PD-1⁺TCF1⁺ cells. FIG. 7C shows the analysis of TOX expression in GzmC+PD-1⁺TCF1⁺ CD8 TILs versus GzmC^{neg}PD-1⁺TCF1⁺ CD8 TILs cells from PD1d/2^{V}. Data shown in FIGS. 7A, 7B, and 7C were obtained from 2 independent experiments (n= 4-6 animals/group). One-way ANOVA test in combination with a Dunnet Test to correct for multiple comparisons was used * p<0.05, ** p<0.01, *** p<0.001, ****p<0.0001. FIG. 7D shows a comparative analysis of Granzyme C expression (normalized MFI to naive OT1) in TCF1⁺ CD8⁺ TILs harvested on day 24 from mice treated with PD1d/2^{V}/33+OT1 (3 independent experiments, n>=4 animals/group) relative to endogenous TCF1⁺ CD8 TILs harvested on day 12 after tumor inoculation (baseline, n=6 animals) and TCF1⁺ gene-engineered OT1 cells post expansion *in vitro* (Before ACT, n=14). Naive OT-1 T cells isolated from the spleen of non-tumor bearing mice were used as internal negative control of the FACS staining.

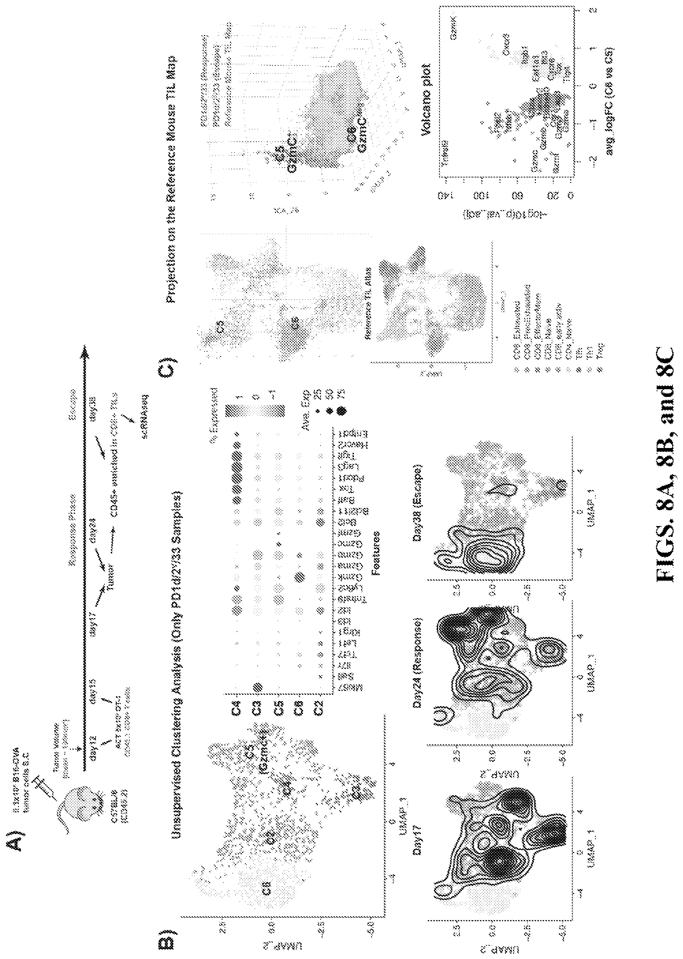

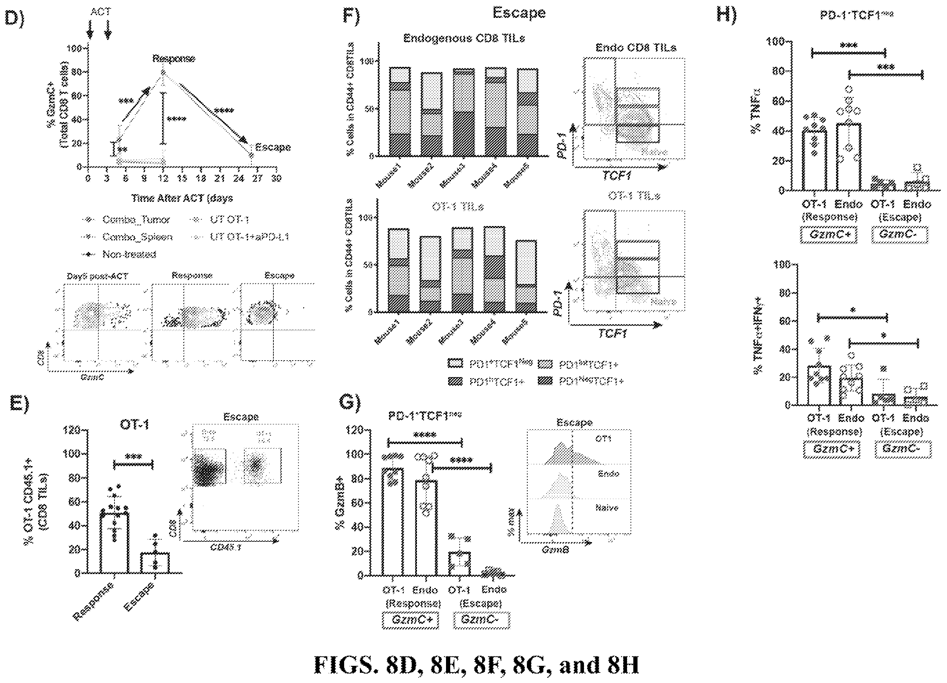

FIGS. 8A, 8B, 8C, 8D, 8E, 8F, 8G, and 8H are a set of diagrams showing that the novel TCF1^{neg}CD8⁺ TIL effector state induced by orthogonal engineering is dynamically associated with tumor response. FIG. 8A shows the experimental design. Mice with B16-OVA tumors were treated as indicated on days 12 and 15 after tumor cell inoculation. Tumors were harvested on days 17, 24, and 38, and a cell suspension of CD45+ enriched in CD8+TILs was obtained by FACS sorting and single cell sequenced using the 10X Genomics. FIG. 8B shows a UMAP plot showing a low-dimensional representation of cell heterogeneity and unsupervised clustering results of only PD1d/2^{V}/33 samples across different time points, where contour plots depict high cell density areas for each treatment. Dot plots showing clusters-specific markers (bottom). FIG. 8C shows projection of clusters C5 and C6 on the reference TIL map using ProjecTILs. The independent component IC26 also significantly separates the unique Cluster C5 observed during tumor control from TILs obtained during escape. Bottom right: Volcano plot showing significant differentially expressed genes between clusters C6 and C5. FIG. 8D shows the analysis of Granzyme C expression in Total CD8⁺ TILs cells harvested during tumor control (day 24) and escape (day 38). CD8⁺ T cells residing in the Spleen of Triple_Combo-treated mice were included as control as well as CD8⁺ TILs from either non-treated or UT OT1-treated mice. Data obtained from either 2 independent experiments (PD1d/2^{V}/33) are shown. FIG. 8E shows the analysis of OT1 (CD45.1⁺) intratumoral persistence in total CD8⁺ TILs harvested during tumor control (day 24) and escape (day 38). FIG. 8F shows the analysis of exogenous and endogenous CD8⁺ TILs harvested during tumor control (day 24) and escape (day38) based on PD-1 and TCF1 expression. Analysis of intracellular expression of granzymeB (FIG. 8G) TNFα and INFγ (FIG. 8H) in PD-1⁺ TCF1^{neg} CD8 TILs harvested during tumor control (day 24) or escape (day 38) after 4 hours *ex vivo* stimulation with anti-CD3 and anti-CD28 antibodies. Data obtained from either 2 independent experiments (tumor control) or 1 (escape) are shown, (n= 4-6 animals/group). A two-tailed Student's t test to compare two groups was used. * p<0.05, ** p<0.01, *** p<0.001, ****p<0.0001. Naive OT-1 T cells isolated from the spleen of non-tumor bearing mice were used as internal negative control of the FACS staining.

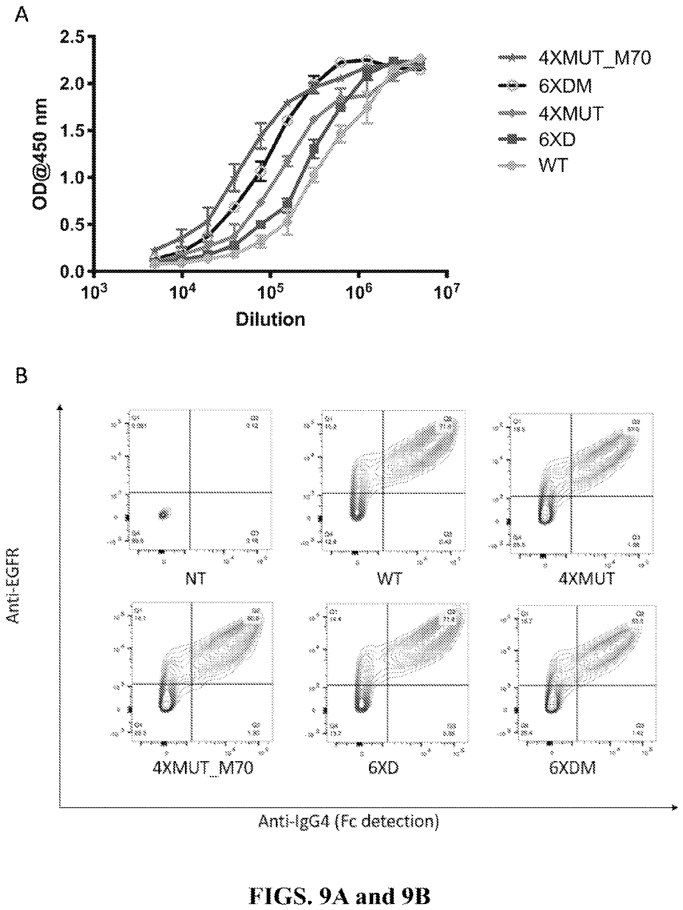

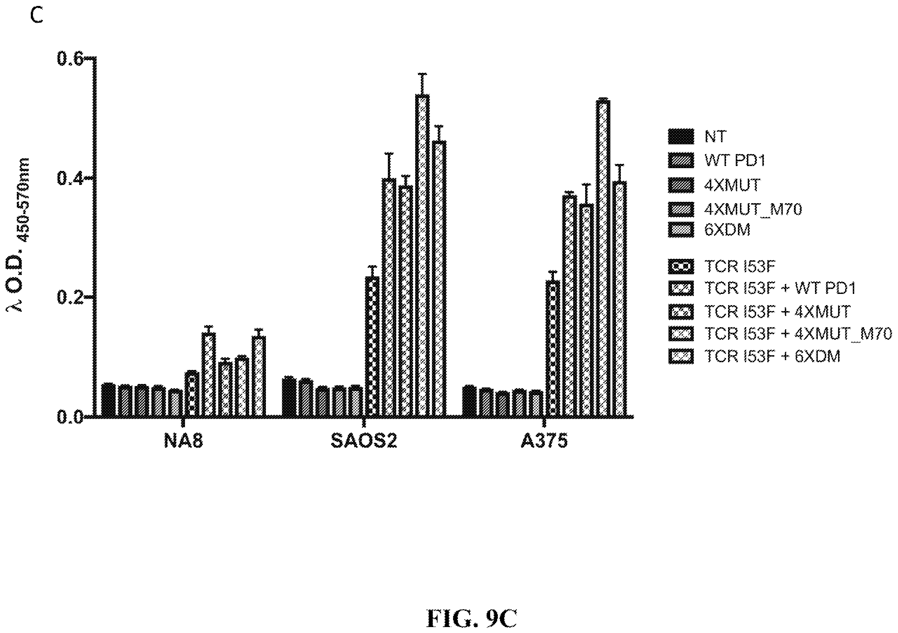

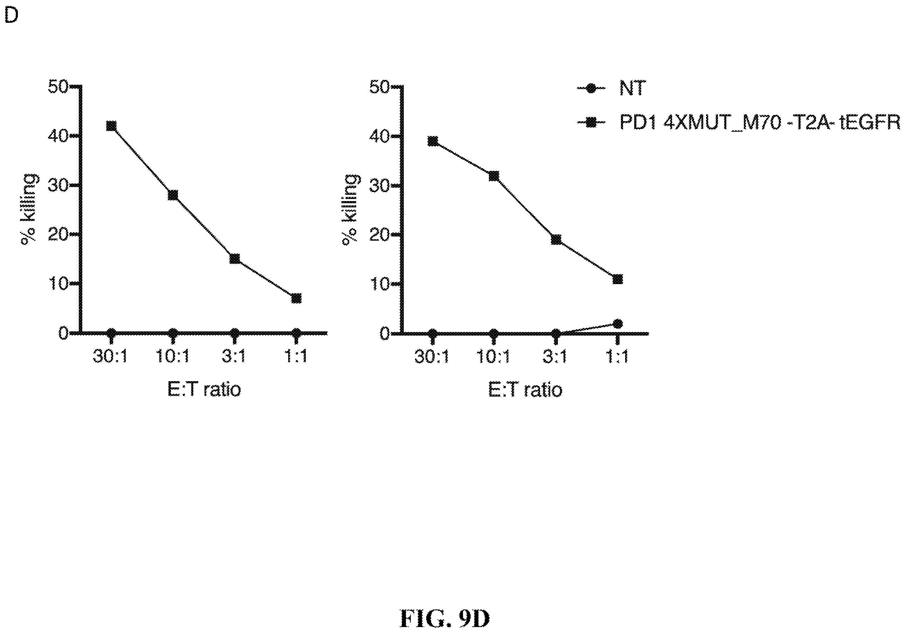

FIGS. 9A, 9B, 9C, and 9D are a set of diagrams showing characterization of PD-1 decoy variants and hCD8+ T cells transduced with the PD-1 decoy variants and tEGFR. FIG. 9A shows titration ELISA of soluble monomeric PD1 decoy variants (bacterial production) against plates coated with human PDL1 protein. Bound PD1 decoy molecules were detected with anti-His tag antibody. The PD-1 decoy variant 4XMUT_M70 binds 10-fold better and the variant 6XDM about 7.5-fold better than the WT PD1 decoy to PD-L1. FIG. 9B shows detection of tEGFR and intracellular PD1 decoy of retrovirally transduced CD8⁺ T cells. FIG. 9C shows IFNγ production by NY-TCR (I53F) engineered CD8⁺ T cells co-expression PD-1 decoy (variants) and tEGFR. The engineered T cells were co-cultured at a 1:1 ratio with different PD-L1+ target tumor cells (100,000 of each cell type) for 48 hours. NA8 and HLA/A2+ NY-ESO-1-, SAOS2, and A375 are HLA/A2+ NY-ESO-1+. The supernatants were collected after 48 hours, diluted 1 in 25 and evaluated for the presence of IFNγ using a commercial ELISA kit from Thermo). Shown are data for a representative T-cell donor. In all assays, the variants do better than the WT PD-1 decoy. FIG. 9D shows the results of an ADCC assay of human T cells transduced to express the PD1 decoy (4XMUT_M70E) and tEGFR. CD8 T cells engineered with PD1 decoy_tEGFR retrovirus were labeled with chromium. The engineered T cells were co-cultured with anti-EGFR Ab and co-cultured with different ratios of PBMCs from the same donor. The negative control is NT (non-transduced) T cells Killing evaluated at 4 hours. As positive control, T cells were treated with HCl.

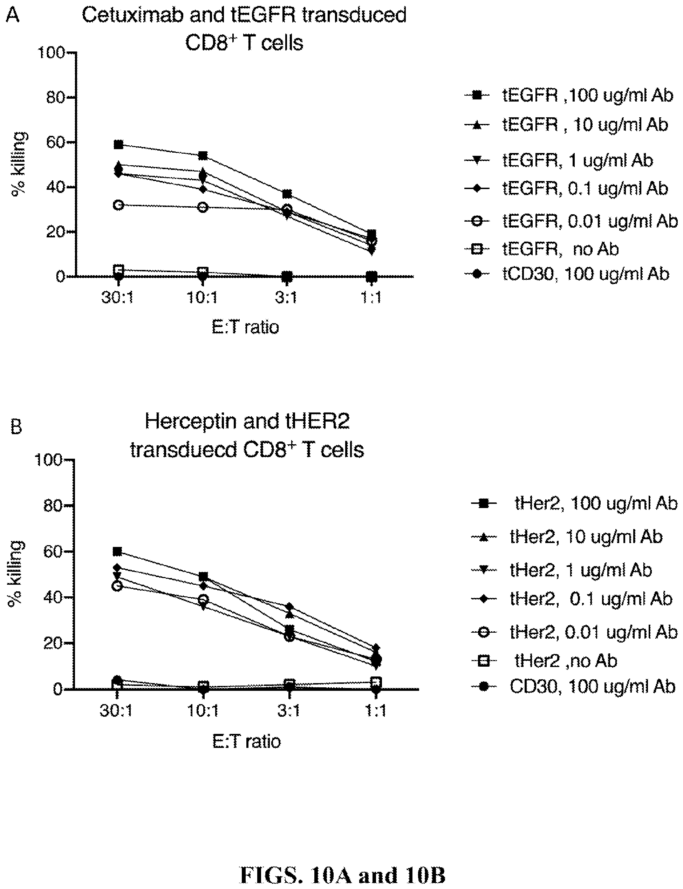

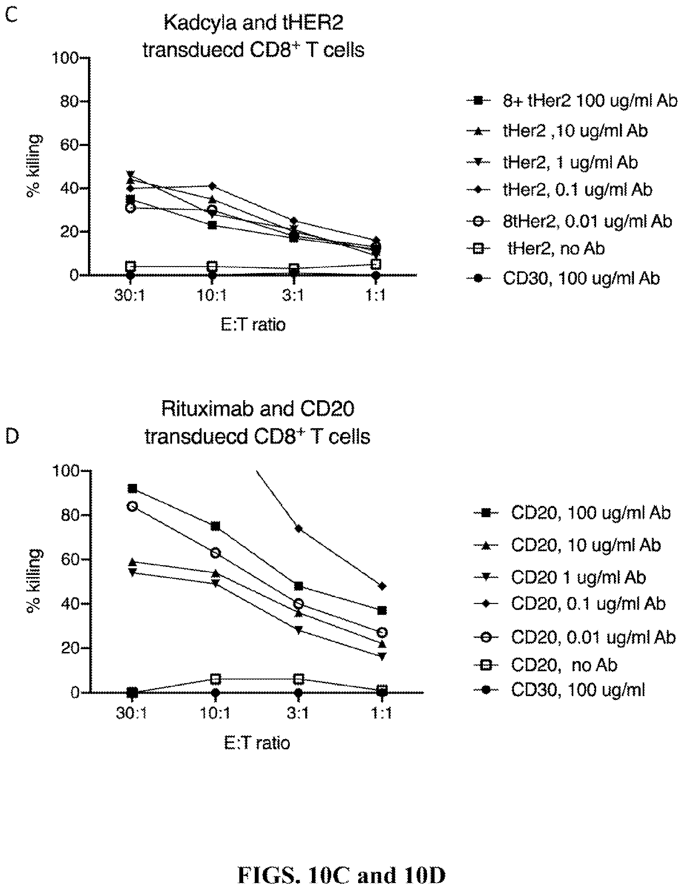

FIGS. 10A, 10B, 10C, and 10D are a set of diagrams showing the results of an antibody-dependent cytotoxicity (ADCC) assay wherein T cells were engineered to express tEGFR. FIG. 10A shows that tEGFR engineered CD8⁺ T cells were loaded with chromium and cultured for 4-5 hours with PBMCs at different ratios along with decreasing concentrations of the anti-EGFR Ab Cetuximab. As a negative control, tCD30 engineered T cells were used in the assay along with the maximum concentration of Cetuximab (100ug/ml). Released chromium is used as a measure of lysed T cells. FIGS. 10B and 10C show the results of an ADCC assay for tHER2 engineered CD8⁺ T cells (left: Herceptin (FIG. 10B); right: Kadcyla (FIG. 10C)). FIGS. 10D shows the results of an ADCC assay for CD20 engineered CD8⁺ T cells.

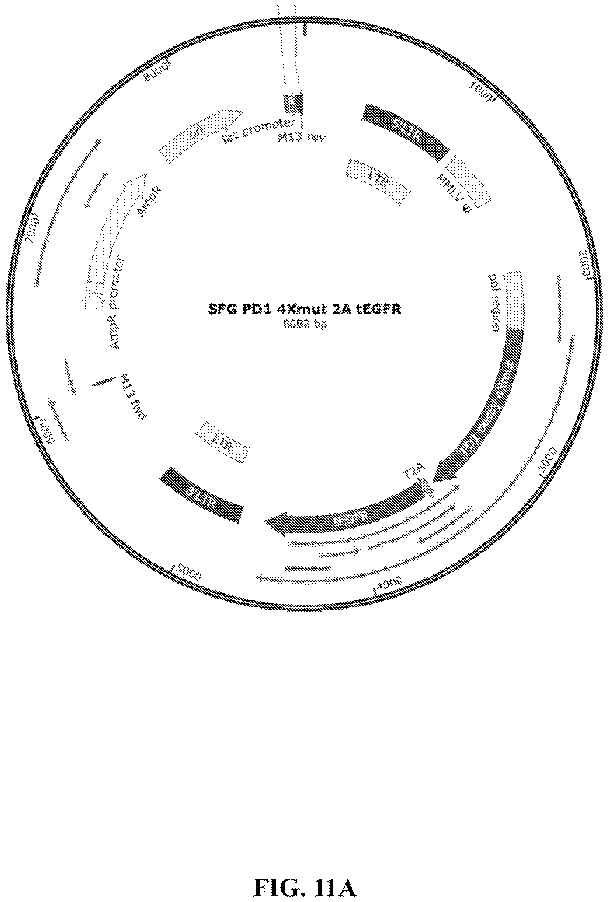

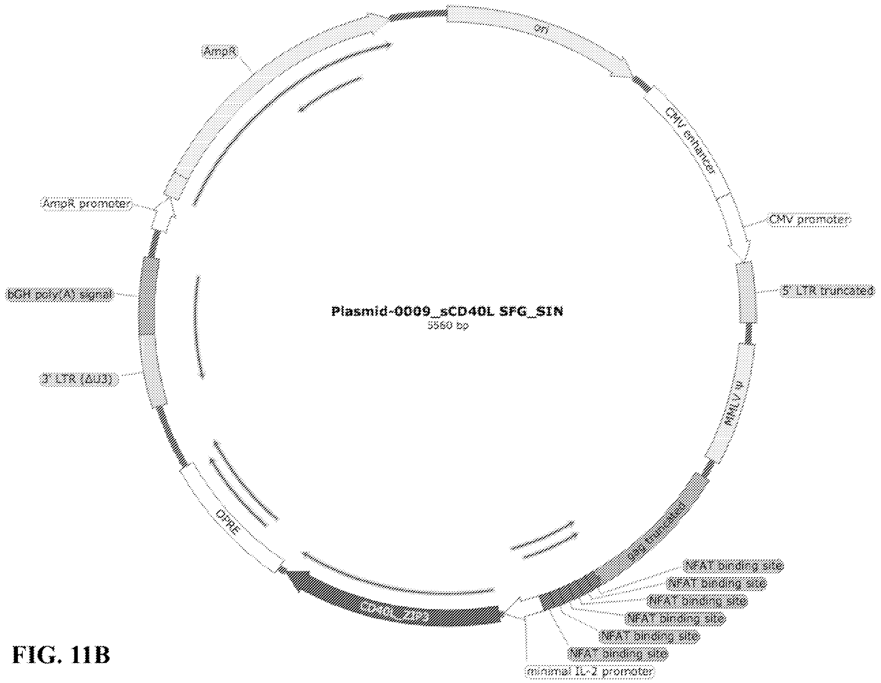

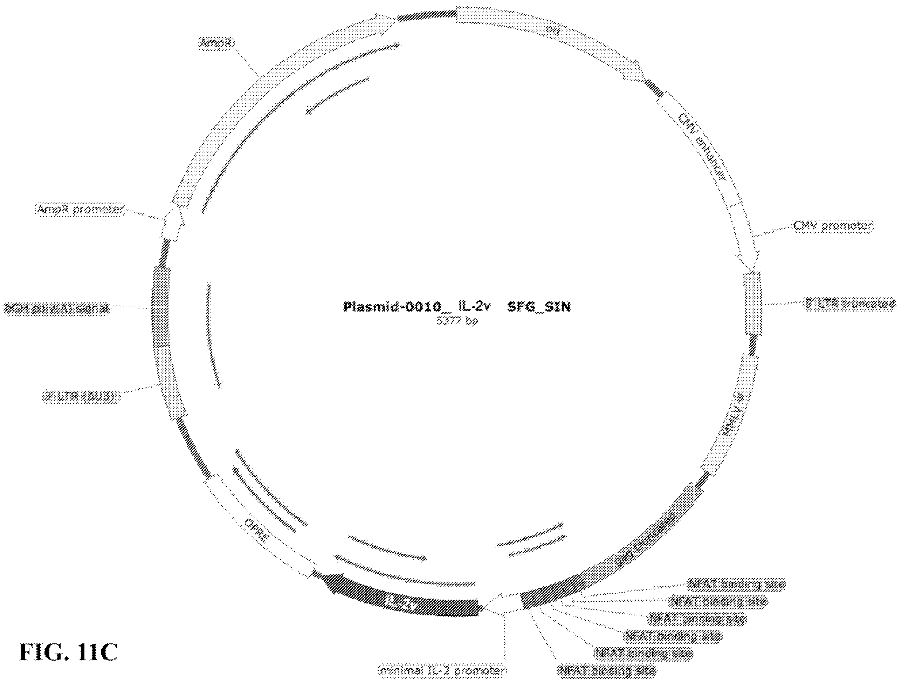

FIGS. 11A, 11B, and 11C are a set of diagrams showing the representative constructs carrying transgenes used to transduce lymphocytes. The representative constructs carry a PD-1 decoy and a tEGFR (FIG. 11A), a CD40L variant (FIG. 11B), and an IL-2 variant (also referred to as IL-2^{V}) (FIG. 11C), respectively.

### DETAILED DESCRIPTION OF THE INVENTION

The technical information set out below may in some respects go beyond the disclosure of the invention, which is defined exclusively by the appended claims. The additional technical information is provided to place the actual invention in a broader technical context and to illustrate possible related technical developments. Such additional technical information which does not fall within the scope of the appended claims, is not part of the invention.

The present disclosure relates to methods and compositions to confer and/or increase immune responses mediated by cellular immunotherapy, such as by adoptively transferring tumor-specific genetically-modified subsets of lymphocytes. The disclosure provides compositions comprising genetically-modified lymphocytes that express at least two transgene(s) having the ability to modulate the immune system and the innate and adaptive immune response. The disclosed methods and compositions are embodiments of the platform technology, termed Genetic Engineering for the Enhanced Performance of T-cells (GEEP-T^{™}). GEEP-T^{™} is aimed to provide genetically-engineered lymphocytes with enhanced anti-tumor functions as well as methods of developing such lymphocytes.

### A. COMPOSITIONS AND KITS

This disclosure provides a composition comprising a plurality of genetically-modified lymphocytes expressing at least two transgenes (*e.g*., therapeutic transgenes) for modulating the immune system of a subject.

In some embodiments, lymphocytes are peripheral blood lymphocytes (PBLs). In some embodiments, lymphocytes are tumor-infiltrating lymphocytes (TILs). Lymphocytes may include T cells, B cells, NK cells, macrophages, neutrophils, dendritic cells, mast cells, eosinophils, and basophils. In some embodiments, lymphocytes are derived from CD34 hematopoietic stem cells, embryonic stem cells, or induced pluripotent stem cells. Lymphocytes can be autologous, allogeneic, syngeneic, or xenogeneic. In some embodiments, lymphocytes are autologous. In some embodiments, lymphocytes are human lymphocytes.

In some embodiments, the lymphocytes can be tumor-infiltrating lymphocytes (TILs). In some embodiments, the lymphocytes may express a chimer antigen receptor (CAR). In some embodiments, the lymphocytes may express a recombinant T cell receptor (TCR). The CAR or TCR may bind to a cancer antigen. In some embodiments, the CAR or TCR may show reactivity against NY-ESO1, MAGE-A1, MAGE-A3, MAGE A-10, MAGE-C2, SSX2, MAGE-A12, or a combination thereof.

The transgene may encode a molecule selected from the group consisting of a soluble receptor, a decoy, a dominant negative, a microenvironment modulator, an enzyme, an oxidoreductase, a transferase, a hydrolases, a lysase, an isomerase, a translocase, a kinase, a transporter, a modifier, a molecular chaperone, an ion channel, an antibody, a cytokine, a chemokine, a hormone, a DNA, a ribozyme, a biosensor, an epigenetic modifier, a transcriptional factor, a coding RNA, a non-coding RNA, a small-RNA, a long-RNA, an IRES element, or an exosomal-shuttle RNA.

The transgene may encode at least two molecules selected from the group consisting of a soluble receptor, a decoy, a dominant negative, a microenvironment modulator, an enzyme, an oxidoreductase, a transferase, a hydrolase, a lysase, an isomerase, a translocase, a kinase, a transporter, a modifier, a molecular chaperone, an ion channel, an antibody, a cytokine, a chemokine, a hormone, a DNA, a ribozyme, a biosensor, an epigenetic modifier, a transcriptional factor, a coding RNA, a non-coding RNA, a small-RNA, a long-RNA, an IRES element, an exosomal-shuttle RNA, or any combination thereof.

The two or more molecules encoded by the transgene may be linked by a self-cleaving peptide sequence. The transgene expression may be regulated by a constitutively activated promoter. The transgene expression may be regulated by an inducible promoter. The transgene expression may be induced by the activation status of the lymphocyte. The transgene may be introduced to the lymphocytes via integration-competent gamma-retroviruses or lentivirus, DNA transposition, etc.

The transgenes may be selected from the group consisting of antibodies, antibody fragments, receptors, decoys, checkpoint blockade modulators, cytokines, chemokines, hormones, cellular elimination tags, and combinations thereof.

The antibodies or antibody fragments may be VEGF, TGF-B, 4-1BB, CD28, CD27, NKG2D, PD1, PDL1, or CTLA4 antibodies. The antibody may be a PD1 antibody. The decoy can be PD1, CTLA4, LAG3, VEGFR1, TIM3, TIGIT, or SIRPalpha decoy. The decoy may be a PD1 decoy, such as a PD-1.IgG4 decoy.

The cytokine may selected from the group consisting of LIGHT or a variant/fragment thereof, IL-33 or a variant/fragment thereof, IL-2 or a variant/fragment thereof, IL-15 or a variant/fragment thereof, IL-12 or a variant/fragment thereof, and CD40L or a variant/fragment thereof. The cytokine may be a mutant cytokine.

In some embodiments, the cellular elimination tag is selected from the group consisting of tEGFR, Her2, CD20, and CD19.

The transgenes may comprise two or more of a PD-1 decoy or a variant/fragment thereof, an IL-2 variant/fragment, LIGHT or a variant/fragment thereof, IL-33 or a variant/fragment thereof, and CD40L or a variant/fragment thereof. The transgenes may further comprise tEGFR or a variant/fragment thereof, tHER2 or a variant/fragment thereof, CD20 or a variant/fragment thereof, or CD19 or a variant/fragment thereof.

The PD-1 decoy or a variant/fragment thereof may be harbored on the same vector as a cellular elimination tag (CET), such as tEGFR or the variant/fragment thereof, tHER2 or a variant/fragment thereof, CD20 or a variant/fragment thereof, and CD19 or a variant/fragment thereof.

The at least two transgenes may comprise: (a) the PD-1 decoy or the variant/fragment thereof and tEGFR or the variant/fragment thereof; (b) the PD-1 decoy or the variant/fragment thereof and the IL-2 variant/fragment; (c) the PD-1 decoy or the variant/fragment thereof and the LIGHT or the variant/fragment thereof; (d) the PD-1 decoy or the variant/fragment thereof and the IL-33 or the variant/fragment thereof; (e) the PD-1 decoy or the variant/fragment thereof and the CD40L or the variant/fragment thereof; (f) the PD-1 decoy or the variant/fragment thereof, the IL-2 variant/fragment, and the IL-33 or the variant/fragment thereof; (g) the PD-1 decoy or the variant/fragment thereof, the tEGFR or the variant/fragment thereof, and the IL-2 variant/fragment; (h) the PD-1 decoy or the variant/fragment thereof, the tEGFR or the variant/fragment thereof, and the LIGHT or the variant/fragment thereof; (i) the PD-1 decoy or the variant/fragment thereof, the tEGFR or the variant/fragment thereof, and the IL-33 or the variant/fragment thereof; (j) the PD-1 decoy or the variant/fragment thereof, the tEGFR or the variant/fragment thereof, and the CD40L or the variant/fragment thereof; (k) the PD-1 decoy or the variant/fragment thereof, the tEGFR or the variant/fragment thereof, the IL-2 variant/fragment, and the IL-33 or the variant/fragment thereof; (l) the PD-1 decoy or the variant/fragment thereof, the tEGFR or the variant/fragment thereof, the IL-2 variant/fragment, and the CD40L or the variant/fragment thereof; or (m) the PD-1 decoy or the variant/fragment thereof, the tEGFR or the variant/fragment thereof, the IL-33 variant/fragment and the CD40L or the variant/fragment thereof.

The PD-1 decoy may comprise an amino acid sequence of any one of SEQ ID NOs: 1-4, 6-17, 42, 44, 47-48, and 51-52 or an amino acid sequence having at least 80% (*e.g.,* 80%, 85%, 90%, 95%, 96%, 97%, 98%, 99%) identity to any one of SEQ ID NOs: 1-4, 6-17, 42, 44, 47-48, and 51-52.

The IL-2 variant may comprise an amino acid sequence of any one of SEQ ID NOs: 21-23 or an amino acid sequence having at least 80% (*e.g.,* 80%, 85%, 90%, 95%, 96%, 97%, 98%, 99%) identity to any one of SEQ ID NOs: 21-23.

The IL-33 may comprise an amino acid sequence of any one of SEQ ID NOs: 25 and 27 or an amino acid sequence having at least 80% (*e.g*., 80%, 85%, 90%, 95%, 96%, 97%, 98%, 99%) identity to any one of SEQ ID NOs: 25 and 27.

The LIGHT may comprise an amino acid sequence of any one of SEQ ID NOs: 28-29 and 31 or an amino acid sequence having at least 80% (*e.g.,* 80%, 85%, 90%, 95%, 96%, 97%, 98%, 99%) identity to any one of SEQ ID NOs: 28-29 and 31.

The CD40L may comprise an amino acid sequence of any one of SEQ ID NOs: 32-34, 36, and 38 or an amino acid sequence having at least 80% (*e.g.,* 80%, 85%, 90%, 95%, 96%, 97%, 98%, 99%) identity to any one of SEQ ID NOs: 32-34, 36, and 38.

The tEGFR may comprise an amino acid sequence having at least 80% identity to SEQ ID NO: 40 or the amino acid sequence of SEQ ID NO: 40.

The HER2 may comprise an amino acid sequence having at least 80% (*e.g.,* 80%, 85%, 90%, 95%, 96%, 97%, 98%, 99%) identity to SEQ ID NO: 45 or the amino acid sequence of SEQ ID NO: 45.

The CD20 may comprise an amino acid sequence having at least 80% (*e.g.,* 80%, 85%, 90%, 95%, 96%, 97%, 98%, 99%) identity to SEQ ID NO: 49 or the amino acid sequence of SEQ ID NO: 49.

Also within the scope of this disclosure are the novel PD-1 decoy variants comprising an amino acid sequence having at least 80% (*e.g.,* 80%, 85%, 90%, 95%, 96%, 97%, 98%, 99%) identity to any one of SEQ ID NOs: 6-17, 42, 44, 47-48, and 51-52 or the amino acid sequence of any one of SEQ ID NOs: 6-17, 42, 44, 47-48, and 51-52.

The composition may comprise at least two subsets of lymphocytes. For example, the composition may include two, three, four, five or more genetically-modified subsets of lymphocytes. Each subset of genetically-modified lymphocytes may express at least one transgene. For example, each subset of genetically-modified lymphocytes may express two, three, four, five or more transgenes.

The composition may comprise two genetically-modified subsets of lymphocytes, in which each subset expresses at least one transgene. The composition may comprise two genetically-modified subsets of lymphocytes, wherein each subset expresses two transgenes. The composition may comprise three genetically-modified subsets of lymphocytes, wherein each subset expresses at least one transgene. The composition may comprises four genetically-modified subsets of lymphocytes, wherein each subset expresses at least one transgene. The composition may comprise five or more genetically-modified subsets of lymphocytes, wherein each subset expresses at least one transgene.

The composition may comprise at least two genetically-modified subsets of lymphocytes, wherein each subset expresses at least two transgenes and wherein each subset shares one transgene. The composition may comprise at least two genetically-modified subsets of lymphocytes, wherein each subset expresses at least two transgenes and wherein each subset expresses different transgenes.

In some embodiments, the plurality of lymphocytes may include: (i) a first subset expressing at least two transgenes; and (ii) a second subset expressing at least two transgenes, wherein at least one of the transgenes of the first subset is different from the transgenes of the second subset or wherein at least one of the transgenes of the first subset is in common with the transgenes of the second subset. In some embodiments, the composition of lymphocytes may express three transgenes after combining the first subset and the second subset.

As disclosed herein, (i) the first subset may express at least a PD-1 decoy or a variant/fragment thereof and an IL-2 variant/fragment and the second subset may express at least a PD-1 decoy or a variant/fragment thereof and LIGHT or a variant/fragment thereof; (ii) the first subset may express at least a PD-1 decoy or a variant/fragment thereof and an IL-2 variant/fragment and the second subset may express at least a PD-1 decoy or a variant/fragment thereof and IL-33 or a variant/fragment thereof; (iii) the first subset may express at least a PD-1 decoy or a variant/fragment thereof and an IL-2 variant/fragment and the second subset may express at least a PD-1 decoy or a variant/fragment thereof and CD40L or a variant/fragment thereof; (iv) the first subset may express at least a PD-1 decoy or a variant/fragment thereof and LIGHT or a variant/fragment thereof and the second subset may express at least a PD-1 decoy or a variant/fragment thereof and IL-33 or a variant/fragment thereof; or (v) the first subset may express at least a PD-1 decoy or a variant/fragment thereof and LIGHT or a variant/fragment thereof and the second subset may express at least a PD-1 decoy or a variant/fragment thereof and CD40L or a variant/fragment thereof; or (vi) the first subset may express at least a PD-1 decoy or a variant/fragment thereof and IL-33 or a variant/fragment thereof and the second subset may express at least a PD-1 decoy or a variant/fragment thereof and CD40L or a variant/fragment thereof.

In some embodiments, the first subset or the second subset further expresses tEGFR or a variant thereof, tHER2 or a variant thereof, CD20 or a variant thereof, or CD19 or a variant thereof.

As disclosed herein, (i) the first subset may express at least the PD-1 decoy or the variant/fragment thereof, tEGFR or the variant/fragment thereof and an IL-2 variant/fragment and the second subset may express at least the PD-1 decoy or the variant/fragment thereof, tEGFR or the variant/fragment thereof and LIGHT or the variant/fragment thereof; (ii) the first subset may express at least the PD-1 decoy or the variant/fragment thereof, tEGFR or the variant/fragment thereof and an IL-2 variant/fragment and the second subset may express at least the PD-1 decoy or the variant/fragment thereof, tEGFR or the variant/fragment thereof and IL-33 or the variant/fragment thereof; (iii) the first subset may express at least the PD-1 decoy or the variant/fragment thereof, tEGFR or the variant/fragment thereof and an IL-2 variant/fragment and the second subset may express at least the PD-1 decoy or the variant/fragment thereof, tEGFR or the variant/fragment thereof and CD40L or the variant/fragment thereof; (iv) the first subset may express at least the PD-1 decoy or the variant/fragment thereof, tEGFR or the variant/fragment thereof and LIGHT or the variant/fragment thereof and the second subset may express at least the PD-1 decoy or the variant/fragment thereof, tEGFR or the variant/fragment thereof and IL-33 or the variant/fragment thereof; (v) the first subset may express at least the PD-1 decoy or the variant/fragment thereof, tEGFR or the variant/fragment thereof and LIGHT or the variant/fragment thereof and the second subset may express at least the PD-1 decoy or the variant/fragment thereof, tEGFR or the variant/fragment thereof and CD40L or the variant/fragment thereof; or (vi) the first subset may express at least the PD-1 decoy or the variant/fragment thereof, tEGFR or the variant/fragment thereof and IL-33 or the variant/fragment thereof and the second subset may express at least the PD-1 decoy or the variant/fragment thereof, tEGFR or the variant/fragment thereof and CD40L or the variant/fragment thereof.

As used herein, the term "variant" refers to a first molecule that is related to a second molecule (also termed a "parent" molecule). The variant molecule can be derived from, isolated from, based on or homologous to the parent molecule. A "functional variant" of a protein as used herein refers to a variant of such protein that retains at least partially the activity of that protein. Functional variants may include mutants (which may be insertion, deletion, or replacement mutants), including polymorphs, etc. Also included within functional variants are fusion products of such protein with another, usually unrelated, nucleic acid, protein, polypeptide, or peptide. Functional variants may be naturally occurring or may be man-made.

In some embodiments, a variant of a transgene may include one or more conservative modifications. The transgene variant with one or more conservative modifications may retain the desired functional properties, which can be tested using the functional assays known in the art.

As used herein, the term "conservative sequence modifications" refers to amino acid modifications that do not significantly affect or alter the binding characteristics of the protein containing the amino acid sequence. Such conservative modifications include amino acid substitutions, additions, and deletions. Modifications can be introduced by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include: amino acids with basic side chains (*e.g*., lysine, arginine, histidine); acidic side chains (*e.g*., aspartic acid, glutamic acid); uncharged polar side chains (*e.g*., glycine, asparagine, glutamine, serine, threonine, tyrosine, cysteine, tryptophan); nonpolar side chains (*e.g*., alanine, valine, leucine, isoleucine, proline, phenylalanine, methionine); beta-branched side chains (*e.g.,* threonine, valine, isoleucine); and aromatic side chains (*e.g.,* tyrosine, phenylalanine, tryptophan, histidine) includes one or more conservative modifications. The Cas protein with one or more conservative modifications may retain the desired functional properties, which can be tested using the functional assays known in the art.

As used herein, the percent homology between two amino acid sequences is equivalent to the percent identity between the two sequences. The percent identity between the two sequences is a function of the number of identical positions shared by the sequences (*i.e.,* % homology = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available at www.gcg.com), using either a Blossum62 matrix or a PAM250 matrix, and a gap weight of 16, 14, 12, 10, 8, 6, or 4 and a length weight of 1, 2, 3, 4, 5, or 6.

The term "homolog" or "homologous," when used in reference to a polypeptide, refers to a high degree of sequence identity between two polypeptides, or to a high degree of similarity between the three-dimensional structure or to a high degree of similarity between the active site and the mechanism of action. In some embodiments, a homolog has a greater than 60% sequence identity, and more preferably greater than 75% sequence identity, and still more preferably greater than 90% sequence identity, with a reference sequence. The term "substantial identity," as applied to polypeptides, means that two peptide sequences, when optimally aligned, such as by the programs GAP or BESTFIT using default gap weights, share at least 75% sequence identity.

A peptide or polypeptide "fragment" as used herein refers to a less than full-length peptide, polypeptide or protein. For example, a peptide or polypeptide fragment can have at least about 3, at least about 4, at least about 5, at least about 10, at least about 20, at least about 30, at least about 40 amino acids in length, or single unit lengths thereof. For example, fragment may be 6, 7, 8, 9, 10, 11, 12, 13, 14, 15, 16, 17, or more amino acids in length. There is no upper limit to the size of a peptide fragment. However, in some embodiments, peptide fragments can be less than about 500 amino acids, less than about 400 amino acids, less than about 300 amino acids or less than about 250 amino acids in length.

Also within the scope of this disclosure are the variants, mutants, and homologs with significant identity to the transgene. For example, such variants and homologs may have sequences with at least about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% sequence identity with the sequences of transgenes described herein.

In some embodiments, the variant of the transgene as described is a fusion polypeptide comprising a transgene sequence fused (*e.g.,* N- or C-terminally fused) to a fusion partner. In some embodiments, the fusion partner comprises a fragment of a human immunoglobulin polypeptide sequence (*e.g*., a CH3 domain; or part or whole of an Fc region, such as IgG4Fc). For example, PD-1 or a variant/fragment thereof, IL-2 or a variant/fragment thereof, IL-33 or a variant/fragment thereof, CD40L or a variant/fragment thereof, or LIGHT a variant/fragment thereof can be N- or C-terminally fused or linked, directly or indirectly via a linker, to a fusion partner, such as an IgG4Fc or a variant/fragment thereof.

The term "fusion polypeptide" or "fusion protein" means a protein created by joining two or more polypeptide sequences together. The fusion polypeptides encompassed in this invention include translation products of a chimeric gene construct that joins the nucleic acid sequences encoding a first polypeptide with the nucleic acid sequence encoding a second polypeptide to form a single open reading frame. In other words, a "fusion polypeptide" or "fusion protein" is a recombinant protein of two or more proteins which are joined by a peptide bond or via several peptides. The fusion protein may also comprise a peptide linker between the two domains.

Immunosuppressive polypeptides known to suppress or decrease an immune response via their binding include CD47, PD-1, CTLA-4, and their corresponding ligands, including SIRPalpha, PD-L1, PD-L2, B7-1, and B7-2. Such polypeptides are present in the tumor microenvironment and inhibit immune responses to neoplastic cells. In various embodiments, inhibiting, blocking, or antagonizing the interaction of immunosuppressive polypeptides and/or their ligands via a transgene enhances the immune response of the immunoresponsive cell. In one aspect, a transgene can function as a gene knock-down for inhibitory/checkpoint molecules, including, but not limited to, PD-1, CTLA-4, LAG-3, TIGIT, VISTA, TIM-3, and CBL-B.

Co-stimulatory polypeptides known to stimulate or increase an immune response via their binding include CD28, OX-40, 4-1BB, CD27, and NKG2D and their corresponding ligands, including B7-1, B7-2, OX-40L, 4-1BBL, CD70, and NKG2D ligands. Such polypeptides are present in the tumor microenvironment and activate immune responses to neoplastic cells. In various embodiments, promoting, stimulating, or agonizing pro-inflammatory polypeptides and/or their ligands via a transgene enhances the immune response of the immunoresponsive cell.

In some embodiments, transgenes are cytokines or growth factors. The terms "growth factors" and "cytokines" mean signaling molecules that control cell activities in an autocrine, paracrine or endocrine manner. They exert their biological functions by binding to specific receptors and activating associated downstream signaling pathways, which in turn, regulate gene transcription in the nucleus and ultimately stimulate a biological response (Nicola N. Oxford; New York: Oxford University Press; 1994). Growth factors and cytokines affect a wide variety of physiological processes such as cell proliferation, differentiation, apoptosis, immunological or hematopoietic response, morphogenesis, angiogenesis, metabolism, wound healing, and maintaining tissue homeostasis in adult organisms. Historically, growth factors were thought to be biological moieties that have a positive effect on cell growth and proliferation, while cytokines were typically considered to have an immunological or hematopoietic response. However, as different lines of research have converged, it has been found that "cytokines" and "growth factors" can have similar functions, and therefore, these terms are herein used interchangeably.

*TGF-β Superfamily*: The TGF-beta superfamily includes the TGF-beta proteins, Bone Morphogenetic Proteins (BMPs), Growth Differentiation Factors (GDFs), Glial-derived Neurotrophic Factors (GDNFs), Activins, Inhibins, Nodal, Lefty, and Mülllerian Inhibiting Substance (MIS). The TGF-beta superfamily members are multifunctional regulators of various biological processes such as morphogenesis, embryonic development, adult stem cell differentiation, immune regulation, wound healing, inflammation, and cancer.
(1) BMP-like family: (BMPs (*i.e.,* BMP1-10, BMP-15), GDFs (*i.e.,* GDF1-15), AMH
(2) GDNFs Family: GDNF, Artemin, Neuturin, and Persephone
(3) TGF-β-like Family: TGF-βs (*i.e.,* TGF-β-1, TGF-β-2, TGF-β-3), Activins (*i.e.,* Activin A/AB/B, Inhibin A/B), Nodal

*Epidermal Growth (EGFs) Factors:* The EGF family members include EGF, TGF-α, Neuregulins, Amphiregulin, Betacellulin, and others. The members of the EGF family are best known for their ability to stimulate cell proliferation, differentiation, and survival. Deregulation of the members of this family and their receptors is closely associated with tumorigenesis (Herbst RS. International Journal of Radiation Oncology, Biology, Physics 2004, 59(2 Suppl):21-26).

*Platelet-Derived Growth Factors (PDGFs):* Platelet-derived growth factors (PDGFs) are potent mitogenic and chemotactic proteins. There are currently four known PDGF proteins encoded by four genes (PDGFA, PDGFB, PDGFC, and PDGFD). PDGFs are secreted as disulfide-linked homodimers or heterodimers that include PDGF-AA, PDGF-BB, PDGF-CC, PDGF-DD, and PDGF-AB. There are two known PDGF receptors with intrinsic tyrosine kinase activity; PDGFRα and PDGFRβ, both of which can form heterodimers and homodimers. Ligand binding promotes receptor dimerization, autophosphorylation, and the consequent activation of multiple downstream intracellular signaling cascades. Signaling via PDGFRα is essential for the development of the facial skeleton, hair follicles, spermatogenesis oligodendrocytes and astrocytes, as well as for the development of the lung and intestinal villi while signaling via PDGFRβ is crucial for the development of blood vessels, kidneys and white adipocytes (Heldin CH. Cell Commun Signal 2013, 11:97).

*Fibroblast Growth Factors (FGFs) Family:* In humans, twenty-two members of the FGF family have been identified, all of which are heparin-binding proteins. High-affinity interactions with cell-surface-associated heparan sulfate proteoglycans are essential for FGF signal transduction as mediated by receptor tyrosine kinases (Ornitz DM, Itoh N. Genome Biology 2001, 2(3): REVIEWS3005). FGFs are pluripotent proteins that are primarily mitogenic but also have regulatory, morphological, and endocrine effects. FGFs are involved in embryonic developmental processes (Heldin CH: Targeting the PDGF signaling pathway in tumor treatment. Cell Commun Signal 2013, 11:97), mature tissues/systems angiogenesis (Kim BS, et al. Biochemical and biophysical research communications 2014, 450(4):1333-1338), keratinocyte organization (Tsuboi R, et al. The Journal of Investigative Dermatology 1993, 101(1):49-53) and wound healing processes (Lee JG, Kay EP. Investigative Ophthalmology & Visual Science 2006, 47(4):1376-1386).

*Insulin-like Growth Factors (IGFs):* The Insulin-like Growth Factors (IGFs) are proteins with high sequence similarity to Insulin. The IGF receptor is a disulfide-linked heterotetrameric transmembrane protein with a cytoplasmic tyrosine kinase domain. There are two types of IGF receptors, IGFI-R and IGFII-R. The availability of IGFs can be regulated by IGF Binding Proteins 1-6 (Griffeth RJ, et al. Basic and clinical andrology 2014, 24:12). The primary action of IGFs is on cell growth. Indeed, most of the actions of pituitary growth hormone are mediated by IGFs, primarily IGF-1. Growth hormone stimulates many tissues, particularly the liver, to synthesize and secrete IGF-1, which in turn stimulates both hypertrophy (increase in cell size) and hyperplasia (increase in cell number) in most tissues, including bone. IGFs can also induce neuron survival, protect cartilage cells, and activate osteocytes (Brahmkhatri VP, et al. BioMed research international 2015, 2015:538019).

*Vascular Endothelial Growth Factors (VEGFs):* VEGFs are homodimeric, glycoprotein growth factors that are specific to endothelial cells (Ferrara N, Gerber HP, LeCouter. Nature Medicine 2003, 9(6):669-676). They regulate angiogenesis and vascular permeability, especially during embryogenesis, skeleton growth, and reproductive functions. They also play important roles in hematopoiesis. VEGFs signal mainly through tyrosine kinases VEGFR1 and VEGFR2 and stimulate cell survival, proliferation, migration, and/or adhesion (Ferrara N. Endocrine Reviews 2004, 25(4):581-611). Deregulation of VEGFs has been associated with tumors, intraocular neovascular disorders, and other diseases (Ferrara N, et al. Nature Medicine 2003, 9(6):669-676). Members of the VEGF gene family include VEGF/VEGF-A, VEGF-B, VEGF-C, VEGF-D, VEGF-E, VEGF-F, and Placental Growth Factor (PlGF) (Holmes DI, Zachary I. Genome Biology 2005, 6(2):209).

*Hepatocyte Growth Factors (HGFs):* HGF is secreted by mesenchymal cells and acts as a multi-functional cytokine on cells that are mainly of epithelial and endothelial origin. It regulates cell growth, cell motility, and morphogenesis by activating a tyrosine kinase signaling cascade via HGFR (Okada M, et al. Pediatric Research 2004, 56(3):336-344). HGF has been shown to have a major role in embryonic organ development, adult organ regeneration, and wound healing. Furthermore, its ability to stimulate mitogenesis, cell motility, and matrix invasion gives it a central role in angiogenesis and tumorigenesis (Sharma NS, et al. FASEB 2010, 24(7):2364-2374).

*Tumor necrosis factors (TNFs):* Cytokines that were known to be involved in tumor cell apoptosis were initially classified as Tumor Necrosis Factors (or under the TNF family). All TNF family members share a trimeric, conserved C-terminal domain called the 'TNF homology domain' or THD. Responsible for receptor binding, THD shares a ~20-30% sequence identity amongst family members. Although most ligands are synthesized as membrane-bound proteins, soluble forms can be generated by limited proteolysis (Bodmer JL, et al. Trends in Biochemical Sciences 2002, 27(1):19-26). The first two members of the family to be identified were TNFα and TNFβ. To date, 19 TNF superfamily ligands have been identified along with 32 TNF superfamily receptors. While many TNF superfamily members promote or inhibit apoptosis, they also regulate critical functions of both the innate and adaptive immune system, including natural killer cell activation, T-cell co-stimulation, and B-cell homeostasis and activation (Croft M. Nature Reviews Immunology 2009, 9(4):271-285). LIGHT (homologous to lymphotoxin, exhibits inducible expression, and competes with HSV glycoprotein D for herpes virus entry mediator, a receptor expressed by T lymphocytes) is a type II transmembrane glycoprotein of the TNF ligand superfamily. LIGHT is expressed on immature DCs and activated T cells and binds to 3 distinct receptors, herpes virus entry mediator (HVEM), lymphotoxin-β receptor (LTβR), and decoy receptor 3/TR6. Upon binding to HVEM, LIGHT costimulates T cells and accelerates proliferation and cytokine production. Another example is CD154, also called CD40 ligand or CD40L. It is a protein that is primarily expressed on activated T cells and is a member of the TNF superfamily of molecules. It binds to CD40 on antigen-presenting cells, which leads to many effects depending on the target cell type. Yet another example is Fas ligand (FasL or CD95L or CD178). Fas ligand/receptor interactions play an important role in the regulation of the immune system and the progression of cancer.

*Interleukins (ILs):* Interleukins are a large group of immunomodulatory proteins that regulate growth, differentiation, and activation of cells in the immune or hematopoietic systems during the immune response. Based on distinguishing structural features, the known ILs can be divided into four major groups that include; the IL1-like cytokines, the class I helical cytokines (IL4-like, γ-chain, and IL-6/12-like), the class II helical cytokines (IL-10-like and IL-28-like), and the IL-17-like cytokines (Table 1).

| | | |
|---|---|---|
| IL4-like | IL-3, IL-4, IL-5, IL-13, CSF2 | Four tightly packed α-helices known as "four-helix bundle" motif; shorter core helices |
| IL--6/12-like | IL-6, IL-11, IL-12A, IL-23A, IL-27A, IL-31, CLCF1, CNTF, CTF1, LIF, OSM, CSF3 | Four tightly packed α-helices known as "four-helix bundle" motif; longer core helices |
| IL10-like | IL-10, IL-19, IL-20, IL-22, IL-24, IL-26 | "Bundle helix" structural motif-containing six or seven stacked helices |
| IL-28-like | IL-28A, IL-28B, IL-29 | "Bundle helix" structural motif-containing six or seven stacked helices |
| IL-17-like | IL-17A, IL-17B, IL-17C, IL-17D, IL-25, IL-17F | Neurotrophin-like cysteine-knot fold |
| Non-classified | IL-8, TXLNA, IL-16, IL-32, IL-34, CSF1 | Varies |

*Interferons (IFNs):* IFNs are a group of signaling proteins that are made and released by host cells in response to the presence of pathogens such as viruses, bacteria, parasites, or tumor cells. Interferons also have immunoregulatory functions; they inhibit B-cell activation, enhance T-cell activity, and increase the cellular-destruction capability of natural killer cells. More than twenty distinct IFN genes and proteins have been identified in animals, including humans. They are typically divided into two classes: Type I IFN and Type II IFN. Type I IFNs are also known as viral IFNs and include IFN-α, IFN-β, and IFN-ω. Type II IFN is also known as immune IFN (IFN-γ). The viral IFNs are induced by virus infection, whereas type II IFN is induced by mitogenic or antigenic stimuli. Most types of virally infected cells are capable of synthesizing Type I IFN in cell culture. By contrast, IFN-γ is synthesized only by certain cells of the immune system, including natural killer cells, CD4 Th1 cells, and CD8 cytotoxic suppressor cells (Samuel CE. Clinical Microbiology Reviews 2001, 14(4):778-809, table of contents).

In some embodiments, a transgene is a decoy receptor. A "decoy receptor" means a receptor that is able to recognize and bind specific growth factors or cytokines efficiently, but is not structurally able to signal or activate the intended receptor complex. It acts as an inhibitor, binding a ligand and keeping it from binding to its regular receptor.

In some embodiments, a transgene is a soluble decoy. A "soluble decoy" means a polypeptide that is expressed and secreted from a cell and that binds to a specific receptor on a different cell, therefore, inhibiting the binding of its native ligand to such receptor. Non-limiting examples of soluble decoys are PD1-decoy, CTLA-4 decoy, LAG3-decoy, VEGFR1 decoy, TIM3 decoy, TIGIT decoy, and SIRPalpha decoy. In one embodiment, PD-1 decoys are expressed and secreted by lymphoid cells, and such PD-1 decoys inhibit binding of native PD-1 on T-cells to PDL-1 on antigen-presenting cells (APCs) by occupying the binding site of PD-L1 on APCs thus inhibiting immunosuppressive signaling of T-cells and therefore enhancing the immune response of the T-cells.

*PD-1 decoy:* PD-1 is a strong negative regulator of T lymphocytes in the tumor microenvironment. In one embodiment, T cells were generated expressing a dominant-negative deletion mutant of PD-1 (a non-limiting example of a PD-1 decoy) via retroviral transduction. This PD-1 decoy increased IFN-γ secretion of antigen-specific T cells in response to tumor cells expressing the cognate antigen. In another embodiment, soluble fragments of the PD-1 ectodomain (a non-limiting example of a PD-1 decoy) that have higher binding affinity to PDL-1 are administered as competitive antagonists of PDL-1. Non-limiting examples of soluble PD-1 ectodomain variants are disclosed in Maute et al. PNAS 2015 Nov 24; 112(47): E6506-E6514. In yet another embodiment, a PD-1 decoy molecule comprising the ectodomain of PD1 fused to the Fc region of human IgG4 (PD-1.IgG4) can be used for enhanced tumor control *in vivo.* In another embodiment, such a PD-1 decoy can be expressed and secreted by TILs.

The PD-1 decoy, as described in this disclosure, can also be generated by computational-based rational design to develop binding and/or solubility enhanced variants of the ectodomain of PD-1. For example, single and multiple amino acid replacements predicted to increase the binding affinity of PD-1 for PD-L1 are evaluated in a recombinant soluble protein produced in a bacterial expression system. The variants can be evaluated by direct titration ELISA for binding to plate-captured PD-L1 variants of interest were then cloned into retroviral vectors for evaluation of secretion by T cells. PD-1 decoy that demonstrated poor solubility during bacterial production are discarded because typically poor solubility corresponds to no or low production by T cells.

PD-1 decoys produced by engineered human T cells also comprised an Fc portion (*e.g*., IgG4Fc) to increase avidity and stability of the protein. PD1-Fc decoy produced by primary human T cells can be evaluated in ELISA. To evaluate functionality, a co-culture assay was established in which primary human T cells co-engineered to express the A2/NY-ESO-1 T cell receptor (TCR) to allow tumor cell recognition (by lentivirus transduction) as well as the PD1-Fc decoy and the cell-surface tEGFR (encoded in a bicistronic retroviral vector). These co-transduced T cells (or control T cells comprising TCR only or PD1 decoy only) were co-cultured with target tumor cells that are PDL1^{pos}. IFNγ levels present in the co-culture supernatant were evaluated to determine the best PD-1 decoy variant (i.e., the higher the IFNγ level, the better the PD1 decoy at blocking PD-L1 on the target tumor cell surface). 4XMUT_M70 and 6XDM are among the PD1-Fc decoy variants showing high binding affinity to PD-L1 and high solubility (FIGS. 9A-D).

*Cellular Elimination Tag (CET):* The transgenes, such as PD1-Fc decoy, can be expressed constitutively from a bicistronic retroviral vector also encoding a CET, such as tEGFR, tHER2, CD20, or CD19 (FIGS. 10A-D). The purpose of the CET is four-fold. First of all, it can be used as a means of evaluating transduction efficiency and second for enriching the engineered cells (on anti-EGFR coated beads) if necessary. Third, it can be used as a means of tracking the engineered T cells in a patient post-engraftment (via FACS from drawn blood samples or tumor biopsies). And finally, it can be used as an elimination tag via ADCC in the event of toxicity in a patient with Cetuximab. A truncated human EGFR polypeptide (huEGFRt) that is devoid of extracellular N-terminal ligand binding domains and intracellular receptor tyrosine kinase activity but retains the native amino acid sequence, type I transmembrane cell surface localization, and a conformationally intact binding epitope for pharmaceutical-grade anti-EGFR monoclonal antibody, cetuximab (Erbitux) is described in Want *et al.* (Wang X, et al. Blood. 2011 Aug 4;118(5):1255-63. Epub 2011 Jun 7). Other examples of CETs ADCC may include tHER2 (with Herceptin or Kadcyla), CD20 (with Rituximab), and CD19. CD20 as a CET is described in Griffioen *et al.* (Griffioen M, et al. Haematologica. 2009 Sep;94(9):1316-20). CD19 as a CET is described in Budde *et al.* (Budde, et al. Blood 2013; 122 (21): 1660) and Annesley *et al.* (Annesley et al., Blood 2019; 134 (Supplement_1): 223).

*LIGHT:* LIGHT is a type II transmembrane glycoprotein of the TNF ligand superfamily (Mauri et al. Immunity 1998 Jan; 8(1):21-30). It is expressed on immature dendritic cells and activated T cells (Tamada K et al. J Immunol. 2000 Apr 15; 164(8):4105-10) and binds to 3 distinct receptors, herpes virus entry mediator (HVEM), lymphotoxin-β receptor (LTβR), and decoy receptor 3/TR6. Upon binding to HVEM, LIGHT costimulates T cells and accelerates proliferation and cytokine production (Tamada et al. Nat Med. 2000 Mar; 6(3):283-9). LIGHT protein may be engineered to express and secreted from TILs.

*IL-33:* Cytokines are central mediators between cells in the inflammatory tumor microenvironment, in which Interleukin-33 (IL-33) is considered as an alarmin released after cellular damage. IL-33 was discovered as a member of the IL-1 family of cytokines. The IL-1 gene family contains 11 members (IL-1α, IL-1β, IL-1RA, IL-18, IL-36RA, IL-36α, IL-37, IL-36β, IL-36γ, IL-38, IL-33), which induces a complex network of pro-inflammatory cytokines and regulates and initiates inflammatory responses, via expressing integrins on leukocytes and endothelial cells (Interleukin-1 in the pathogenesis and treatment of inflammatory diseases. (Dinarello CA., Blood. 2011 Apr 7; 117(14):3720-32). The process of tumor development can trigger anti-tumor immune responses. The type 1 immune response is a critical component of cell-mediated immunity, which includes tumor-induced IFN-γ-producing Th1 cells, cytotoxic T lymphocytes, NK T cells, and γδ T cells, to limit tumor growth and metastasis (Galon J et al. Science. 2006 Sep 29; 313(5795):1960-4.). Since inflammation is another important component in malignancies, IL-33 can play roles in improving cancerous surveillance and immunity against tumors. IL-33 may be engineered to express and secreted from TILs.

*IL-2:* Interleukin-2 (IL-2) was one of the first cytokines discovered to be molecularly characterized. It was primarily shown to support the growth and expansion of T and NK cells. IL-2 was approved for clinical use in 1992, but the precise description of the biology of its receptor is still under study. Systemic high dose (HD) IL-2 treatment produces durable responses in melanoma and renal cancer carcinoma patients, but only in a relatively small fraction of patients. Moreover, systemic HD IL-2 treatments induce significant toxicities, further limiting its clinical relevance. IL-2 promotes the activation and expansion of T cells and NK cells *in vitro.* In one embodiment, IL-2 or its functional variants can be engineered to express and secreted from TILs. Such TILs can further be engineered to secrete additional transgenes.

*CD40L:* As immune co-stimulatory molecules, CD40 and its ligand CD40L can complement each other. Previous studies have shown that CD40 and CD40L play pivotal roles in humoral and cellular immunity, and the expression of CD40 and CD40L are closely related to the occurrence and development of various diseases (Elgueta et al. Immunol Rev 2009; 229:152-172). CD40 was found to be highly expressed in bladder cancer, breast cancer, ovarian cancer, and other tumors (Hussain et al. Br J Cancer 2011; 88:586). CD40L, as the primary ligand of CD40, is mainly expressed on the surface of activated CD4+ T cells. When CD40 binds CD40L, CD40L can activate T lymphocytes and the Fas-mediated apoptotic pathway in tumor cells.

In another aspect, the above-described genetically-modified lymphocytes can be incorporated into pharmaceutical compositions suitable for administration. The pharmaceutical compositions generally comprise substantially isolated/purified lymphocytes and a pharmaceutically acceptable carrier in a form suitable for administration to a subject. Pharmaceutically-acceptable carriers are determined in part by the particular composition being administered, as well as by the particular method used to administer the composition. The pharmaceutical compositions are generally formulated in full compliance with all Good Manufacturing Practice (GMP) regulations of the U.S. Food and Drug Administration.

The terms "pharmaceutically acceptable," "physiologically tolerable," as referred to compositions, carriers, diluents, and reagents, are used interchangeably and include materials are capable of administration to or upon a subject without the production of undesirable physiological effects to the degree that would prohibit administration of the composition. For example, "pharmaceutically-acceptable excipient" includes an excipient that is useful in preparing a pharmaceutical composition that is generally safe, non-toxic, and desirable, and includes excipients that are acceptable for veterinary use as well as for human pharmaceutical use.

Examples of such carriers or diluents include, but are not limited to, water, saline, Ringer's solutions, dextrose solution, and 5% human serum albumin. The use of such media and compounds for pharmaceutically active substances is well known in the art. Except insofar as any conventional media or compound is incompatible with the disclosed composition, use thereof in the compositions is contemplated. In some embodiments, a second therapeutic agent, such as an anti-cancer or anti-tumor, can also be incorporated into pharmaceutical compositions.

Pharmaceutical compositions suitable for injectable use include sterile aqueous solutions (where water-soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate-buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, *e.g*., water, ethanol, polyol (*e.g*., glycerol, propylene glycol, and liquid polyethylene glycol, and the like), and suitable mixtures thereof. The proper fluidity can be maintained, *e.g*., by the use of a coating such as lecithin, by the maintenance of the required particle size in the case of dispersion, and by the use of surfactants.

In some embodiments, the composition includes the genetically-modified lymphocytes as described above and optionally a cryo-protectant (*e.g*., glycerol, DMSO, PEG).

The composition or the pharmaceutical composition described herein can be provided in a kit. The kit may include (a) a container that contains the composition and optionally (b) informational material. The informational material can be descriptive, instructional, marketing or other material that relates to the methods described herein and/or the use of the agents for therapeutic benefit. For example, kits may include instruction for the manufacturing, for the therapeutic regimen to be used, and periods of administration. The kit may include also includes an additional therapeutic agent (*e.g.,* a checkpoint modulator). The kit may comprise one or more containers, each with a different reagent. For example, the kit includes a first container that contains the composition and a second container for the additional therapeutic agent.

The containers can include a unit dosage of the pharmaceutical composition. In addition to the composition, the kit can include other ingredients, such as a solvent or buffer, an adjuvant, a stabilizer, or a preservative.

The kit optionally includes a device suitable for administration of the composition, e.g., a syringe or other suitable delivery device. The device can be provided pre-loaded with one or both of the agents or can be empty, but suitable for loading.

### B. METHODS FOR PREPARING THE COMPOSITIONS

In another aspect, this disclosure further provides a method of preparing the above-described composition. The method comprises: (a) providing a plurality of lymphocytes; (b) introducing to the plurality of lymphocytes a nucleic acid molecule encoding at least two transgenes to obtain a plurality of genetically-modified lymphocytes; and (c) expanding the plurality of genetically-modified in a cell culture medium.

The method may include: (a) providing a plurality of lymphocytes; (b) introducing to the plurality of lymphocytes two or more nucleic acid molecules, each of the two or more nucleic acid molecules encoding at least one transgene, thereby obtaining a plurality of genetically-modified lymphocytes; and (c) expanding the plurality of genetically-modified in a cell culture medium.

The transgenes may comprise two or more of a PD-1 decoy, an IL-2 variant/fragment, LIGHT or a variant/fragment thereof, IL-33 or a variant/fragment thereof, and CD40L or a variant/fragment thereof. The transgenes may further comprise tEGFR or a variant/fragment thereof. The PD-1 decoy or the variant/fragment thereof and the tEGFR or the variant/fragment thereof (or the tHER2 or a variant/fragment thereof, CD20 or a variant/fragment thereof, or CD19 or a variant/fragment thereof) may be harbored on the same vector.

The at least two transgenes may comprise: (a) the PD-1 decoy or the variant thereof and tEGFR or the variant thereof; (b) the PD-1 decoy or the variant thereof and the IL-2 variant; (c) the PD-1 decoy or the variant thereof and the LIGHT or the variant thereof; (d) the PD-1 decoy or the variant thereof and the IL-33 or the variant thereof; (e) the PD-1 decoy or the variant thereof and the CD40L or the variant thereof; (f) the PD-1 decoy or the variant thereof, the IL-2 variant, and the IL-33 or the variant thereof; (g) the PD-1 decoy or the variant thereof, the tEGFR or the variant thereof, and the IL-2 variant; (h) the PD-1 decoy or the variant thereof, the tEGFR or the variant thereof, and the LIGHT or the variant thereof; (i) the PD-1 decoy or the variant thereof, the tEGFR or the variant thereof, and the IL-33 or the variant thereof; (j) the PD-1 decoy or the variant thereof, the tEGFR or the variant thereof, and the CD40L or the variant thereof; (k) the PD-1 decoy or the variant thereof, the tEGFR or the variant thereof, the IL-2 variant, and the IL-33 or the variant thereof; (l) the PD-1 decoy or the variant thereof, the tEGFR or the variant thereof, the IL-2 variant, and the CD40L or the variant thereof; or (m) the PD-1 decoy or the variant thereof, the tEGFR or the variant thereof, the IL-33 variant and the CD40L or the variant thereof.

The method may include: (a) introducing to a first plurality of lymphocytes a first nucleic acid molecule encoding at least two transgenes to obtain a first plurality of genetically-engineered lymphocytes; and (b) introducing to a second plurality of lymphocytes a second nucleic acid molecule encoding at least two transgenes to obtain a second plurality of genetically-engineered lymphocytes. The method may further comprise combining the first plurality of genetically-engineered lymphocytes with the first plurality of genetically-engineered lymphocytes at a predetermined ratio between about 1:1 and about 1:100 (*e.g.,* 1:1, 1:2, 1:5, 1:10, 1:20, 1:30, 1:40, 1:50, 1:60, 1:70, 1:80, 1:90, 1:100).

The method may include: a) introducing transgenes in different lymphocytes subsets, wherein each subset expresses at least one transgene, and b) combining at least two subsets of lymphocytes. Each subset may express at least two transgenes according to the embodiments described above. The composition of lymphocytes may express at least three different transgenes.

Methods to obtain a composition of tumor-specific genetically-modified subsets of lymphocytes described above can be performed *in vitro* or *ex vivo.* Methods in more particular form may be as disclosed in PCT/EP2018/080343.

The method may additionally include expanding the first plurality of lymphocytes in a cell culture medium following the step of introducing the first nucleic acid or expanding the second plurality of lymphocytes in a cell culture medium following the step of introducing the second nucleic acid.

The term "culturing" or "expanding" refers to maintaining or cultivating cells under conditions in which they can proliferate and avoid senescence. For example, cells may be cultured in media optionally containing one or more growth factors, *i.e.,* a growth factor cocktail. In some embodiments, the cell culture medium is a defined cell culture medium. The cell culture medium may include neoantigen peptides. Stable cell lines may be established to allow for the continued propagation of cells.

### a. Lymphocytes

Prior to the expansion and genetic modification of the lymphocytes described herein, a source of lymphocytes from a subject is obtained. Lymphocytes can be obtained from several sources, including peripheral blood mononuclear cells, bone marrow, lymph node tissue, umbilical cord blood, thymus tissue, tissue from an infection site, ascites, pleural effusion, splenic tissue, and tumors. As described herein, any number of lymphocyte lines available in the art can be used. Lymphocytes can be obtained from a unit of blood collected from a subject using any number of techniques known to the person skilled in the art, such as the Ficoll^{™} separation. Circulating blood cells of an individual are obtained by apheresis. The apheresis product typically contains lymphocytes, including T lymphocytes, monocytes, granulocytes, B lymphocytes, other nucleated white blood cells, red blood cells, and platelets. The cells harvested by apheresis can be washed to remove the plasma fraction and place the cells in a suitable buffer or medium for the subsequent processing steps. The cells may be washed with phosphate-buffered saline (PBS). Alternatively, the wash solution may lack calcium and may lack magnesium or may lack many, if not all, divalent cations. As those of ordinary skill in the art would readily appreciate, a washing step can be achieved by methods known to those skilled in the art, such as using a semiautomatic continuous flow centrifuge (*e.g*., the Cobe 2991 cell processor, the Baxter CytoMate , or e1Haemonetics Cell Saver 5) according to the manufacturer's instructions. After washing, the cells can be resuspended in a variety of biocompatible buffers, such as, for example, Ca2+ free, PBS free Mg2+, PlasmaLyte A, or other saline solution with or without buffer. Alternatively, the undesirable components of the apheresis sample can be removed and the cells resuspended directly in a culture medium.

As described herein, lymphocytes may be isolated from peripheral blood by lysis of red blood cells and depletion of monocytes, for example, by centrifugation through a PERCOLL^{™} gradient or by countercurrent centrifugal elutriation. If needed, specific subpopulation lymphocytes, such as T lymphocytes (*i.e.,* Cd3 +, CD28 +, CD4 +, CD8 +, CD45RA + or CD45RO + T lymphocytes) can be further isolated by positive or negative selection techniques. For example, T lymphocytes may be isolated by incubation with conjugated anti-CD3 / anti-CD28 beads (*i.e.,* 3x28), such as DYNABEADS^{®} M-450 CD3 / CD28 T, for a sufficient period of time (*i.e*., 30 minutes to 24 hours) for positive selection of the desired T lymphocytes. For the isolation of T lymphocytes from patients with leukemia, the use of longer incubation times, such as 24 hours, can increase cellular performance. Longer incubation times can be used to isolate T lymphocytes in any situation where there are few T lymphocytes compared to other cell types, such as isolating tumor-infiltrating lymphocytes (TILs) from tumor tissue or from immunocompromised individuals. The person skilled in the art will recognize that multiple rounds of selection may also be used. It may be desirable to perform the selection procedure and use the "unselected" cells in the activation and expansion process. "Unselected" cells can also undergo new rounds of selection.

Enrichment of a population of lymphocytes (*e.g*., T lymphocytes) by negative selection can be performed with a combination of antibodies directed to unique surface markers for the negatively selected cells. One method is the sorting and/or selection of cells by negative magnetic immune adherence or flow cytometry using a cocktail of monoclonal antibodies directed to cell surface markers present in the negatively selected cells. For example, to enrich CD4+ cells by negative selection, a monoclonal antibody typically includes antibodies against CD14, CD20, CD11b, CD16, HLA-DR, and CD8. Alternatively, the regulatory T lymphocytes are depleted by anti-C25 conjugate beads or other similar selection method.

Lymphocytes for stimulation can also be frozen after a washing step. Wishing not to be bound by theory, freezing and the following thawing step provide a more uniform product by eliminating granulocytes and, to some extent, monocytes in the cell population. After the washing step that removes the plasma and platelets, the cells can be suspended in a freezing solution. Although many solutions and freezing parameters are known in the art and will be useful in this context, one method involves the use of PBS containing 20% DMSO and 8% human serum albumin, or culture medium containing 10% dextran 40 and 5% dextrose human albumin and 7.5% DMSO or 31.25% Plasmalyte A, 31.25% dextrose 5%, 0.45% NaCl, 10% dextran 40 and 5% of dextrose, 20% serum of human albumin and 7.5% of DMSO or other suitable cell freezing medium containing for example Hespan and PlasmaLyte A. The cells may then be frozen at -80°C at a rate of 1°C per minute and stored in the vapor phase of a liquid nitrogen storage tank. Other methods of controlled freezing can be used, as well as uncontrolled freezing immediately at -20°C or in liquid nitrogen.

The cryopreserved cells may be thawed and washed as described herein and allowed to stand for one hour at room temperature before activation using the methods of the present disclosure. As described herein, lymphocytes can be expanded, frozen, and used later. As described herein, samples may be collected from a patient shortly after the diagnosis of a particular disease as described herein, but before any treatment. The cells may be isolated from a blood sample or an apheresis of a subject before any number of relevant treatment modalities, including but not limited to treatment with agents such as natalizumab, efalizumab, antiviral agents, chemotherapy, radiation, immunosuppressive agents such as cyclosporine, azathioprine, methotrexate, mycophenolate and FK506, antibodies or other immunoablatories such as CAMPATH, anti-CD3 antibodies, cytoxane, fludarabine, cyclosporin, FK506, rapamycin, mycophenolic acid, steroids, FR901228, and irradiation. These drugs inhibit calcium-dependent calcineurin phosphatase (e.g., ciclosporin and FK506) or inhibit p70S6 kinase that is important for signaling induced by the growth factor (rapamycin) (Liu et al., Cell 66: 807-815, 1991; Henderson et al., Immun 73: 316-321, 1991, Bierer et al., Curr. Opin. Immun., 5: 763-773, 1993). The cells may be isolated from a patient and frozen for later use together with (*e.g*., before, simultaneously or after) bone marrow or stem cell transplant, therapy with T lymphocyte ablation using chemotherapeutic agents such as fludarabine, radiotherapy external beam (XRT), cyclophosphamide, or antibodies such as OKT3 or CAMPATH. As described herein, the cells may be isolated before and can be frozen for later use in the treatment after therapy with ablation of B lymphocytes, such as agents that react with CD20, for example, Rituxan.

Either before or after the genetic modification of lymphocytes (*e.g*., T lymphocytes) to express a desirable transgene, lymphocytes can be activated and expanded generally using methods such as those described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and the publication of US patent application. No. 20060121005.

### b. Vectors

Transgenes can be introduced into lymphoid cells using various methods. These methods include, but are not limited to, transduction of cells using integration-competent gamma-retroviruses or lentivirus, and DNA transposition.

A wide variety of vectors can be used for the expression of the transgenes. The ability of certain viruses to infect cells or enter cells via receptor-mediated endocytosis, and to integrate into a host cell genome and express viral genes stably and efficiently have made them attractive candidates for the transfer of foreign nucleic acids into cells. Accordingly, a viral vector may be used to introduce a nucleotide sequence encoding one or more transgenes or fragment thereof into a host cell for expression. The viral vector may comprise a nucleotide sequence encoding one or more transgenes or fragment thereof operably linked to one or more control sequences, for example, a promoter. Alternatively, the viral vector may not contain a control sequence and will instead rely on a control sequence within the host cell to drive expression of the transgenes or fragment thereof. Non-limiting examples of viral vectors that may be used to deliver a nucleic acid include adenoviral vectors, AAV vectors, and retroviral vectors.

For example, an adeno-associated virus (AAV) can be used to introduce a nucleotide sequence encoding one or more transgenes or fragment thereof into a host cell for expression. AAV systems have been described previously and are generally well known in the art (Kelleher and Vos, Biotechniques, 17(6):1110-7, 1994; Cotten et al., Proc Natl Acad Sci USA, 89(13):6094-6098, 1992; Curiel, Nat Immun, 13(2-3):141-64, 1994; Muzyczka, Curr Top Microbiol Immunol, 158:97-129, 1992). Details concerning the generation and use of rAAV vectors are described, for example, in U.S. Pat. Nos. 5,139,941 and 4,797,368.

A retroviral expression vector can be used to introduce a nucleotide sequence encoding one or more transgenes or fragment thereof into a host cell for expression. These systems have been described previously and are generally well known in the art (Nicolas and Rubinstein, In, Rodriguez and Denhardt, eds., Stoneham: Butterworth, pp. 494-513, 1988; Temin, In: Gene Transfer, Kucherlapati (ed.), New York: Plenum Press, pp. 149-188, 1986). Examples of vectors for eukaryotic expression in mammalian cells include AD5, pSVL, pCMV, pRc/RSV, pcDNA3, pBPV, etc., and vectors derived from viral systems such as vaccinia virus, adeno-associated viruses, herpes viruses, retroviruses, etc., using promoters such as CMV, SV40, EF-1, UbC, RSV, ADV, BPV, and β-actin.

Combinations of retroviruses and an appropriate packaging line may also find use, where the capsid proteins will be functional for infecting the target cells. Usually, the cells and viruses will be incubated for at least about 24 hours in the culture medium. The cells are then allowed to grow in the culture medium for short intervals in some applications, *e.g*., 24-73 hours, or for at least two weeks, and may be allowed to grow for five weeks or more, before analysis. Commonly used retroviral vectors are "defective," *i.e.*, unable to produce viral proteins required for productive infection. Replication of the vector requires growth in the packaging cell line. The host cell specificity of the retrovirus is determined by the envelope protein, env (pl20). The envelope protein is provided by the packaging cell line. Envelope proteins are of at least three types, ecotropic, amphotropic, and xenotropic. Retroviruses packaged with ecotropic envelope protein, *e.g*., MMLV, are capable of infecting most murine and rat cell types. Ecotropic packaging cell lines include BOSC23. Retroviruses bearing amphotropic envelope protein, *e.g.,* 4070A, are capable of infecting most mammalian cell types, including human, dog, and mouse. Amphotropic packaging cell lines include PA12 and PA317. Retroviruses packaged with xenotropic envelope protein, *e.g*., AKR env, are capable of infecting most mammalian cell types, except murine cells. The vectors may include genes that must later be removed, *e.g*., using a recombinase system such as Cre/Lox, or the cells that express them destroyed, *e.g*., by including genes that allow selective toxicity such as herpesvirus TK, BCL-xs, etc. Suitable inducible promoters are activated in a desired target cell type, either the transfected cell or progeny thereof.

Non-limiting examples of the vectors useful for the present invention include retroviral vector SFG.MCS, and helper plasmids RD114, Peg-Pam3 (Arber et al. J Clin Invest 2015 Jan 2; 125(1): 157-168), lentiviral vector pRRL, and helper plasmids R8.74 and pMD2G (*e.g*., Addgene Plasmid #12259). The Sleeping Beauty transposon system can be used (Deniger et al. 2016 Mol Ther. Jun;24(6):1078-1089). Transgenes can be introduced into cells via deforming a cell as it passes through a small opening, disrupting the cell membrane and allowing material to be inserted into the cell, for example, electroporation (Xiaojun et al. 2017 Protein Cell, 8(7): 514-526), or the Cell Squeeze^{®} method. Such electroporation methods of an RNA encoding a transgene allow for transient expression of such transgene in cells which can limit toxicity and other undesirable effects of engineered cells (Barrett et al. 2011 Hum Gene Ther. Dec; 22 (12): 1575-1586).

Genome-editing techniques, such as CRISPR/Cas9 systems, designer zinc fingers, transcription activator-like effectors (TALEs), or homing meganucleases are available to induce expression of the transgenes in an immune cell. In general, "CRISPR/Cas9 system" refers collectively to transcripts and other elements involved in the expression of or directing the activity of CRISPR-associated ("Cas") genes, including sequences encoding a Cas gene, a tracr (transactivating CRISPR) sequence (*e.g*., tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. One or more elements of a CRISPR system may be derived from a type I, type II, or type III CRISPR system. Alternatively, one or more elements of a CRISPR system may be derived from a particular organism comprising an endogenous CRISPR system, such as *Streptococcus pyogenes.* In general, a CRISPR system is characterized by elements that promote the formation of a CRISPR complex at the site of a target sequence (also referred to as a protospacer in the context of an endogenous CRISPR system).

The genetic modification may be introduced by transfecting the lymphocyte cell with a vector (*e.g*., lentiviral vector) encoding one or more transgenes or a functional fragment thereof and CA9 or a functional fragment thereof. One or more transgenes or a functional fragment thereof and CA9 or a functional fragment thereof can be introduced into the immune cell using one, two, or more vectors.

Physical methods for introducing a polynucleotide into a host cell include calcium phosphate precipitation, lipofection, particle bombardment, microinjection, electroporation, and the like. Methods for producing cells comprising exogenous vectors and/or nucleic acids are well known in the art. See, for example, Sambrook et al. (2001, Molecular Cloning: A Laboratory Manual, Cold Spring Harbor Laboratory, New York).

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as an *in vitro* and *in vivo* release vehicle is a liposome (*e.g.,* an artificial membrane vesicle).

In the case where a non-viral delivery system is used, an exemplary delivery vehicle is a liposome. The use of lipid formulations is contemplated for the introduction of the nucleic acids into a host cell (*in vitro, ex vivo,* or *in vivo*). In another aspect, the nucleic acid may be associated with a lipid. The nucleic acid associated with a lipid may be encapsulated in the aqueous interior of a liposome, interspersed within the lipid bilayer of a liposome, bound to a liposome via a binding molecule that is associated with both the liposome and the oligonucleotide, entrapped in a liposome, in a complex with a liposome, dispersed in a solution containing a lipid, mixed with a lipid, combined with a lipid, contained as a suspension in a lipid, content or in a complex with a micelle, or associated otherwise with a lipid. The compositions associated with lipids, lipids/DNA or lipids/expression vector are not limited to any particular structure in solution. For example, they can be present in a bilayer structure, as micelles, or with a "collapsed" structure. They can also be simply interspersed in a solution, possibly forming aggregates that are not uniform in size or shape. Lipids are fatty substances that can be natural or synthetic lipids. For example, lipids include fatty droplets that occur naturally in the cytoplasm as well as the class of compounds containing long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; Dicetylphosphate ("DCP") can be obtained from K & K Laboratories (Plainview, NY); Cholesterol ("Choi") can be obtained from Calbiochem-Behring; dimyristyl phosphatidylglycerol ("DMPG") and other lipids can be obtained from Avanti Polar Lipids, Inc. (Birmingham, AL). Lipid stock solutions in chloroform or chloroform/methanol can be stored at about -20°C. Chloroform is used as the sole solvent since it evaporates more easily than methanol. "Liposome" is a generic term that encompasses a variety of unique and multilamellar lipid vehicles formed by the generation of bilayers or closed lipid aggregates. Liposomes can be characterized as having vesicular structures with a bilayer membrane of phospholipids and an internal aqueous medium. Multilamellar liposomes have multiple layers of lipids separated by an aqueous medium. They form spontaneously when phospholipids are suspended in an excess of aqueous solution. The lipid components undergo self-rearrangement before the formation of closed structures and trap dissolved water and solutes between the lipid bilayers (Ghosh et al., 1991 Glycobiology 5: 505-10). However, compositions that have different structures in solution than the normal vesicular structure are also included. For example, lipids can assume a micellar structure or simply exist as nonuniform aggregates of lipid molecules. Lipofectamine-nucleic acid complexes are also contemplated.

Regardless of the method used to introduce exogenous nucleic acids into a host cell, the presence of the recombinant DNA sequence in the host cell can be confirmed by a series of tests. Such assays include, for example, "molecular biology" assays well known to those skilled in the art, such as Southern and Northern blot, RT-PCR and PCR; biochemical assays, such as the detection of the presence or absence of a particular peptide, for example, by immunological means (ELISA and Western blot) or by assays described herein to identify agents that are within the scope of the invention.

### C. METHODS OF TREATMENT

This disclosure further provides a method of treating cancer or a tumor. The method comprises administering a therapeutically effective amount of a composition or a pharmaceutical composition, as described above, to a subject in need thereof.

As used herein, the terms "subject" and "patient" are used interchangeably irrespective of whether the subject has or is currently undergoing any form of treatment. As used herein, the terms "subject" and "subjects" may refer to any vertebrate, including, but not limited to, a mammal (*e.g.,* cow, pig, camel, llama, horse, goat, rabbit, sheep, hamsters, guinea pig, cat, dog, rat, and mouse, a non-human primate (for example, a monkey, such as a cynomolgus monkey, chimpanzee, etc.) and a human). The subject may be a human or a non-human. In more exemplary aspects, the mammal is a human.

In some embodiments, the subject is a human. In some embodiments, the subject has a cancer. In some embodiments, the subject is immune-depleted.

As used to describe the present invention, "cancer," "tumor," and "malignancy" all relate equivalently to hyperplasia of a tissue or organ. If the tissue is a part of the lymphatic or immune system, malignant cells may include non-solid tumors of circulating cells. Malignancies of other tissues or organs may produce solid tumors. The methods of the present invention may be used in the treatment of lymphatic cells, circulating immune cells, and solid tumors.

Cancers that can be treated include tumors that are not vascularized or are not substantially vascularized, as well as vascularized tumors. Cancers may comprise non-solid tumors (such as hematologic tumors, *e.g*., leukemias and lymphomas) or may comprise solid tumors. The types of cancers to be treated with the compositions of the present invention include, but are not limited to, carcinoma, blastoma and sarcoma, and certain leukemias or malignant lymphoid tumors, benign and malignant tumors and malignancies, *e.g*., sarcomas, carcinomas, and melanomas. Also included are adult tumors/cancers and pediatric tumors/cancers.

Hematologic cancers are cancers of the blood or bone marrow. Examples of hematologic (or haematogenous) cancers include leukemias, including acute leukemias (such as acute lymphocytic leukemia, acute myelocytic leukemia, acute myelogenous leukemia, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high-grade forms), myeloma Multiple, Waldenstrom's macroglobulinemia, heavy chain disease, myelodysplastic syndrome, hairy cell leukemia, and myelodysplasia.

Solid tumors are abnormal masses of tissue that usually do not contain cysts or liquid areas. Solid tumors can be benign or malignant. The different types of solid tumors are named for the type of cells that form them (such as sarcomas, carcinomas, and lymphomas). Examples of solid tumors, such as sarcomas and carcinomas, include fibrosarcoma, myxosarcoma, liposarcoma, chondrosarcoma, osteosarcoma and other sarcomas, synovium, mesothelioma, Ewing tumor, leiomyosarcoma, rhabdomyosarcoma, colon carcinoma, lymphoid malignancy, pancreatic cancer, breast cancer , gastric cancer, oesophageal cancer, pancreatic cancer, lung cancer, ovarian cancer, endometrial cancer, cervical cancer, prostate cancer, hepatocellular carcinoma, squamous cell carcinoma, basal cell carcinoma, adenocarcinoma, carcinoma of the sweat gland, medullary thyroid carcinoma, papillary thyroid carcinoma, sebaceous gland carcinoma of pheochromocytomas, carcinoma papillary, papillary adenocarcinomas, medullary carcinoma, bronchogenic carcinoma, renal cell carcinoma, hepatoma, bile duct carcinoma, choriocarcinoma, Wilms tumor, cervical cancer, testicular tumor, seminoma, bladder carcinoma, melanoma, and CNS tumors (such as glioma) (such as brainstem glioma and mixed gliomas), glioblastoma (also astrocytoma, CNS lymphoma, germinoma, medulloblastoma, Schwannoma craniopharyngioma, ependymoma, pinealoma, hemangioblastoma, acoustic neuroma, oligodendroglioma, meningioma, neuroblastoma, retinoblastoma, and brain metastasis).

In some embodiments, the cancer is selected from the group consisting of melanoma, sarcoma, ovarian cancer, prostate cancer, lung cancer, bladder cancer, MSI-high tumors, head and neck tumors, kidney cancer, and breast cancer.

The pharmaceutical compositions, as described, can be administered in a manner appropriate to the disease to be treated (or prevented). The amount and frequency of administration will be determined by factors such as the condition of the patient, and the type and severity of the patient's disease, although appropriate dosages can be determined by clinical trials.

When "an immunologically effective amount," "an effective antitumor quantity," "an effective tumor-inhibiting amount" or "therapeutic amount" is indicated, the precise amount of the compositions of the present invention to be administered can be determined by a physician having account for individual differences in age, weight, tumor size, extent of infection or metastasis, and patient's condition (subject). It can generally be stated that a pharmaceutical composition comprising the lymphocytes described herein can be administered at a dose of 10⁴ to 10⁹ cells/kg body weight, *e.g*., 10⁵ to 10⁶ cells/kg body weight, including all values integers within these intervals. The lymphocyte compositions can also be administered several times at these dosages. The cells can be administered using infusion techniques that are commonly known in immunotherapy (*see,* for example, Rosenberg et al., New Eng. J. of Med. 319: 1676, 1988). The optimal dose and treatment regimen for a particular patient can be readily determined by one skilled in the art of medicine by monitoring the patient for signs of the disease and adjusting the treatment accordingly.

The administration of the present compositions can be carried out in any convenient way, including infusion or injection (*i.e.,* intravenous, intrathecal, intramuscular, intraluminal, intratracheal, intraperitoneal, or subcutaneous), transdermal administration, or other methods known in the art. Administration can be once every two weeks, once a week, or more often, but the frequency may be decreased during a maintenance phase of the disease or disorder. In some embodiments, the composition is administered by intravenous infusion.

In certain cases, the cells activated and expanded using the methods described herein, or other methods known in the art wherein the lymphocytes are expanded to therapeutic levels, are administered to a patient together with (*e.g*., before, simultaneously or after) any number of relevant treatment modalities. Also described herein, the lymphocytes can be used in combination with chemotherapy, radiation, immunosuppressive agents, such as cyclosporin, azathioprine, methotrexate, mycophenolate, and FK506, antibodies, or other immunoablating agents such as CAMPATH, anti-cancer antibodies. CD3 or other antibody therapies, cytoxine, fludarabine, cyclosporine, FK506, rapamycin, mycophenolic acid, steroids, FR901228, cytokines, and irradiation.

The compositions of the present invention can also be administered to a patient together with (*e.g*., before, simultaneously or after) bone marrow transplantation, therapy with T lymphocyte ablation using chemotherapy agents such as fludarabine, radiation therapy external beam (XRT), cyclophosphamide, or antibodies such as OKT3 or CAMPATH. Also described herein, the compositions can be administered after ablative therapy of B lymphocytes, such as agents that react with CD20, for example, Rituxan. For example, subjects may undergo standard treatment with high-dose chemotherapy, followed by transplantation of peripheral blood stem cells. In certain cases, after transplantation, the subjects receive an infusion of the expanded lymphocytes, or the expanded lymphocytes are administered before or after surgery.

In some embodiments, the method may further include administering to the subject a second therapeutic agent. The second therapeutic agent is an anti-cancer or anti-tumor agent. In some embodiments, the composition is administered to the subject before, after, or concurrently with the second therapeutic agent, including chemotherapeutic agents and immunotherapeutic agents.

In some embodiments, the method further comprises administering a therapeutically effective amount of an immune checkpoint modulator. Examples of the immune checkpoint modulator may include PD1, PDL1, CTLA4, TIM3, LAG3, and TRAIL. The checkpoint modulators may be administered simultaneously, separately, or concurrently with the composition of the present invention.

A "chemotherapeutic agent" is a chemical compound useful in the treatment of cancer. Examples of chemotherapeutic agents include alkylating agents such as thiotepa and cyclophosphamide (CYTOXANTM); alkyl sulfonates such as busulfan, improsulfan and piposulfan; aziridines such as benzodopa, carboquone, methyldopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (*e.g.* calicheamicin, see, *e.g.,* Agnew Chem. Intl. Ed. Engl. 33:183-186 (1994); dynemicin, including dynemicin A; an esperamicin; as well as neocarzinostatin chromophore and related chromoprotein enediyne antibiotic chromomophores), aclacinomysins, actinomycin, authramycin, azaserine, bleomycins, cactinomycin, carabicin, caminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; podophyllinic acid; 2-ethylhydrazide; procarbazine; PSK^{®}.; razoxane; rhizoxin; sizofuran; spirogermanium; tenuazonic acid; triaziquone; 2,2',2''-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, *e.g*. paclitaxel (TAXOL^{®}, Bristol-Myers Squibb Oncology, Princeton, N.J.) and doxetaxel (TAXOTERE^{®}, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; capecitabine; and pharmaceutically acceptable salts, acids or derivatives of any of the above. Also included in this definition are anti-hormonal agents that act to regulate or inhibit hormone action on tumors such as anti-estrogens, including, for example, tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, xeloda, gemcitabine, KRAS mutation covalent inhibitors and goserelin; and pharmaceutically acceptable salts, acids or derivatives of any of the above. Additional examples include irinotecan, oxaliplatinum, and other standard colon cancer regimens.

An "immunotherapeutic agent" may include a biological agent useful in the treatment of cancer. In some embodiments, the immunotherapeutic agent may include an immune checkpoint inhibitor (*e.g*., an inhibitor of PD-1, PD-L1, TIM-3, LAG-3, VISTA, DKG-α, B7-H3, B7-H4, TIGIT, CTLA-4, BTLA, CD160, TIM1, IDO, LAIR1, IL-12, or combinations thereof). Examples of immunotherapeutic agents include atezolizumab, avelumab, blinatumomab, daratumumab, cemiplimab, durvalumab, elotuzumab, laherparepvec, ipilimumab, nivolumab, obinutuzumab, ofatumumab, pembrolizumab, cetuximab, and talimogene.

### D. DEFINITIONS

To aid in understanding the detailed description of the compositions and methods according to the disclosure, a few express definitions are provided to facilitate an unambiguous disclosure of the various aspects of the disclosure. Unless otherwise defined, all technical and scientific terms used herein have the same meaning as commonly understood by one of ordinary skill in the art to which this disclosure belongs.

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

As used herein, "expression" refers to the process by which a polynucleotide is transcribed from a DNA template (such as into an mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

As used herein, the term "recombinant" refers to a cell, microorganism, nucleic acid molecule or vector that has been modified by the introduction of an exogenous nucleic acid molecule or has controlled expression of an endogenous nucleic acid molecule or gene. , Deregulated or altered to be constitutively altered, such alterations or modifications can be introduced by genetic engineering. Genetic alteration includes, for example, modification by introducing a nucleic acid molecule encoding one or more proteins or enzymes (which may include an expression control element such as a promoter), or addition, deletion, substitution of another nucleic acid molecule. , Or other functional disruption of, or functional addition to, the genetic material of the cell. Exemplary modifications include modifications in the coding region of a heterologous or homologous polypeptide derived from the reference or parent molecule or a functional fragment thereof.

By "transgene" or "therapeutic transgene," it is meant a molecule selected from the group consisting of a soluble receptor, a decoy, a decoy receptor, a dominant negative, a microenvironment modulator, an enzyme, an oxidoreductase, a transferase, a hydrolases, a lysases, an isomerase, a translocase, a kinase, a transporter, a modifier, a molecular chaperone, an ion channel, an antibody, a cytokine, a growth factor, a chemokine, a hormone, a DNA, a ribozyme, a biosensor, an epigenetic modifier, a transcriptional factor, a coding RNA, a non-coding RNA, a small-RNA, a long-RNA, an IRES element, or an exosomal-shuttle RNA.

The term "functional variant" as used herein refers to a modified transgene having substantial or significant sequence identity or similarity to a wild type transgene, such functional variant retaining the biological activity of the wild type transgene of which it is a variant. In some embodiments, functional variants of transgenes are used.

The term "antigen recognizing receptor," as used herein, refers to a receptor that is capable of activating an immune cell *(e.g.,* a T-cell) in response to antigen binding. Exemplary antigen recognizing receptors may be native or genetically engineered TCRs, or genetically engineered TCR-like mAbs (Hoydahl et al. Antibodies 2019 8:32) or CARs in which a tumor antigen-binding domain is fused to an intracellular signaling domain capable of activating an immune cell *(e.g.,* a T-cell). T-cell clones expressing native TCRs against specific cancer antigens have been previously disclosed (Traversari et al., J Exp Med, 1992 176:1453-7; Ottaviani et al., Cancer Immunol Immunother, 2005 54:1214-20; Chaux et al., J Immunol, 1999 163:2928-36; Luiten and van der Bruggen, Tissue Antigens, 2000 55:149-52; van der Bruggen et al., Eur J Immunol, 1994 24:3038-43; Huang et al., J Immunol, 1999 162:6849-54; Ma et al., Int J Cancer, 2004 109:698-702; Ebert et al., Cancer Res, 2009 69:1046-54; Ayyoub et al. J Immunol 2002 168:1717-22; Chaux et al., European Journal of Immunology, 2001 31:1910-16; Wang et al., Cancer Immunol Immunother, 2007 56:807-18; Schultz et al., Cancer Research, 2000 60:6272-75; Cesson et al., Cancer Immunol Immunother, 2010 60:23-25; Zhang et al., Journal of Immunology, 2003 171:219-25; Gnjatic et al., PNAS, 2003 100:8862-67; Chen et al., PNAS, 2004). In one embodiment, such TCRs can be sequenced and genetically engineered into TILs for use in adoptive cell therapy. In certain aspects, TCRs that recognize MAGE-A1 antigen, MAGE -A3 antigen, MAGE A-10 antigen, MAGE-C2 antigen, NY-ESO-1 antigen, SSX2 antigen, and MAGE-A12 antigen can be genetically engineered into TILs for use in adoptive cell therapy. In yet other embodiments, genetically engineered TILs with TCRs are further engineered to secrete transgenes. In yet other embodiments, CARs are used. In other embodiments, CARs are further engineered to secrete transgenes.

As used herein, the term "antibody" means not only intact antibody molecules, but also fragments of antibody molecules that retain immunogen-binding ability. Such fragments are also well known in the art and are regularly employed both *in vitro* and *in vivo.* Accordingly, as used herein, the term "antibody" means not only intact immunoglobulin molecules but also the well-known active fragments f(ab')2, and fab. F(ab')2, and fab fragments that lack the Fe fragment of intact antibody, clear more rapidly from the circulation and may have less non-specific tissue binding of an intact antibody (Wahl et al., J. Nucl. Med. 24:316-325 (1983). The antibodies of the invention comprise whole native antibodies, bispecific antibodies; chimeric antibodies; fab, fab', single-chain v region fragments (scFv), fusion polypeptides, and unconventional antibodies.

As used herein, the term "single-chain variable fragment" or "scFv" is a fusion protein of the variable regions of the heavy (VH) and light chains (VL) of an immunoglobulin covalently linked to form a VH::VL heterodimer. The heavy (VH) and light chains (VL) are either joined directly or joined by a peptide-encoding linker (*e.g.,* 10, 15, 20, 25 amino acids), which connects the n-terminus of the VH with the C-terminus of the VL, or the C-terminus of the VH with the N-terminus of the VL. The linker is usually rich in glycine for flexibility, as well as serine or threonine for solubility. Despite removal of the constant regions and the introduction of a linker, scFv proteins retain the specificity of the original immunoglobulin. Single-chain Fv polypeptide antibodies can be expressed from a nucleic acid including VH- and VL-encoding sequences as described by Huston, et al. (Proc. Nat. Acad. Sci., 85:5879-5883, 1988). See, also, US. Pat. Nos. 5,091,513, 5,132,405 and 4,956,778; and US patent publication nos. 20050196754 and 20050196754. Antagonistic scFvs having inhibitory activity have been described (see, *e.g.,* Zhao et al., Hybridoma (Larchmont) 2008 27(6):455-51; Peter et al., J cachexia sarcopenia muscle 2012 Aug. 12; Shieh et al., J Immunol 2009 183(4):2277-85; Giomarelli et al., Thromb Haemost 2007 97(6):955-63; Fife et al., J Clin Invst 2006 116(8):2252-61; Brocks et al., Immunotechnology 1997 3(3):173-84; Moosmayer et al., Ther Immunol 1995 2(10:31-40). Agonistic scFvs having stimulatory activity have been described (see, *e.g.,* Peter et al., J Bioi Chern 2003 25278(38):36740-7; Xie et al., Nat Biotech 1997 15(8):768-71; Ledbetter et al., Crit Rev Immunol 1997 17(5-6):427-55; Ho et al., Biochim Biophys Acta 2003 1638(3):257-66).

"Treating" or "treatment" as used herein refers to administration of a compound or agent to a subject who has a disorder with the purpose to cure, alleviate, relieve, remedy, delay the onset of, prevent, or ameliorate the disorder, the symptom of a disorder, the disease state secondary to the disorder, or the predisposition toward the disorder.

The term "eliciting" or "enhancing" in the context of an immune response refers to triggering or increasing an immune response, such as an increase in the ability of immune cells to target and/or kill cancer cells or to target and/or kill pathogens and pathogen-infected cells (*e.g.,* EBV-positive cancer cells).

The term "immune response," as used herein, refers to any type of immune response, including, but not limited to, innate immune responses (*e.g*., activation of Toll receptor signaling cascade), cell-mediated immune responses (*e.g.,* responses mediated by T cells (*e.g.,* antigen-specific T cells) and non-specific cells of the immune system) and humoral immune responses (*e.g.,* responses mediated by B cells (*e.g.,* via generation and secretion of antibodies into the plasma, lymph, and/or tissue fluids). The term "immune response" is meant to encompass all aspects of the capability of a subject's immune system to respond to antigens and/or immunogens (*e.g.,* both the initial response to an immunogen (*e.g.,* a pathogen) as well as acquired (*e.g.,* memory) responses that are a result of an adaptive immune response).

As used herein, the term *"in vitro"* refers to events that occur in an artificial environment, *e.g.,* in a test tube or reaction vessel, in cell culture, etc., rather than within a multi-cellular organism.

As used herein, the term *"in vivo"* refers to events that occur within a multi-cellular organism, such as a non-human animal.

The term "disease" as used herein is intended to be generally synonymous and is used interchangeably with, the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The terms "decrease," "reduced," "reduction," "decrease," or "inhibit" are all used herein generally to mean a decrease by a statistically significant amount. However, for avoidance of doubt, "reduced," "reduction" or "decrease" or "inhibit" means a decrease by at least 10% as compared to a reference level, for example, a decrease by at least about 20%, or at least about 30%, or at least about 40%, or at least about 50%, or at least about 60%, or at least about 70%, or at least about 80%, or at least about 90% or up to and including a 100% decrease (*e.g.* absent level as compared to a reference sample), or any decrease between 10-100% as compared to a reference level.

As used herein, the term "modulate" is meant to refer to any change in biological state, *i.e.,* increasing, decreasing, and the like.

The terms "increased," "increase" or "enhance" or "activate" are all used herein to generally mean an increase by a statically significant amount; for the avoidance of any doubt, the terms "increased," "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The term "effective amount," "effective dose," or "effective dosage" is defined as an amount sufficient to achieve or at least partially achieve a desired effect. A "therapeutically effective amount" or "therapeutically effective dosage" of a drug or therapeutic agent is any amount of the drug that, when used alone or in combination with another therapeutic agent, promotes disease regression evidenced by a decrease in severity of disease symptoms, an increase in frequency and duration of disease symptom-free periods, or a prevention of impairment or disability due to the disease affliction. A "prophylactically effective amount" or a "prophylactically effective dosage" of a drug is an amount of the drug that, when administered alone or in combination with another therapeutic agent to a subject at risk of developing a disease or of suffering a recurrence of disease, inhibits the development or recurrence of the disease. The ability of a therapeutic or prophylactic agent to promote disease regression or inhibit the development or recurrence of the disease can be evaluated using a variety of methods known to the skilled practitioner, such as in human subjects during clinical trials, in animal model systems predictive of efficacy in humans, or by assaying the activity of the agent in *in vitro* assays.

Doses are often expressed in relation to bodyweight. Thus, a dose which is expressed as [g, mg, or other unit]/kg (or g, mg etc.) usually refers to [g, mg, or other unit] "per kg (or g, mg etc.) bodyweight," even if the term "bodyweight" is not explicitly mentioned.

The term "agent" is used herein to denote a chemical compound, a mixture of chemical compounds, a biological macromolecule (such as a nucleic acid, an antibody, a protein or portion thereof, *e.g.,* a peptide), or an extract made from biological materials such as bacteria, plants, fungi, or animal (particularly mammalian) cells or tissues. The activity of such agents may render it suitable as a "therapeutic agent," which is a biologically, physiologically, or pharmacologically active substance (or substances) that acts locally or systemically in a subject.

The terms "therapeutic agent," "therapeutic capable agent," or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

"Combination" therapy, as used herein, unless otherwise clear from the context, is meant to encompass administration of two or more therapeutic agents in a coordinated fashion, and includes, but is not limited to, concurrent dosing. Specifically, combination therapy encompasses both co-administration (*e.g*., administration of a co-formulation or simultaneous administration of separate therapeutic compositions) and serial or sequential administration, provided that administration of one therapeutic agent is conditioned in some way on administration of another therapeutic agent. For example, one therapeutic agent may be administered only after a different therapeutic agent has been administered and allowed to act for a prescribed period of time. See, *e.g.,* Kohrt et al. (2011) Blood 117:2423.

"Sample," "test sample," and "patient sample" may be used interchangeably herein. The sample can be a sample of, serum, urine plasma, amniotic fluid, cerebrospinal fluid, cells (*e.g.,* antibody-producing cells) or tissue. Such a sample can be used directly as obtained from a patient or can be pre-treated, such as by filtration, distillation, extraction, concentration, centrifugation, inactivation of interfering components, addition of reagents, and the like, to modify the character of the sample in some manner as discussed herein or otherwise as is known in the art. The terms "sample" and "biological sample" as used herein generally refer to a biological material being tested for and/or suspected of containing an analyte of interest such as antibodies. The sample may be any tissue sample from the subject. The sample may comprise protein from the subject.

The terms "inhibit" and "antagonize," as used herein, mean to reduce a molecule, a reaction, an interaction, a gene, an mRNA, and/or a protein's expression, stability, function or activity by a measurable amount or to prevent entirely. Inhibitors are compounds that, *e.g.,* bind to, partially or totally block stimulation, decrease, prevent, delay activation, inactivate, desensitize, or down-regulate a protein, a gene, and mRNA stability, expression, function and activity, *e.g*., antagonists.

"Parenteral" administration of a composition includes, *e.g*., subcutaneous (s.c.), intravenous (i.v.), intramuscular (i.m.), or intrasternal injection, or infusion techniques.

As used herein, the term "pharmaceutical composition" refers to a mixture of at least one compound useful within the invention with other chemical components, such as carriers, stabilizers, diluents, dispersing agents, suspending agents, thickening agents, and/or excipients. The pharmaceutical composition facilitates administration of the compound to an organism.

As used herein, the term "pharmaceutically acceptable" refers to a material, such as a carrier or diluent, which does not abrogate the biological activity or properties of the composition, and is relatively non-toxic, *i.e.,* the material may be administered to an individual without causing undesirable biological effects or interacting in a deleterious manner with any of the components of the composition in which it is contained.

The term "pharmaceutically acceptable carrier" includes a pharmaceutically acceptable salt, pharmaceutically acceptable material, composition or carrier, such as a liquid or solid filler, diluent, excipient, solvent or encapsulating material, involved in carrying or transporting a compound(s) of the present invention within or to the subject such that it may perform its intended function. Typically, such compounds are carried or transported from one organ, or portion of the body, to another organ, or portion of the body. Each salt or carrier must be "acceptable" in the sense of being compatible with the other ingredients of the formulation, and not injurious to the subject. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; diluent; granulating agent; lubricant; binder; disintegrating agent; wetting agent; emulsifier; coloring agent; release agent; coating agent; sweetening agent; flavoring agent; perfuming agent; preservative; antioxidant; plasticizer; gelling agent; thickener; hardener; setting agent; suspending agent; surfactant; humectant; carrier; stabilizer; and other non-toxic compatible substances employed in pharmaceutical formulations, or any combination thereof. As used herein, "pharmaceutically acceptable carrier" also includes any and all coatings, antibacterial and antifungal agents, and absorption delaying agents, and the like that are compatible with the activity of the compound, and are physiologically acceptable to the subject. Supplementary active compounds may also be incorporated into the compositions.

It is noted here that, as used in this specification and the appended claims, the singular forms "a,'' "an," and "the" include plural reference unless the context clearly dictates otherwise.

The terms "including," "comprising," "containing," or "having" and variations thereof are meant to encompass the items listed thereafter and equivalents thereof as well as additional subject matter unless otherwise noted.

The phrases "in one embodiment," "in various embodiments," "in some embodiments," and the like are used repeatedly. Such phrases do not necessarily refer to the same embodiment, but they may unless the context dictates otherwise.

The terms "and/or" or "/" means any one of the items, any combination of the items, or all of the items with which this term is associated.

The word "substantially" does not exclude "completely," *e.g*., a composition which is "substantially free" from Y may be completely free from Y. Where necessary, the word "substantially" may be omitted from the definition of the invention.

As used herein, the term "approximately" or "about," as applied to one or more values of interest, refers to a value that is similar to a stated reference value. The term "approximately" or "about" may refer to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value). Unless indicated otherwise herein, the term "about" is intended to include values, *e.g*., weight percents, proximate to the recited range that are equivalent in terms of the functionality of the individual ingredient, the composition, or the embodiment.

It is to be understood that wherever values and ranges are provided herein, all values and ranges encompassed by these values and ranges, are meant to be encompassed within the scope of the present invention. Moreover, all values that fall within these ranges, as well as the upper or lower limits of a range of values, are also contemplated by the present application.

As used herein, the term "each," when used in reference to a collection of items, is intended to identify an individual item in the collection but does not necessarily refer to every item in the collection. Exceptions can occur if explicit disclosure or context clearly dictates otherwise.

The use of any and all examples, or exemplary language (*e.g*., "such as") provided herein, is intended merely to better illuminate the invention and does not pose a limitation on the scope of the invention unless otherwise claimed. No language in the specification should be construed as indicating any non-claimed element as essential to the practice of the invention.

All methods described herein are performed in any suitable order unless otherwise indicated herein or otherwise clearly contradicted by context. In regard to any of the methods provided, the steps of the method may occur simultaneously or sequentially. When the steps of the method occur sequentially, the steps may occur in any order, unless noted otherwise.

In cases in which a method comprises a combination of steps, each and every combination or sub-combination of the steps is encompassed within the scope of the disclosure, unless otherwise noted herein.

Publications disclosed herein are provided solely for their disclosure prior to the filing date of the present invention. Nothing herein is to be construed as an admission that the present invention is not entitled to antedate such publication by virtue of prior invention. Further, the dates of publication provided may be different from the actual publication dates, which may need to be independently confirmed.

### E. EXAMPLES

### EXAMPLE 1

This example describes the materials and methods used in the subsequent EXAMPLES.

### Mice and cell lines

Female C57BL/6 mice aged 6 weeks were purchased from Harlan (Harlan, Netherlands) and housed at the animal facility at the University of Lausanne (UNIL, Epalinges, Switzerland) in compliance with guidelines. C57BL/6 OT-1 CD45.1+ and C57BL/6 CD8a-/- mice are described in Hogquist KA *et al.* (Hogquist KA et al. Cell 76(1):17-27 PubMed: 8287475MGI: J:92867) and Fung-Leung WP *et al.* (Fung-Leung WP et al. Cell 65(3):443-9 PubMed: 1673361MGI: J:68956). All *in vivo* experiments were conducted in accordance and with approval from the Service of Consumer and Veterinary Affairs (SCAV) of the Canton of Vaud, Switzerland.

The B16 melanoma cell line expressing ovalbumin (B16-OVA) was previously generated by retroviral transduction of the B16.F10 cell line purchased from ATCC and was grown as a monolayer in DMEM supplemented with 10% fetal calf serum (FCS), 100 U/ml of penicillin, and 100 µg/ml streptomycin sulfate. Cells were passaged twice weekly to maintain them under exponential growth conditions and were routinely tested for mycoplasma contamination. The Phoenix Eco retroviral ecotropic packaging cell line, derived from immortalized normal human embryonic kidney (HEK) cells, was maintained in RPMI 1640-Glutamax media supplemented with 10% heat-inactivated FBS, 100 U/ml penicillin, and 100 µg/ml streptomycin sulfate.

Human embryonic kidney (HEK) 293T cells were purchased from the ATCC (CRL-3216) and cultured in RPMI 1640 Glutamax medium (Invitrogen), 10% FBS (heat-inactivated for 30 min at 56C; Gibco), 1% Penicillin/Streptomycin (ThermoFisher Scientific). HEK 293T cells were used to produce retroviral and lentiviral particles. The HLA-A2.1^{pos}/NY-ESO^{pos} melanoma cell lines Me275 and A375, and the HLA-A2.1^{pos}/NY-Eso^{neg} cell line NA8 (obtained from the UNIL Department of Oncology) were cultured in IMDM supplemented with 10% FBS and 1% Penicillin/Streptomycin.

### Design of Bi-Cistronic Expression Cassettes

The retroviral vector pMSGV1 (murine stem cell virus (MSCV)-based splice-gag vector) comprising the MSCV long terminal repeat (LTR) was used as the backbone for all the constructs. Expression cassettes typically encoded the signal peptide of a murine IgG Kappa Chain region V-III MOPC 321 *(e.g.,* Uniprot ID: P01650) (SEQ ID NO: 20) followed by the N-terminal ectodomain of murine PD-1 *(e.g.,* Uniprot ID: Q02242 residues S21-Q167, C83S) (SEQ ID NO: 1) fused to human IgG4_Fc (*e.g*., Uniprot ID: P01861.1, residues P104-K327) (SEQ ID NO: 19) referred here as PD-1.IgG4 decoy. The restriction sites AgeI and EcoRI flanked this first part at the 5' and 3' ends, respectively. The second part followed the T2A sequence and was composed by the signal peptide of murine IFN-beta (*e.g*., Uniprot ID:P01575.1) (SEQ ID NO: 39) followed by a gene-string encoding one of the following molecules: murine IL-33 *(e.g.,* Uniprot ID:Q8BVZ5.1, residues S109-I266 ) (SEQ ID NO: 27), murine LIGHT (*e.g.*, Uniprot ID:Q9QYH9.1, residues D72-V239) (SEQ ID NO: 31), murine CD40L (*e.g.*, Uniprot ID:P27548, residues M112-L260) (SEQ ID NO: 33) and no alpha mutant IL-2 *(e.g.,* Uniprot ID:P60568.1, residues A21-T153, mutations: R58A, F62A, Y65A, E82A, and C145S) (SEQ ID NO: 23). The restriction sites MluI and SalI flanked this second part at the 5' and 3' ends, respectively. Consequently, after respective cloning, the following constructs were obtained: PD-1.IgG4_T2A_IL-2^{V}, PD-1.IgG4_T2A_IL-33, PD-1.IgG4_T2A_LIGHT, and PD-1.IgG4_T2A_CD40L. All genes-strings were murine codon-optimized and synthesized by GeneArt AG, and all constructs were fully sequenced by Microsynth AG after cloning in the MSGV vector.

As shown in FIGS. 11A-C, codon-optimized gene strings encoding the PD1 decoy and truncated EGFR, separated by the picorna virus-derived 2A sequence, as well as the CD40 ligand decoy, and IL-2 variant, were ordered from GeneArt (ThermoFisher Scientific) and cloned into the retroviral vector pSFG (for constitutive expression) or pSFG-SIN (self-inactivating) for activation based gene-expression under NFAT promoter. The vectors were amplified in Stellar competent cells (E. coli HST08, #636763, Takara) and purified with plasmid mini/maxi-prep kit (Genomed) upon sequence confirmation (Microsynth AG).

A gene string encoding the HLA/A2:NY-ESO-1 peptide T cell receptor (TCR) comprising TCRα23 and TCRβ13.1 was ordered from GeneArt (ThermoFisher Scientific). The TCRα and TCRβ chains were codon-optimized and separated by the picorna virus-derived 2A sequence. The gene string was incorporated into the lentiviral vector pRRL, in which most of the U3 region of the 3' long terminal repeat was deleted, resulting in a self-inactivating 3' long terminal repeat (SIN).

### Lentivirus Production

10x10⁶ HEK 293T cells were seeded in T150 flasks with RPMI complete medium (RPMI 1640 Glutamax medium (Invitrogen) 10% FBS (Gibco), 1% Penicillin/Streptomycin). Approximately 24 hours later (at 70-80% confluency), and the cells were transfected with 7 µg pVSV-G (VSV glycoprotein expression plasmid), 18 µg of µg R874 (Rev and Gag/Pol expression plasmid), and 15 µg of pRRL transgene plasmid using a mix of 107µl of Turbofect and 2 ml of Optimem media (51985026, Invitrogen). After 30 minutes of incubation at room temperature, the DNA mixture was added on top of the cells, and the volume was adjusted up to a total of 30 ml. After 24 hours, the medium was refreshed, and the viral supernatant was harvested at 48 hours post-transfection. The viral particles were concentrated by ultracentrifugation and resuspended in 400 µl of RPMI complete media. Aliquots of virus of 100-200 µl per Eppendorf tube were prepared and stored at -80C.

### Retrovirus Production

Phoenix Eco cells were seeded at 1 × 10⁷ per T-150 tissue culture flask in 25 ml culture medium 24 hours prior to transfection with 14.4 µg of pCL-Eco Retrovirus Packaging Vector and 21.4 µg of pMSGV transfer plasmid using Turbofect (Thermo Fisher Scientific). All plasmids were purified using JETSTAR 2.0 Plasmid Maxiprep Kit (Genomed). For the transfection mixture, a 3:1 ratio of turbofect:plasmid was prepared in 2 ml of Optimem and incubated for 30 minutes at RT. Medium was then removed from T-150 flasks bearing 80-90% confluent Phoenix Eco cells, and the transfection mixture was applied and incubated for 1 minute, followed by addition of 25 ml fresh medium. The viral supernatant was harvested 48 hours post-transfection, followed by addition of 25 ml of fresh media. A second harvest was performed again 24 hours later. The viral particles in both SN were concentrated by ultracentrifugation for 2-hours at 24,000g at 4°C with a Beckman JS-24 rotor (Beckman Coulter) and suspended in 0.5 ml murine T-cell medium, then viral titer was determined. Finally, the retrovirus was aliquoted, frozen on dry ice, and stored at - 80°C.

In another example, 10x10⁶ HEK 293T cells were seeded in 17 ml RPMI, 10% fetal bovine serum (FBS, Gibco), 1% Penicillin/Streptomycin (ThermoFisher Scientific) in a T150 flask overnight at 37 degrees. The following day (at 85-95% confluency of 293T cells), a mix of 120 µl turbofect (LifeTechnologies) and 3 ml OptiMem per transfection (per T150 flask) was prepared and then combined with the retroviral plasmids: 22 µg PamPeg, 7 µg RDF-RD114, 18 µg SFG or SFG-SIN encoding the gene of interest. The medium was gently removed from the 293T cells, and the retroviral plasmid mix was pipetted onto the 293T cells. After resting 5 minutes, an additional 16 ml medium was gently added. Incubate at 37°C overnight. The next day, the medium was refreshed, and the day following (at 48 hours), the virus was harvested from the filtered supernatant by ultracentrifugation (2 hours at 24000x g). Fresh medium was added to the 293T cells for a second harvest of virus at 72 hours. Aliquots of virus on both days of 100-200 µl per Eppendorf tube were prepared and stored at -80C.

### Murine T-cell transduction

Primary murine OT-1 cells were isolated from single-cell suspensions of dissociated spleens from CD45.1+ congenic OT-1 C57BL/6 mice aged 6-10 weeks using the Pan T cell Isolation Kit II for the mouse (Miltenyi Biotec cat# 130-095-130) and cultured in RPMI 1640-Glutamax media supplemented with 10% heat-inactivated FBS, 100 U/ml penicillin, 100 µg/ml streptomycin sulfate, 1mM Pyruvate, 50 µM BME, and 10mM non-essential amino acids (T-cell medium).

The cultures were maintained at a cell density of 0.5-1x106 cells/ml, replenished with fresh T-cell media every other day until day 15 (media was supplemented with 10 IU/ml of human no alpha mutant IL-2 alone until day 3 and then together with 10 ng/ml of hIL-7/IL-15). On day 7, the cell expression of the molecules was assessed by intracellular flow-cytometric analysis, and their presence in the supernatant was assessed by ELISA. Finally, engineered OT-1 T cells were adjusted according to the transduction efficiency of the PD-1.IgG4 decoy prior to cell transfer. Recombinant human IL-7 and human IL-15 were obtained from Miltenyi Biotec.

Isolated naive OT-1 T cells were plated at 1x10⁶/ml in 24-well plates in T-cell medium and stimulated with αCD-3/αCD-28 Ab-coated beads (Invitrogen) and 10 IU/ml human no alpha mutant IL-2. Twenty-four hours post-activation, T cells were transduced for the first time with retrovirus at a *multiplicity* of infection (MOI) of 10. This transduction was performed in non-tissue culture grade 24-well plates (Becton Dickinson Labware) pre-coated overnight at 4°C with 20 mg/ml of recombinant retronectin (RetroNectin; Takara), washed, blocked with 2% bovine serum albumin (BSA) in PBS for 30-minutes at RT, and then given a final wash. Following addition of the retrovirus (250 µl), the plates were centrifuged at 2000xg for 1.5-hours at 32 °C. 125 µl of supernatant was aspirated, and 1x10⁶ of activated T cells were transferred to each coated well. The plates were centrifuged for 10 min at 1200 rpm and incubated overnight at 37°C, 5% of CO₂. The second transduction was done at 48-hours post activation following the protocol explained above. On day 7, the cell expression of the molecules was assessed by intracellular flow-cytometric analysis, and their presence in the supernatant by ELISA. Finally, engineered OT-1 T cells were adjusted based on the PD-1.IgG4 decoy expression prior to cell transfer.

The cultures were maintained at a cell density of 0.5-1x10⁶ cells/ml and replenished with fresh T-cell media every other day until day 15 following an *in vitro* expansion protocol optimized to generate CD44+CD62L+TCF1+ central memory CD8 T cells. T cell media was supplemented with 10 IU/ml of human no alpha mutant IL-2 alone until day 3 and then together with 10 ng/ml of hIL-7/IL-15 until the end of the culture. Recombinant human IL-7 and human IL-15 were obtained from Miltenyi Biotec.

### Human T-cell purification and activation

Healthy donor apheresis and buffy coats were purchased from the Transfusion Interrégionale CRS SA, Epalinges, Switzerland, with written consent under an approved University Institutional Review Board protocol. Peripheral blood mononuclear cells (PBMCs) were prepared using Lymphoprep (StemCell Technologies) density gradient centrifugation, and CD8⁺ or CD4⁺ T cells were negatively isolated using CD8 or CD4 magnetic Microbeads (Miltenyi), following the manufacturer's protocol. Isolated CD8⁺ and CD4⁺ T cells were stimulated with anti-CD3/CD28 beads (Invitrogen) at a 2:1 Beads: T cell ratio in the presence of human IL-2 (GlaxoSmithKline).

### Human T-cell lentiviral and retroviral transduction

Lentiviral transduction of T cells was performed 24 hours post-activation by direct addition of the viral particles in the culture medium (MOI 20) and enhanced by concurrent addition of Lentiboost (Sirion Biotech). Retroviral transduction of T cells was performed 48h post-activation. T cells were transferred to retronectin-coated plates previously spinoculated with retroviral particles at 2000×g for 1.5 hours. T cells were removed from retronectin-coated plates the next day. The antiCD3/antiCD28 beads (Thermo Fisher Scientific) were removed 5 days post-activation, and the T cells were maintained thereafter in RPMI 1640-Glutamax (Thermo Fisher) supplemented with 10% heat-inactivated FBS (Gibco), 1% Penicillin/Streptomycin, 10 ng/ml human IL-7 (Miltenyi), and 10 ng/ml IL-15 (Miltenyi) at 0.5-1x10⁶ T cells/ml.

### Human T-cell co-transduction with lentivirus and retrovirus

For co-transduction, human T cells were purified and bead-activated (Per 48-well: 0.5x10⁶ T cells + 1x10⁶ antiCD3/antiCD28 beads + 501U/ml IL-2) for 18-22 hours prior to the addition of concentrated lentivirus (100 µl), and optionally also 1 µl Lentiboost (Sirion Biotech) to enhance transduction efficiency. The next day, the transduced T cells were transferred to retronectin-coated plates previously spinoculated with retroviral particles at 2000×g for 1.5 hours. The following day, the T cells were transferred to a tissue culture plate. On day five the beads were removed, and the T cells were transferred to larger wells and provided fresh medium supplemented with 10 ng/ml IL-15 and 10 ng/ml IL-7. The provision of fresh medium plus cyotokines was performed every 2-3 days. From day 7-10, co-transduction efficiency can be determined by flow cytometry.

### Retrovirus transduction of tumor-infiltrating lymphocytes (TILs)

Defrost TILs were previously expanded from dissociated patient tumor fragments. 0.5x10⁶ TILs were stimulated in 48-well plates in 500 µl RPMI, 10% FBS plus 25 µl GMP-grade TransAct (1:20, Miltenyi Biotech) and 6000 IU/ml IL-2. A non-tissue culture plate was coated with retronectin (Takara Bio, dilute 1mg/ml 50 times, 250 µl per 48well) overnight at 4C. The next day, the retronectin was removed and blocked with 500 µl of RPMI, 10% FBS (Gibco), 1% Penicillin/Streptomycin for 30 minutes at 37°C. Subsequently, the medium was removed, and 50-100 µl of concentrated retrovirus was added in 50 µl medium, followed by spinning for 1 hour at 2000g at 25°C. Then the supernatant was removed, the TILs were added and spun for 10 minutes at 1000g at 25C. Incubate overnight at 37°C and then transfer to 48-well tissue culture plates with fresh medium. On day 5, the TILs were transferred to larger well plates and supplemented with fresh medium (RPMI, 10% FBS (Gibco), 1% Penicillin/Streptomycin, 6000IU/ml IL-2). Transduction efficiency was evaluated on day 7-10. From day 5 onwards, fresh medium was provided every 2-3 days.

### Flow cytometric analysis

All FACS data were acquired at an LCRII flow cytometer (BD) and analyzed using FlowJo software. The fixable aqua dead dyes L34965 or L34975 (Invitrogen) were used as per manufacturer's instructions for dead cell exclusion. The following antibodies were used for T cell staining: anti-Vb13.1:PE (IM2292, BD Bioscience), anti-IFNγ:PeCy7 (502527, Biolegend). Tetramer (A2/NY-ESO-1₁₅₇₋₁₆₅; produced in-house) staining was used to evaluated TCR transduction efficiency.

### Flow Cytometric analysis for evaluating the expression of immunomodulatory factors by gene-engineered T cells

One-week post-transduction gene engineered OT-1 T cells were incubated with 50 µl of Live/Dead Fixable aqua dead for 30 minutes in PBS at room temperature, washed and then incubated again with 50 µl of FCR blocking reagent (clone 2.4G2 BD Pharmingen) for 30 minutes at 4°C. Cells were washed again and incubated at 4°C for another 30 minutes with surface markers directed Abs against CD3 (145-2C11, Invitrogen), CD8α (53-6.7, BioLegend), and CD45.1 (A20, BioLegend). For intracellular staining, the following antibodies were used: anti-human hIgG4-Fc (Abcam, clone: HP6025) for detecting the PD-1.IgG4 decoy and anti-mouse IL-33 (eBioscience, clone: 396118). After surface staining, gene-engineered OT-1 cells were washed twice and fixed/permeabilized using the FoxP3 transcription factor staining buffer set (Invitrogen) according to the manufacturer's recommendations. For the detection of each molecule, the cells were further washed and incubated for 30 minutes with respective antibodies at room temperature. Cells were washed and resuspended in PBS supplemented with 2% BSA and 0.01% azide (FACS buffer) FACS buffer to be acquired with a BD flow cytometer LSRII cytometer and analyzed using FlowJo software v11 (Tree Star Inc.).

### Flow cytometric analysis to evaluate intracellular cytokine or PD1-Fc decoy or CD40L decoy production

In order to assess intracellular cytokine production or PD1 decoy or CD40L decoy production via FACS, 50,000 live T cells per well were activated with the combination of plate-coated anti-CD3 (5 µg/ml) and soluble anti-CD28 (2 µg/ml) antibody for 7 hours in round-bottom 96-well plates (or with anti-CD3/anti-CD28 beads). To prevent protein secretion, Golgi stop was added (BD Biosciences) at a dilution of 1:400 to the wells 1.5 hours after the initiation of the assay. A standard fixation/permeabilization kit (BD Biosciences) was used according to manufacturer's instructions to fix and permeabilize the T cells before assessing their transduction efficiency or their capacity to produce the molecule of interest. An anti-Fc antibody was used for detection of the decoys. Antibodies specific for the cytokine of interest (IL-2, IFN-γ) were used.

### ELISA for evaluating the secretion of immunomodulatory factors by gene-engineered T cells

One-week post activation and transduction, 106 genetically engineered OT-1 T cells were seeded in 1 ml of serum-free RPMI media for 72 hours. Then SN was harvested and tested for each molecule. For PD1.IgG4, a modified-ELISA with the following setup was used. Plates were coated with anti-mouse PD1 Ab (R&D, AF1021, 2 µg/ml), incubated with SN and PD1.IgG4 was detected with anti-hIgG4-HRP Ab (Abcam, ab99817, dilution 1:1000).

For IL-2^{V}, a modified-ELISA with the following setup was used. Plates were coated with anti-human IL-2 Ab (R&D, AF-202-NA, 3 µg/ml), incubated with supernatant, and IL-2^{V} was detected by biotinylated polyclonal anti-Human IL-2 Ab (Invitrogen, 13-7028-81, dilution 1:500) followed by streptavidin-HRP (BioLegend, dilution 1:1000). SN from OT-1 T cell transduced for expressing either the fusion molecule TIM-3.IgG4 or IL-2^{V} were used as negative controls. For detection of LIGHT, IL-33, and CD40L, three commercial ELISA kits were used: mouse LIGHT/TNFSF14 DuoSet ELISA developed by R&D (DY1794-05), LEGEND MAXTM Mouse IL-33 ELISA Kit developed by BioLegend (436407), Mouse CD40Ligand/TNFSF5 ELISA Kit developed by Novus Biological (NBP1-92662).

### Adoptive cell transfer in tumor-bearing mice

B16-OVA tumor cells were harvested with 0.05% trypsin, washed, and resuspended in PBS for injection. 1x10⁵ tumor cells were injected subcutaneously in the right flank of C57BL/6 mice, aged 7 weeks. On day 11 (average tumor volume 100-200 mm³), mice were regrouped in order to have comparative average tumor volumes between experimental arms, with n ≥ 5 mice/group. On day 12 and 15 mice were treated with i.v transfer of 5x10⁶ gene-engineered CD44+ CD62L+ TCF1+ OT-1 T cells or control non-transduced OT-1. Mice were monitored three times/week, and tumor length (L; greatest longitudinal measurement) and width (W; greatest transverse measurement) measured by caliper by an independent investigator in a blinded manner. Tumor volumes (V) were calculated using the formula: V = (L x W²)/2. The average tumor volumes/group were plotted ± SD. Mice were sacrificed once tumors reached 1000mm³, or, according to regulation, if they became distressed or moribund.

### ELISA for evaluating the secretion of immunomodulatory factors by gene-engineered T cells.

One-week post-transduction 1x10⁶ gene-engineered OT-1 T cells were seeded in a 24-well plate in 1 ml of serum-free RPMI media for 72 hours. SN was then harvested and tested for each molecule by ELISA. PD1.IgG4 homemade-ELISA: coating Ab: anti-mouse PD-1 (R&D, AF1021, 2 µg/ml), Detection Ab: anti-hIgG4-HRP (Abcam, ab99817, dilution 1:1000). IL-2^{V} home-made ELISA: coating Ab: anti-human IL-2 (R&D, AF-202-NA, 3 µg/ml), Secondary Ab biotinylated polyclonal anti-Human IL-2 (Invitrogen,13-7028-81, dilution 1:500 ), streptavidin-HRP (BioLegend, dilution 1:1000 ). SN from OT-1 T cell transduced for expressing either the fusion molecule TIM-3.IgG4 or IL-2^{V} were used as negative controls. For detection of IL-33, a commercial LEGEND MAXTM Mouse IL-33 ELISA Kit developed by BioLegend (436407) was used.

### ADCC measured by chromium release assay

Autologous PBMCs were defrosted, placed at a concentration of 1x10⁶/ml, and added in a 6-well plate at 3 ml per well in the presence of 10 ng/ml GM-CSF. The next day, 0.5x10⁶ EGFR⁺ T cells were loaded with 50 µCi Chromium-51, re-suspended, and put in a 37°C water-bath for approximately 1 hour. The T cells were then washed twice and suspended at a concentration of 400,000 cells/ml, and 50 µl of the T cells (=2,000 cells) per well was transferred. Cetuximab (anti-EGFR antibody) at 300 µg/ml or 30 µg/ml was prepared. 50 µl of Cetuximab was added to the T cells and incubated for 30 minutes at 37°C. The PBMCs (effector cells) were harvested at 1.2x10⁶ cells/ml in RPMI, 10% FBS (Gibco), 1% Penicillin/Streptomycin. 1 in 3 dilutions of the effector PBMCs (1.2x10⁶, 0.4x10⁶, 1.33x10⁶, and 0.42x10⁶ PBMC/ml) were prepared, and 50 µl was added to the wells containing tEGFR⁺ T cells plus anti-EGFR antibody. different ratios of effector :target cells (30:1, 10:1, 3:1, 1:1, in triplicate) were set up. As a positive control, 1x TritonX was added to the T cells (=maximum chromium release). All negative controls (medium only, T cells no PBMCs, T cells plus PBMCs but no antibody, etc.) were set up. The plates were spun at 1500 rpm and placed at 37°C for 4-5 hours. 50 µl of supernatant was transferred to lumaplate wells and allowed to dry overnight. The following day, chromium levels were evaluated with the topCounter.

### Co-Culture assay and ELISA to measure cytokine production

TCR-T cells co-engineered to express the PD1 decoy plus truncated EGFR (and all control T cell conditions) were prepared at a concentration of 1x10⁶ TCR⁺ T cells/ml, and tumor cells were prepared at 1x10⁶ cells/ml. 100 µl each of the T cells and the tumor cells were combined in 96-well round-bottom plates. the plates were spun for 1 minute at 1500 rpm and incubated at 37°C for 48-72 hours. Evaluate IFN-γ levels in the supernatant by ELISA (Invitrogen) according to the manufacturer's recommendation.

### Co-Culture assay and ELISA for evaluating secretion of PD1 and CD40L decoys

1x10⁶ primary UTD and co-transduced T cells (engineered to express the NY TCR and secrete the PD1 decoy plus tEGFR) were co-cultured with 1x10⁶ target cells per well in 96-well round bottom plates, in duplicate, in a final volume of 200 µL complete RPMI media. The plates were spun for 1 minute at 1500 rpm and incubate at 37C. After 24-hours, the co-culture supernatants were harvested and tested for the presence of PD1-Fc fusion decoy molecules by capture on plate-bound anti-PD1 antibody or plate-bound human PD-L1 protein. The bound PD1-Fc decoy molecules were detected by anti-IgG-Fc Ab. The same conditions were used to evaluate CD40L decoy secreted in the supernatant except that a commercial ELISA kit is used (Invitrogen).

### Immune subsets depletions, checkpoint blockade and FTY720 treatment.

Specific cellular subsets were depleted by administering 250 µg/dose of depleting antibody i.p. every three days beginning 1 day before therapy: CD4 T cells with α-mouse CD4 (clone GK1.5, BioXCell), NK cells with α-mouse NK1.1 (clone PK136, BioXCell), neutrophils with α-mouse Ly6G (clone 1A8, BioXCell). For checkpoint blockade, mice were injected i.p. every three days with 250 µg/dose of α-mouse PD-L1 (BioXcell, 10F.962) and α-mouse TIM-3 (BioXcell, RMT3-23). To block emigration of lymphocytes from secondary lymph organs, a stock solution of FTY720 (10 mg/ml in DMSO) obtained from SIGMA was prepared and then diluted to 1 mg/ml in water before administration. Finally, 100 µg of the drug was administrated i.p. every three days beginning 2 days before therapy. Both depletions and sequestration (FTY720) of immune cells were confirmed by flow cytometry of PBMC.

### Preparation of single tumor-cell suspensions, antibodies for flow cytometry and ex vivo re-stimulation for cytokine production.

Tumors were excised 5 and 12 days after the first adoptive cell transfer and dissociated into a single-cell suspension by combining mechanical dissociation with enzymatic degradation of the extracellular matrix using the commercial Tumor Dissociation kit for mouse (Miltenyi Biotec, 130-096-730). Following the single-cell suspension, 2.5x10⁶ live cells were seeded in 96-well plates and incubated with 50 µl of Live/Dead Fixable aqua dead for 30' in PBS at room temperature, then Fc receptors were blocked by incubation for 30 min. at 4°C with 50 µl of purified anti-CD16/CD32 mAb (clone 2.4G2 BD Pharmingen). Cells were then stained for 30 min. at 4°C with the fluorochrome-conjugated mAbs of interest in 50 µl of FACS Buffer. Subsequently, the cells were washed twice and fixed/permeabilized using the FoxP3 transcription factor staining buffer set (Invitrogen) for intracellular staining. Analysis of stained cells was performed using an LSRII cytometer and FlowJo software.

The following antibodies were used: CD45.1 (clone A20, BioLegend); CD3 (clone 145-2C11, Invitrogen), CD4 (clone GK1.5, BioLegend); CD8 (clone 53.6.7, BioLegend), FOXP3 (clone FJK-16S, Invitrogen), NK1.1 (clone PK136, BioLegend), CD44 (clone IM7, BioLegend), PD-1 (clone 29F.1A12, BioLegend), LY6C (clone HK1.4, BioLegend), Granzyme C (clone SFC1D8, BioLegend), TCF1 (clone C63D9, Cell Signaling Technology), anti-rabbit IgG (H+L), F(ab')₂ Fragment AF488 or PE conjugated (Cell Signaling Technology), Granzyme B (clone GB11, Novul Biological), CD69 (clone H1.2F3, BioLegend), TIM-3 (clone RMT3-23, BioLegend), CD137/4-1BB (clone 17B5, Invitrogen), KLRG1 (clone 2F1/KLRG1, BioLegend), KI67 (clone 16A8, BioLegend), IFNg (clone XM61.L, Invitrogen), TNFa (clone MP6-XT22, BioLegend), TOX (clone TXRX10, Invitrogen), CD45 (clone 30-F11, BioLegend),

Fluorescence minus one (FMO) controls were stained in parallel using the panel of antibodies with sequential omission of one antibody. FMO staining was performed as a control for the following antibodies: TCF1, Ki67, 4-1BB, Granzyme B, TNFa, IFNg, PD-1, and TIM-3. Isotype control was used for Granzyme C staining (clone HTK888, BioLegend). Precision Count Beads^{™} (BioLegend) were used to obtain absolute counts of cells during acquisition on the flow cytometer.

For the detection of cytokine production, single tumor cells suspension (2.5x10⁶ live cells) were *in vitro* re-stimulated in 24-well plates with 1 µg/ml well-coated anti-mouse CD3 (clone 17A2, Invitrogen) and 2 µg/ml of soluble anti-mouse CD28 (clone 37.51, Invitrogen) for 4h in the presence of Brefeldin A (5 µg/ml). Cells were surface stained before fixation and permeabilization as described above, which was followed by intracellular staining.

### Immunofluorescence labeling and microscopy

For immunohistochemistry analysis, tumor tissues were isolated and were fixed in 1% PFA in PBS overnight, infiltrated with 30% sucrose the next day (overnight) and then embedded and frozen in OCT compound. Cryostat sections were collected on Superfrost Plus slides (Fisher Scientific), air-dried, and preincubated with a blocking solution containing BSA, normal mouse serum, normal donkey serum (Sigma), and 0.1% triton. Then they were labeled overnight at 4°C with primary antibodies diluted in PBS with 0.1% triton. After washing with PBS with 0.1% triton, the secondary reagents were diluted in PBS with 0.1% triton and applied for 45 minutes at RT. Finally, after additional washes with PBS and 0.1% triton, DAPI (Sigma) was used to stain the nuclei, followed by a PBS wash and mounting in DABCO (homemade). Images were acquired with a Zeiss AxioImager Z1 microscope and an AxioCam MRC5 camera. Images were treated using Fiji (NIH) or Adobe Photoshop. Exposure and image processing were identical for mouse groups, which were directly compared.

Antibodies (ab) used for the CD8/CD45.1/CD105 labeling: 1° ab: Rat-a-mouse CD8a (clone 53-6.7) , Rabbit-a-mouse CD105 (clone MJ7/18), Mouse-a-mouse CD45.1 Biotin (clone A20.1). 2° reagent: Donkey-a-Rat Alexa 488 (Invitrogen, # A21208), Donkey-a-Rabbit Cy3 (Jackson ImmunoResearch # 711-165-152) , Streptavidin APC (Biolegend, #405207).

Antibodies (ab) used for the CD8-CD45.1-TCF1 labeling: 1° ab: Rat-a-mouse CD8a (53-6.7), Rabbit-a-mouse TCF-1 (Cellsignalling. clone C63D9), #2203), Mouse-a-mouse CD45.1 Biotin (clone A20.1). 2° ab: Donkey-a-Rat Alexa 488 (Invitrogen, # A21208), Donkey-a-Rabbit Cy3 (Jackson ImmunoResearch # 711-165-152.), Streptavidin APC (Biolegend, #405207).

### Single-cell RNA-seq analysis.

Aggregated UMI counts matrix generated by CellRanger was filtered in order to select high-quality CD8 TIL transcriptomes. First, cells having 500 to 5000 detected genes, 2000 to 30000 UMI counts, mitochondrial content below 5%, and % ribosomal protein content below 50 % were kept. Next, CD8 T cells were filtered as those expressing *Cd2, Cd8a* and *CD8b1* (>= 1 UMI) but not *Cd4* (0 UMI). Cells expressing Cd14, Csf1r, Cd19, Spi1, Foxp3, H2-Aa, and H2-Ab1 were further removed, and 1788 high-quality CD8 TIL transcriptomes were obtained.

For dimensionality reduction, highly variable genes (HVG) were first identified using Seurat 3.1.1 *vst* method with default parameters (Stuart et al., Cell, vol. 177, issue 7, p1888-1902.e21, June 13, 2019). Next, mitochondrial, ribosomal protein-coding genes and cell cycle genes (those bearing Gene Ontology term GO:0007049) were removed from the set of HVG, and the remaining HVG (1649) was scaled to have mean=0 and variance=1. Standardized HVG was used for a first step of dimensionality reduction using PCA, and a second set using UMAP (as implemented in Seurat v3.1.1) on the first 10 principal components (with other parameters by default). Clustering was performed using the shared nearest neighbor method of Seurat with parameters using FindNeighbors with default parameters and FindClusters with resolution=0.2. For supervised classification of CD8 TIL states, TILPRED (https://github.com/carmonalab/TILPRED; Santiago J. Carmona, et al., OncoImmunology, 9:1(2020)) was used with default parameters. Differentially expressed genes between clusters were identified using FindAllMarkers and MAST v1.10 (Finak, G., et al. Genome Biol 16, 278 (2015) with parameters min.pct=0.25 and logfc.threshold=0.25. For comparison of Gzmc cluster and conventional exhausted cluster, the origin 'exhausted' cluster was sub-clustered by increasing the 'resolution' parameter to 0.3. Differential expression analysis between the refined exhaustion cluster and Gzmc cluster was assessed using FindAllMarkers and MAST v1.10 with parameters min.pct=0.1 and logfc.threshold=0.25. Gene set enrichment analysis of these clusters vs. TOX-KO signature (Scott, A.C., et al. Nature 571, 270-274 (2019).) was calculated using GSEA function from clusterProfiler package v3.12 (Guangchuang Yu, et al., OMICS: A Journal of Integrative Biology. May 2012.284-287) with default parameters and using the top 200 differentially expressed cluster genes with p-value < 0.01 ordered by decreasing fold-change.

### Statistical analysis

Normal Distribution of data was evaluated using the Shapiro-Will normality test. A two-tailed Student's t-test was used to compare two groups (if normal distribution and homoscedasticity), or a t-test with Welch's correction (if normal distribution but not homoscedasticity), and if data were not normally distributed, the non-parametric Mann-Whitney test was used. For comparing more than two groups, a similar strategy was followed. A Kruskal Wallis Test was used if normal distribution was absent. One-way ANOVA test was used if normal distribution and homoscedasticity, or a Brown-Forsythe and Welch ANOVA test was used in case of normal distribution but not homoscedasticity. Correction for multiple comparisons was done using a Dunn's Test (for Kruskal Wallis Test), a Dunnet Test (for one-way ANOVA test), and a Tukey test (for Brown-Forsythe's test). Survival Analysis was done using a log-rank Mantel-Cox model. The Pearson correlation test was used to calculate the correlation between the number of TCF1+ OT-1 intratumoral CD8 T cells and the total number of tumor-infiltrated OT-1. All these statistical analyses were done with GraphPad Prism 8.0, *p<0.05, **p<0.01, ***p<0.001 ****p<0.0001

Statistical analysis of tumor control was performed using the change (%) of tumor volume relative to day 17 after tumor inoculation. The best response (smallest tumor volume) observed for each animal after at least 12 days post-1^{st} ACT was taken for the calculation. The Objective Response rate and Clinical Benefit rate by treatment group were calculated over the total number of mice per group, as (1) Objective Response includes Complete Response (CR; 100% reduction in tumor volume) and Partial Response (PR; ≤-30% tumor change); and (2) Clinical Benefit includes CR, PR and Stable Disease (-30%<tumor change≤+20%).

Predicted probabilities of the variables "Objective response" and "Clinical benefit" were calculated using exact logistic regression. The values of tumor change as a continuous variable were further analyzed using linear regression. P-values lower than 0.05 were considered as statistically significant.

### EXAMPLE 2

The PD1 decoy molecule was cloned in retroviral constructs containing the sequences forIL-2^{V}, LIGHT, or interleukin 33 (IL-33): each construct was codon-optimized and encoded for only two molecules separated by the self-cleaving peptide T2A. Thus, at least four different constructs were available: i) PD1.IgG4_T2A_IL-2^{V}, expressing both PD1 decoy and IL-2^{V}; ii) PD1.IgG4_T2A_LIGHT, expressing PD1 decoy and LIGHT; iii) PD1.IgG4_T2A_IL-33, expressing PD1 decoy and IL-33; and iv) PD1.IgG4_T2A_CD40L, expressing PD1 decoy and CD40L.

FIG. 1 shows the efficiency of transfection and transduction of OT-1 T cells with the designed constructs. In each tested composition, the cells showed a high efficiency of transduction and high secretion levels for each of the expressed secreted protein.

Using specific ELISAs, it has also been confirmed that engineered OT-1 T cells can secrete each particular combination of immunomodulatory factors PD1.IgG4_T2A_IL-2^{V}, PD1.IgG4_T2A_LIGHT; and PD1.IgG4_T2A_IL-33, and PD1.IgG4_T2A_CD40L.

The results show that T cells can be successfully engineered to express two different exogenous secreted proteins. These cells show advanced properties by continuing expressing and secreting a high quantity of the respective exogenous secreted proteins.

### EXAMPLE 3

As depicted in FIGS. 2A and 2B, ACT with OT-1 T cells secreting PD1.IgG4, LIGHT, and IL-2^{V} significantly improved control of large established B16-OVA tumors. This combinatorial strategy induced tumor regression after ACT, improving the overall survival when compared with response to untransduced OT-1 T cells. As depicted in FIGS. 2C and D, ACT with OT-1 T cells secreting PD1.IgG4, LIGHT, and IL-33 also significantly improved control of large established B16-OVA tumors. This combinatorial strategy showed better antitumor activity than PD1.IgG4, LIGHT combinatorial strategy. The PD1.IgG4, LIGHT, and IL-33 combinatorial strategy improved the overall survival when compared with response to untransduced OT-1 T cells.

The adoptive transfer of OT-1 T cells secreting PD1.IgG4, IL-2^{V}, and IL-33 almost doubled overall survival as compared to the administration of untransduced OT-1 T cells, and, in comparison with the other combinatorial strategies, the overall survival was extended by ten days or more (FIG. 2F). As depicted in FIGS. 2G and H, ACT with OT-1 T cells secreting PD1.IgG4, IL-2^{V}, and CD40L significantly improved control of large established B16-OVA tumors. This combinatorial strategy induced tumor regression after ACT, improving the overall survival when compared with response to untransduced OT-1 T cells.

In summary, T cells can be gene-engineered for secreting combinations of immunomodulatory factors to control advanced tumors. The above examples also highlight the therapeutic feasibility of mixing populations of T cells with the same antigen specificity but with different secretory properties for obtaining high-order combinations of immunomodulatory factors. An important advantage of this approach is increased safety as the molecules will be largely secreted in the tumor microenvironment (*i.e.,* they were not systemically applied).

### EXAMPLE 4

Adoptive immunotherapy offers opportunities to reprogram T cells and the tumor microenvironment. As demonstrated in this example, orthogonal engineering of adoptively transferred T cells with an IL-2Rβγ-binding IL-2-variant, PD1-decoy, and IL-33 led to cell-autonomous T-cell expansion, engraftment, and tumor control in immunocompetent hosts through reprogramming of both transferred and endogenous CD8⁺ cells. Tumor-infiltrating lymphocytes (TILs) adopted a novel effector state, different from canonical TOX-driven exhaustion, characterized by TOX suppression, abundance of granzyme-C, and effector molecules, survival and cell-precursor markers. Driven dynamically by an interaction between IL-2-variant and IL-33, TILs in this state uncoupled persistence from TOX-driven exhaustion and successfully controlled tumors. Rational T-cell engineering without host lymphodepletion, therefore, enables optimal reprogramming of adoptively transferred T cells as well as mobilizing endogenous immunity into new states compatible with tumor control.

It was hypothesized that T cells could be endowed with intrinsic properties that enable them to autonomously reach the necessary expansion in the absence of lymphodepletion and the desired functional state compatible with engraftment into and rejection of moderately immunogenic tumors. In this disclosure, it was sought to modify T cells with orthogonal combinatorial engineering, *i.e.,* introducing genes whose products could produce favorable perturbations that reprogram T cells and also enable T cells to reprogram adaptive and innate immunity in the TME. the PD-1/PD-L1 inhibitory pathway was targeted with a secreted PD-1 decoy (PD1d), *i.e.,* a fusion molecule comprising the ectodomain of murine PD-1 linked to the Fc region of human IgG4. To support T-cell expansion, a human IL-2 variant (IL-2^{V}) that does not engage the high-affinity IL-2Rα-chain (CD25) was employed ( T. Carmenate et al., Journal of immunology 190, 6230-6238 (2013); G. Rojas et al., Scientific Reports 9, 800 (2019).). Important advantages of this molecule as compared to wild-type IL-2 include decreased toxicity and lower sequestration by regulatory T cells (Tregs). It was also hypothesized that unlike wild-type IL-2, which drives terminal effector differentiation, IL-2^{V} would promote CD8⁺T-cell stemness, a favorable feature for ACT (J. G. Crompton, et al. Immunol Rev 257, 264-276 (2014)). Finally, to generate advantageous inflammatory signals in tumors, IL-33 was employed. Two retroviral vectors were constructed, one encoding soluble PD1d and IL-2^{V} (PD1d/2^{V} module) and a second encoding soluble PD1d and mouse IL-33 (PD1d/33 module). CD8⁺ T cells were separately transduced with a retrovirus carrying one or the other module and then pooled in a 1:1 ratio to generate an ACT cocktail endowed with the triple combination (PD1d/2^{V}/33). Also, OT1 T cells were used to treat advanced B16-OVA melanoma tumors in immunocompetent recipient mice. As shown below, this example investigates what CD8⁺T-cell state might these interventions lead to, and what might a desired CD8⁺T-cell state be to achieve T-cell engraftment and tumor regression in the absence of preconditioning lymphodepletion or exogenous cytokine support.

### Orthogonal T-cell engineering improves ACT efficacy in a cell-autonomous manner

As the initial engineering module, the antitumor potential of the PD-1 decoy was first evaluated. This molecule was well expressed and secreted by engineered OT1 cells and bound to plate-immobilized PD-L1 *in vitro* (which was outcompeted by saturating PD-L1 neutralizing antibody). ACT using PD1d-engineered OT1 cells showed significant anti-tumor activity *in vivo* in lymphodepleted (irradiated) mice. Next, whether OT1 cells could be efficiently transduced with the PD1d/2^{V} or PD1d/33 module to secrete simultaneously PD1d and IL-2^{V}, or PD1d and IL-33, respectively, was tested. To assess PD1d expression, transduction efficiencies of greater than 75% were obtained, and simultaneous secretion of all molecules by ELISA was confirmed.

Next, orthogonal-engineering ACT was conducted in the absence of preconditioning lymphodepletion (FIG. 3A). Two infusions of 5x10⁶ non-transduced OT1 cells with ~70% T_{CM} and ~30% T_{EM} (effector-memory) phenotype administered to mice with palpable (100 m³) tumors in the absence of lymphodepletion or exogenous cytokines were unable to control tumor growth (FIG. 3B). The same dose of OT1 cells transduced with either PD1d or IL-2^{V} had a small but insignificant effect on tumor growth (FIG. 3B; Tables 2 and 3), and double-engineered PD1d/2^{V}-OT1 cells did not prove more effective under the same conditions. Systemic administration of anti-(α)PD-L1 antibody with non-transduced OT1 cells produced comparable results to PD1d-OT1 cells. Similarly, OT1 cells transduced with IL-33 had minimal effect, as did PD1d/33-OT1 cells. Strikingly, the treatment with PD1d/2^{V}/33-OT1 cells (*i.e.,* 1:1 mixed PD1d/2^{V} and PD1d/33 cells) was statistically significantly superior when compared with any other treatment (FIG. 3B; Tables 2 and 3). The objective response rate (ORR) for this therapeutic approach was 85.7%, with a predicted probability of occurrence of 83.3% (Tables 2 and 3), while it was between 0 and 9% for all others. Finally, confirming the effectiveness of PD1d/2^{V}/33-OT1 cells in the absence of lymphodepletion and exogenous cytokine support, earlier treatment of mice (starting on day 6) led to complete tumor eradication and cures.

### Orthogonal engineering in immunocompetent hosts leads to cell-autonomous expansion of adoptively transferred CD8⁺ cells and contributory engagement of endogenous antitumor immunity

The antitumor effect of double-engineered PD1d/2^{V} or PD1d/33 was then compared to the triple-engineered PD1d/2^{V}/33 OT1 cells (FIG. 3A). At baseline (day 12), treatment-naive B16-OVA tumors displayed a modest spontaneous infiltration of CD8⁺ T cells exhibiting a phenotype of CD44⁺ antigen-experienced cells. Adoptively transferred CD8⁺ T cells start accumulating in tumors within 4 days, and by day 5 post-ACT (day 17), tumors of mice treated with triple-engineered cells demonstrated already significantly higher levels of CD8⁺ T-cell engraftment. One week later (day 24), it was found that significantly more CD8⁺ TILs in tumors of mice treated with PD1d/2^{V}/33-OT1 cells relative to those treated with double-engineered cells, which in turn displayed significantly more CD8⁺ TILs relative to mice receiving non-transduced OT1 cells (FIG. 3C). Focusing on OT1 (CD45.1⁺) TILs, a marked expansion of PD1d/2^{V}/33-OT1 cells in tumors two weeks post-ACT was observed (FIG. 3D), while PD1d/2^{V}-OT1 and PD1d/33-OT1 cells exhibited modest or minimal levels of engraftment, respectively. Thus, efficacy of triple-engineering combinatorial ACT was associated with a unique expansion of adoptively transferred T cells in tumors and tumor regression.

Given that ACT was performed in entirely immunocompetent hosts, whether endogenous immune effector cells contributed to its effect was investigated. Strikingly, approximately 50% or more of total CD8⁺ TILs seen two weeks post-ACT were endogenous (CD45.1^{neg} CD45.2⁺) (FIG. 3E). Even though some expansion of endogenous TILs in tumors treated with PD1d/2^{V}-OT1 or PD1d/33-OT1 cells were observed, these were particularly prominent in tumors treated with triple-engineered cells.

The presence of a pool of stem-like cells expressing the TCF1 transcription factor within the CD8⁺ T-cell compartment has been previously associated with the ability to mobilize immunity against tumors (and chronic viral infections) upon PD-1 blockade, while the use of such precursor cells for ACT may enhance efficacy. However, TME conditions do not promote the presence or persistence of TCF1⁺CD8⁺ TILs. Notably, a marked expansion of TCF1⁺OT1⁺ TILs was observed specifically following the transfer of the triple-engineered PD1d/2^{V}/33 or the double-engineered PD1d/2^{V} OT1 cells (FIGS. 3F and 3G), indicating that only these two approaches sharing IL-2^{V} expanded the stem-like compartment. Importantly, expansion of stem-like TCF1⁺CD8⁺ extended also onto endogenous TILs (FIGS. 3F and 3G). In these tumors, 10-20 % of OT1 and 30-50% of endogenous CD8⁺ TILs expressed TCF1 post PD1d/2^{V}/33-ACT, and a strong direct correlation between the presence of TCF1⁺ OT1 cells and the total number of OT1 TILs was observed. Thus, the engineering approach comprising IL-2^{V} achieved conditions that promoted stemness and, consequently, persistence of transferred as well as endogenous T cells.

Importantly, effective tumor control by gene-engineered OT1 cells was associated not only with an increased presence of TCF1⁺ CD8⁺ TILs, but also with high numbers of TCF1^{neg} effector-like CD8⁺ TILs-a condition met solely following PD1d/2^{V}/33-ACT (FIG. 3G). Indeed, in these tumors, 80-90% of OT1 TILs and 50-70% of endogenous CD8⁺ TILs were TCF1^{neg}. Importantly, although a high frequency of TCF1⁺ CD8⁺ TILs was also seen post PD1d/2^{V}-ACT, there were far fewer TCF1^{neg} CD8⁺ TILs in these tumors, indicating that IL-2^{V} drove the expansion of the TCF1⁺ CD8⁺ TILs, but in the absence of IL-33 coexpression, these cells were unlikely to transition to a TCF1^{neg} status, confirming that *Tcf1* suppression is associated with effector differentiation. By contrast, PD1d/33-ACT was associated with low TCF1⁺CD8⁺ and high TCF1^{neg}CD8⁺ TIL frequency, and overall poor TIL expansion (FIGS. 3F and 3G).

To understand the contribution of endogenous T cells in tumor control post-ACT, CD8-knockout tumor-bearing mice were treated with PD1d/2^{V}/33-OT1 cells under the same conditions. It was observed that the anti-tumor effect of ACT was lost in the absence of endogenous CD8⁺ T cells (FIG. 3H). Thus, engagement of endogenous CD8⁺ T cells was critical for effective tumor control. Strikingly, tumor control was not dependent on recruitment of endogenous T cells from lymph nodes, as evidenced by co-administration of FTY720 (a drug that impairs lymphocyte egress from the lymph nodes) (FIG. 3H). Thus, *in situ* expansion of pre-existing endogenous TILs, rather than recruitment of systemic CD8⁺T cells to tumors, was harnessed and necessary for tumor control by triple-engineered ACT.

Next, the interactions of ACT with tumor Treg were assessed. Secretion of IL-2^{V} by transferred cells preferentially expanded CD8⁺ over Treg and consistent with the largest expansion of CD8⁺ cells, the CD8/Treg ratio was highest following PD1d/2^{V}/33-ACT (FIG. 3I). Total CD4⁺ TILs globally expanded less than CD8⁺ TILs, and antibody-mediated depletion of CD4⁺ T cells prior to PD1d/2^{V}/33-ACT did not compromise but rather significantly improved tumor control and mouse survival (FIG. 3J).

Finally, whether triple-engineered ACT mobilized innate immunity was investigated. Although tumor NK cells increased post-ACT containing IL-2^{V}, especially with PD1d/2^{V}/33-ACT, they failed to activate, and they were dispensable. Importantly, tumor control was, however, co-dependent on neutrophil mobilization and activation (FIG. 3K). Thus, orthogonal engineering achieves tumor regression in immunocompetent hosts through mobilization of both adaptive and innate immunity. While there are limited examples of ACT-mediated tumor control in lymphoreplete mice bearing hematological tumors, this is the first demonstration of successful ACT in advanced, poor immunogenic solid tumors in the absence of any supportive treatment S. K. Vodnala et al., Science 363, eaau0135 (2019)).

### A novel subset of intratumoral GzmC⁺ TCF1^{neg} effector CD8⁺ T cells induced by orthogonal engineering persists independently of TOX

To learn more about the molecular states of TILs associated with tumor control upon triple-engineering ACT, as well as on the impact of the individual double-engineered modules, TILs were analyzed by single-cell (sc)RNA-seq (FIG. 4A). Unsupervised clustering analysis of CD8⁺ TILs derived from the different experimental conditions revealed five distinct transcriptomic states (clusters C1 to C5) (FIG. 4B). To interpret the results, TILPRED, a machine learning tool that assigns cells into previously described molecular TIL states identified in untreated murine tumors, was used (S. J. Carmona, et al. OncoImmunology 9, 1737369 (2020)). It was found that TILs from tumors treated with non-engineered OT1 cells displayed features similar to TILs of untreated tumors, with a predominant progenitor- and terminal-exhausted cell pool (C4) and few cycling (C3), effector-memory (C2), and naive cells (C1) (FIG. 4B and 4C). Thus, in the absence of engineering and with no host conditioning, ACT did not impact the TIL state. Following PD1d/2^{V}-ACT, TILs exhibited a predominant naive-like pool (C1) consistent with the marked expansion of TCF1⁺ cells seen above, with some additional effector-memory (C2), cycling (C3), and precursor and terminal exhausted cells (C4). Conversely, TILs exhibited a predominant effector-memory state (C2) following PD1/33-ACT, with some cycling cells (C3). Thus, the local expression of only IL-2^{V} or only IL-33 redirected TILs towards a naïve-like versus an effector-memory state, respectively. In addition, the combination of the two cytokines resulted in an entirely novel state (C5), associated with tumor control, which departed from either state supported by each cytokine separately. C5 was exclusively found in triple-engineered ACT TILs during the response phase and not seen in any other tumor condition (FIG. 4B). The novelty of this state was further supported by ProjecTILs, a tool that projects (sc)RNAseq data onto a reference TIL atlas, which revealed that while cells in clusters C1-C4 aligned to previously described reference states, C5 emerged as a novel state, never described before, and characterized by the upregulation of a unique effector-like transcriptional program (FIG. 4D).

C5 largely comprised TILs identified by both TILPRED and ProjecTILs were broadly classified as "terminal-exhausted" CD8⁺ cells, exactly as TILs in cluster C4. Indeed, cells in both clusters shared a relatively high expression of coinhibitory receptor genes, including *Pdcd1, Lag3, Tigit, Havcr2*/*TIM3,* and *Entpdl*/*CD39* and the costimulatory receptor and activation marker *Tnfsfr9*/4-1BB (FIG. 4E). Given their separation by UMAP and the up-regulation of the ProjecTII,s effector-like transcriptional program, differential expression analysis was used to identify the distinct molecular features of C5 versus C4 terminal-exhausted TILs. Relative to canonical terminal exhausted cells of C4, C5 TILs exhibited a unique effector signature, with significant downregulation of exhaustion-associated transcription factors *Tox, bhlhe40, and Batf,* as well as multiple inhibitory receptors. Notably, it also downregulated *Cx3cr1-a* distinctive marker of the transitory effector-like exhausted cells (FIG. 4C, bottom part; Table 3). In addition, C5 TILs significantly upregulated effector cell markers including multiple granzymes, most prominently *Gzmc,* which constitutes a C5 specific marker (FIG. 4E), the anti-apoptotic gene *Bcl2,* and *Ly6c2,* a marker associated with precursor CD8⁺ T cells, which is also absent in CX3CR1⁺ transitory effector-like exhausted cells. Consistently, C5 cells were enriched in the signature of *Tox*-knockout CD8 TILs when compared to C4 (FIG. 4F). Accordingly, it was confirmed by FACS that the majority of OT1 (~80%) and endogenous (~70%) CD8⁺ TIL during the response phase express granzyme-C (FIG. 4G). No GzmC⁺CD8⁺ cell was detected in spleens, indicating that local cues in the reprogrammed TME drove T cells specifically into this state in tumors. Importantly, negligible frequencies of GzmC⁺CD8⁺T cells were found in the remaining experimental groups (FIG. 4G) as well as in the endogenous CD8⁺ TILs at baseline or in OT1 cells post-expansion *in vitro,* indicating that PDd1/2^{V}/33-ACT specifically led to profound intratumoral reprogramming of TILs, including endogenous TILs, with generation of a novel CD8⁺ T-cell effector phenotype, which evidently required local interactions between IL-2^{V} and IL-33.

### TOX^{neg/}^{low} GzmC⁺ TCF1^{neg} effector CD8⁺ TILs are polyfunctional cells with inconsequential expression of coinhibitory receptors

The *GzmC⁺* effector CD8⁺ TILs state accounting for tumor rejection following PD1d/2^{V}/33-ACT was further characterized. A gating strategy was used to identify TCF1^{neg} effector CD8⁺ cells in the OT1 and endogenous compartments. It was found that the majority of OT1 and two-thirds of endogenous GzmC⁺CD8⁺ TILs post PD1/2^{V}/33-ACT were indeed TCF1^{neg} effector cells (FIG. 5A). These cells were then compared to TCF1^{neg} effector CD8⁺ TILs from other groups, when available (FIG. 5B) (which can be analyzed from PD1d/33-OT1, non-transduced OT1, and non-transduced OT1 ACT plus αPD-L1) and importantly, all these cells were GzmC^{neg} (FIG. 5A). The majority of GzmC⁺TCF1^{neg}CD8⁺ TILs from PD1d/2^{V}/33-ACT and GzmC^{neg}TCF1^{neg}CD8⁺ effector OT1 and endogenous TILs from other groups were PD-1⁺(FIG. 5C), and a marked proportion of these cells were also TIM3⁺ (FIG. 5D). Remarkably, and in agreement with the (sc)RNA-seq data, almost none of the OT1 and only ~40% of endogenous PD-1⁺GzmC⁺TCF1^{neg} cells expressed TOX (FIG. 5E). In agreement with *Ly6c2* being one of the most differentially expressed genes between C5 (GzmC⁺) and C4 (GzmC^{neg}) exhausted cells, the majority of PD1⁺GzmC⁺TCF1^{neg} OT1 and endogenous TILs displayed high expression of LY6C, a marker which is absent in terminal-exhausted CD8⁺ TILs. GzmC⁺TCF1^{neg} OT1 or endogenous TILs also expressed low/no KLRG1, a marker of short-lived effector cells (FIG. 5F). Further indicating that the TOX^{low/neg} GzmC⁺TCF1^{neg}CD8⁺ TILs from PD1d/2^{V}/33-ACT were not canonical terminal_exhausted cells, it was found that most OT1 and approximately half of the endogenous cells expressed CD69, suggesting recent TCR-induced activation (FIG. 6A). In addition, TOX^{neg/low}GzmC⁺PD-1⁺TCF1^{neg}CD8⁺ T cells from both OT1 and endogenous compartments expressed more Ki-67 than TILs from other groups (FIG. 6B). Finally, most OT1 and endogenous CD8⁺ TILs from PD1/2^{V}/33-ACT (but no other groups) were GrzmB⁺, in accordance with the (sc)RNAseq analysis (FIGS. 6C and 6D). Among these PD-1⁺ OT1 cells, an important fraction was identified that was double GzmC^{high} and GzmB^{high} (FIG. 6E), which were not detected among the endogenous cells. Finally, PD-1⁺CD8⁺ TILs collected during tumor regression from PD1d/2^{V}/33-ACT were interrogated for cytokine response to *ex-vivo* CD3/CD28 stimulation. Approximately half of OT1 and endogenous TILs produced TNFα upon stimulation, and a proportion of cells produced both TNFα and IFNγ (FIG. 6F), demonstrating polyfunctional effector properties. Thus, triple engineering ACT yields a unique phenotype of powerful tumor-rejecting CD8⁺ effector TILs that do not acquire the TOX program.

To test whether under the unique TIL state achieved, coinhibitory receptors like PD-1 or TIM-3 are decoupled from the TOX exhaustion program and whether their inhibitory function is inconsequential, PD1d/2^{V}/33-ACT were combined with αPD-L1 or double αPD-L1/αTIM3 antibody. However, no improvement in tumor control was observed (FIGS. 6G and 6H). It was thus reasoned that PD-1 blockade was entirely dispensable in the context of IL-2^{V}/IL-33 combinatorial-engineering ACT. Indeed, removing the PD-1 ectodomain from the PD-1_IgG4 decoy did not affect tumor control by ACT that only had the active 2^{V}/33 modules (FIG. 6I). These data collectively indicate that orthogonal engineering of T cells with a βγ-binding IL-2 and IL-33 in the immunocompetent host enabled the generation of a novel effector TIL state endowed with the ability of controlling tumors, in which TOX remains suppressed and coinhibitory receptors are expressed but are inconsequential.

### Orthogonal engineering drives TOX^{neg/}^{low} GzmC⁺ precursor differentiation

Similar to chronic viral infection, CD8⁺ T-cell mediated anti-tumor response (also following PD-1 blockade) is likely maintained by intratumoral TCF1⁺PD-1⁺ precursor-exhausted CD8⁺ T cells with stem-like properties, which express TOX, a transcription factor that is critical to their generation and persistence. Given the role of IL-2^{V} in the ACT context to suppress TOX in TCF1^{neg} cells, whether IL-2^{V} also suppressed the TOX program in TCF1⁺ precursors was investigated. During the response phase post PD1d/IL-2^{V}/33-ACT, important numbers of TCF1⁺CD8⁺ TILs were detected (FIGS. 3F and 3G). All of these cells expressed GzmC (FIG. 5A), and most were also PD-1⁺ (FIG. 7A), but these (both OT1 and endogenous) were mostly TOX^{neg} (FIGS. 7B and 7C). Thus, upon orthogonal-engineering ACT, tumor-responsive TCF1⁺ precursors already deactivate the TOX program and upregulate GzmC (FIG. 7D). Importantly, downregulation of TOX in TCF1⁺PD-1⁺CD8⁺ TILs post PD1d/2^{V}-ACT was also found, while marked expression in the same TIL subset post PD1/33-ACT indicates that IL-2^{V} suppresses TOX at the level of the TCF1⁺ CD8⁺ precursor state.

After PD1d/IL-2^{V}-ACT, a significant frequency of PD-1^{neg}TCF1⁺ precursors (both OT1 and endogenous) was identified (FIG. 7A). These were also TOX^{neg} and exhibited features of antigen-experienced T_{CM} or T_{EM} cells. Thus, in the presence of IL-2^{V}, tumor-specific TCF1⁺ TILs are not exclusively PD-1⁺precursor-exhausted cells. However, in the presence of IL-33 alone, 40% of endogenous TCF1⁺PD-1^{neg}CD8⁺ TILs were TOX⁺ and mostly T_{EM} cells. Thus, it was concluded that orthogonal engineering reprograms the TCF1⁺ stem-like compartment towards suppression of the TOX program and upregulation of a transcriptional program heralded by expression of GzmC. Importantly, the two programs appear to be independent. IL-2^{V} was the key factor supporting stemness and persistence in a TOX-independent manner, while the combination with IL-33 was required to trigger also activation of the GzmC⁺ differentiation program in precursor CD8⁺ TILs.

### Dynamic evolution of the GzmC⁺ effector state

Finally, the dynamic evolution of the GzmC⁺ TIL state following triple-engineered ACT was assessed. (sc)RNA-seq data from CD8⁺ TILs collected 5 days post-ACT (day 17) upon tumor response (day 24) and from tumors that escaped following initial response to PD1d/2^{V}/33-ACT (day 38) were compared (FIG. 8A). Adding the escape timepoint to all previous TILs did not alter the previously described cell annotation and cluster distribution. Thus, early post-ACT, TILs were distributed mainly in the proliferating (C3), effector-memory (C2), and exhausted pool (C4) (FIG. 8B). By day 12 post ACT, cells had migrated within the new C5 state, associated with tumor regression. Interestingly, subsequent progression was associated with migration of TILs away from C5 and to a new C6 state (FIG. 8B). The new adaptation state at tumor escape was indeed different from the C5 GzmC⁺ effector state associated with rejection (FIGS. 8B and 8C). As expected from the inability of PD-1 (or TIM-3) blockade to control tumors (FIGS. 6G, 6H, and 6I), escaping cells were distant from exhausted terminally differentiated cells of C4 and expressed markedly lower TOX than these cells (FIG. 8B). Clustering of PD1d/2^{V}/33 samples alone increased resolution, showing that C6 cells were closer to but distinct from the canonical effector-memory C2 state observed early or during response post-ACT and displayed lower *Fosl2, Bcl2, Gzma, Gzmb,* and *Tnfrsj9* (CD137) (FIG. 8B). Consistently, the ProjecTILs analysis revealed that escape-phase C6 state deviated from the reference map effector-memory state, down-regulating a *Fosl2*-driven effector gene program. Thus, escape was not mediated by canonical exhaustion, and cells retained at least in large part the TOX suppression program characteristic of the PD1d/2^{V}/33-ACT, but lost expression of GzmC (and GzmB).

The above observations were validated by flow cytometry. Following triple-engineered ACT, the hallmark GzmC⁺CD8⁺ TIL population of cluster 5 markedly expanded in tumors from day 5 to day 12, coinciding with tumor regression (FIG. 8D), while these cells were lost upon tumor escape. Also, a marked reduction in OT1 cells upon tumor progression was observed (FIG. 8E), which was associated with a contraction of the TCF1^{neg} population of both OT1 and endogenous TILs (FIG. 8F). Consistent with the C6 state, residual CD8⁺ TILs displayed a PD-1^{neg/lo} T_{EM}-like CD8⁺ phenotype, with significant downregulation of Granzyme-B relative to *in vitro* expanded T_{EM}-like CD8⁺ T cells. Finally, TCF1^{neg} PD-1⁺CD8⁺ T cells harvested during escape exhibited loss of both GranzymeB expression and polyfunctionality relative to TCF1^{neg} PD-1⁺CD8⁺ T cells harvested during tumor control (FIGS. 8G and 8H). Thus, It was concluded that the optimal TIL effector state is dynamically associated with tumor response.

### Discussion

This example demonstrates that orthogonal combinatorial T-cell engineering in the context of solid tumor ACT can successfully overcome homeostatic barriers in the host and lead - in the absence of lymphodepletion or exogenous cytokine support - to profound reprogramming of TILs and the tumor microenvironment and tumor regression. Conducting ACT in the immune-competent host offers unique advantages not only because it may dramatically reduce the toxicity and costs of present-time ACT, but it can also leverage the full spectrum of host immunity. This example further shows that under these circumstances, both endogenous CD8⁺ cells (specifically pre-existing CD8⁺ TILs) as well as neutrophils were mobilized against tumors and were essential for achieving tumor regression.

Recent studies using high-dimensional computational analysis have uncovered the existence of multiple types of CD8⁺ states - naïve-like, effector-memory, cytotoxic, and exhausted - in human and mouse tumors ( S. J. Carmona, et al. Oncolmmunology 9, 1737369 (2020)). While the cytotoxic TIL state observed mainly in human samples is mostly enriched in bystander cells, there is substantial evidence showing that the exhausted compartment is enriched in tumor-specific CD8 TILs ( A. M. van der Leun, et al. Nature Reviews Cancer 20, 218-232 (2020)). Notably, this compartment is highly heterogeneous, formed by a continuum of cell states hierarchically organized along a differentiation axis as either precursors or terminal exhausted CD8⁺ T cells. Although immune checkpoint blockade (ICB) has achieved an important level of clinical responses to date, its effect is primarily based on inducing changes in exhausted CD8 T cells states that already existed before therapy rather than inducing novel, non-exhausted, effector-like states (J.-C. Beltra et al., Immunity 52, 825-841.e828 (2020)). Thus, pharmacological reprogramming of CD8 TILs towards such "desired" effector states represents an effective strategy to improve clinical response to current immunotherapy.

T-cell engineering offers unlimited opportunity to rationally reprogram TILs and, in a paracrine manner, the TME. This example demonstrates orthogonal engineering using an IL-2 variant engaging only the βγ-chain receptor, together with PD-1 blockade to stimulate CD8⁺ T-cell and IL-33, a potent innate immunity activator, to reprogram the TME. This combination led to the adoption by both exogenous and endogenous TILs, of a novel effector state, distinguished by unique expression of multiple granzymes (most prominent granzyme-C) and suppression of TOX - a transcription factor that is critical for the generation and maintenance of exhausted CD8⁺ T-cell populations during chronic viral infection and in cancer (A. C. Scott et al., Nature 571, 270-274 (2019)). This state was reproducibly associated with significant local CD8⁺ T-cell expansion in the TME, potent effector function, and effective tumor control. Under this novel program, PD-1 and other coinhibitory receptors were still expressed, demonstrating that their upregulation in the context of sustained antigen stimulation is not strictly TOX-dependent. In addition, pharmacological blockade of PD-1 and TIM3 pathways corroborated that their expression was functionally inconsequential.

The CD8⁺ T-cell exhausted program is stably enforced in the TCF1⁺ progenitor compartment by expression of TOX. However, the TCF1⁺ CD8 state expanded by orthogonal engineering remained TOX-negative and upregulated granzyme-C, a marker never detected in the canonical precursor exhausted compartment. Thus, the progenitor-like cell state induced by the therapy diverges from the TCF1⁺TOX⁺ precursor cell state that has been consistently described in the context of chronic viral infection and in cancer. In a similar way, the polyfunctional GzmC⁺TCF1^{neg}PD-1⁺TOX^{low/neg} effector-like state expanded by orthogonal engineering diverges not only from the canonical terminal exhausted cell state (TOX⁺PD-1⁺ CX3CR1^{neg}GzmC^{neg}), but also from the transitory effector-like exhausted state (CX3CR1⁺ TIM3⁺PD-1⁺). Indeed, although TOX is downregulated in this state, it arises from canonical GzmC^{neg}TCF1⁺ precursor exhausted cells and does not significantly upregulate *Gzmc* ( J.-C. Beltra et al., Immunity 52, 825-841.e828 (2020)). Furthermore, it expresses *Cx3cr1* and *Klrg1* that are significantly downregulated in the *GzmC⁺* effector state induced by orthogonal engineering. In addition, CD4⁺ T- help cell is required for the formation of the CX3CR1⁺ effector states. However, these cells are deleterious in the context of the approach. Therefore, it was hypothesized that both TCF1⁺ and TCF1^{neg} GzmC⁺CD8⁺ TILs represent the precursor and effector states, respectively, of a novel TOX-independent CD8⁺ T-cell differentiation program.

The therapeutic manipulation of TOX has emerged as a promising strategy to abrogate T-cell exhaustion in the context of cancer. Recently, it has been demonstrated that TOX knockdown, or deletion of *TOX2,* improves the functionality of CAR-T cells, while heterozygous deletion of *TOX* strengthens anti-tumor T-cell responses ( H. Seo et al., Proceedings of the National Academy of Sciences 116, 12410-12415 (2019); O. Khan et al., Nature 571, 211-218 (2019).). This example demonstrates an alternative and novel approach to therapeutically target TOX in order to induce non-exhausted, highly functional CD8⁺ effector states. Indeed, it was shown that IL-2^{V} promoted both CD8 T-cell stemness and suppression of TOX not only in the transferred T cells, but also in the endogenous CD8⁺ T-cell intratumoral compartment. While IL-33, presumably indirectly through reprogramming of the TME, drove *Gzmc* upregulation, *Tcf1* suppression and consequently CD8⁺ T-cell differentiation to polyfunctional effector cells. This optimal TIL state was lost at tumor escape, and the resultant EM-like state was not only different from the effector *GzmC⁺* but also from the canonical effector-memory CD8 TIL state. Thus, tumor escape to orthogonal T-cell engineering was not mediated by reactivation of TOX-driven exhaustion program, but rather due to lack of intratumoral CD8⁺ T-cell differentiation towards the optimal *GzmC⁺* effector CD8⁺ state. This is a key difference with PD-1 blockade reinvigorated exhausted CD8⁺ T cells, which reacquire their exhausted phenotype during tumor escape.

In summary, this example shows that orthogonal combinatorial engineering of CD8⁺ T cells for ACT, specifically to secrete a variant of IL-2 that does not engage CD25 and the alarmin IL-33, can control advanced melanoma tumors in the absence of preconditioning, cytokine therapy or other support (*e.g*., vaccination). While the combinatorial T-cell therapy was non-curative, it was demonstrated that CD4 depletion enabled long-term survival, indicating that Tregs play a role in disease progression and thus offering opportunities for additional combinatorial interventions. Thus, this disclosure demonstrates the potential for clinical translation of combinatorial engineered T cells for reprogramming the TME and inducing highly functional CD8⁺ states endowed with the ability to control advanced, poorly immunogenic solid tumors.

**Table 2: Observed response and predicted probability of objective response and clinical benefit for each treatment group.**

| **Treatment Group** | **No of mice** | **Objective Response (CR/PR)** | | **Clinical Benefit (CR/PR/SD)** | |
|---|---|---|---|---|---|
| | | Observed n (%) | predicted probability (%) | Observed n (%) | predicted probability (%) |
| UT | 14 | 0 | 3.33% | 0 | 3.33% |
| UT + aPDL1 | 7 | 0 | 6.25% | 0 | 6.25% |
| PD1d | 6 | 0 | 7.14% | 0 | 7.14% |
| IL-2V | 4 | 0 | 10% | 0 | 10% |
| IL-33 | 5 | 0 | 8.33% | 1 (20.0) | 25% |
| PD1d + IL-2^{V} | 12 | 0 | 3.85% | 0 | 3.85% |
| PD1d + IL-33 | 12 | 1 (8.33%) | 11.5% | 4 (33.3) | 34.6% |
| PD1d + IL-2V + IL-33 | 14 | 12 (85.7%) | 83.3% | 12 (85.7) | 83.3% |

| | | | | | |
|---|---|---|---|---|---|
| Notes: Objective Response includes Complete Response (CR; 100% reduction in tumor volume) and Partial Response (PR; ≤-30% tumor change). Clinical Benefit includes CR, PR, and Stable Disease (-30%<tumor change≤+20%). Probability of occurrence was calculated using exact logistic regression. | | | | | |

**Table 3: Predicted tumor size change from baseline for each group using linear regression.**

| **Treatment** | **Predicted (average) (%) change in tumor size** | **95% CI of the expected (%) change in tumor size** | **p-value** |
|---|---|---|---|
| UT | *287.1%* | (190% , 384%) | <0.0001 |
| UT + aPDL1 | 274.1*%* | (136% , 412%) | 0.0002 |
| PD1d | 343.7*%* | (195% , 493%) | <0.0001 |
| IL-2^{V} | 608.0*%* | (426% , 790%) | <0.0001 |
| IL-33 | 145.7*%* | (-17% , 309%) | 0.0377 |
| PD1d + IL-2^{V} | 290.8*%* | (186% , 396%) | <0.0001 |
| PD1d + IL-33 | 83.17*%* | (-22% , 188%) | 0.0570 |
| PD1d + IL-2V + IL-33 | -56*%* | (-100%* , 41%) | - |

| | | | |
|---|---|---|---|
| *: -100% is the minimum plausible value for tumor size change. Notes: p-values compare each treatment effect versus the triple combination (PD1d + IL-2V + IL-33). Adjusted R² of the model: 43.8%. | | | |

### EXAMPLE 5

### CD40L Decoy and IL-2 variant and combination GEEP therapy

SIN retroviral vectors were constructed encoding a trimeric CD40L decoy as well as a variant of IL-2 that does not engage CD25. These molecules are expressed under NFAT and hence only produced in an activated T cell, which should only take place in the tumor microenvironment. It was shown in preclinical models that the IL-2 variant promotes a less differentiated phenotype and supports *in vivo* engraftment (*i.e.,* persistence of the T cells). In the preclinical studies, it was also shown that CD40L promotes tumor control. It can act on antigen-presenting cells such as dendritic cells to activate them and thereby provide better T-cell support. Hence, CD40L decoy is a tumor microenvironment re-programmer.

T cells are first engineered with PD1 decoy-tEGFR and then to combine, either by co-transduction or by mixing different engineered T cell populations, with the CD40L decoy and IL2^{V}. The tEGFR (or referred to as Cellular Elimination Tag (CET)) can be used as a means of evaluating transduction efficiency and for enriching the engineered cells (on anti-EGFR coated beads) if necessary. It can be used as a means of tracking the engineered T cells in a patient post-engraftment (via FACS from drawn blood samples or tumor biopsies). In addition, it can be used as an elimination tag via ADCC in the event of toxicity in a patient with Cetuximab.

**Table 4. Representative sequences of example transgenes**

| SEQ ID NO | SEQUENCE | NOTE |
|---|---|---|
| 1 | SGWLLEVPNGPWRSLTFYPAWLTVSEGANATFTCSLSNWSE DLMLNWNRLSPSNQTEKQAAFSNGLSQPVQDARFQIIQLPN RHDFHMNILDTRRNDSGIYLCGAISLHPKAKIEESPGAELVV TERILETSTRYPSPSPKPEGRFQ | N-terminal ectodomain of murine PD-1 (Uniprot ID: Q02242 residues S21-Q167, C83S) |
| 2 | MQIPQAPWPVVWAVLQLGWRPGWFLDSPDRPWNPPTFSPA LLVVTEGDNATFTCSFSNTSESFVLNWYRMSPSNQTDKLAA FPEDRSQPGQDCRFRVTQLPNGRDFHMSVVRARRNDSGTY LCGAISLAPKAQIKESLRAELRVTERRAEVPTAHPSPSPRPAG QFQTLVVGVVGGLLGSLVLLVWVLAVICSRAARGTIGARRT GQPLKEDPSAVPVFSVDYGELDFQWREKTPEPPVPCVPEQT EYATIVFPSGMGTSSPARRGSADGPRSAQPLRPEDGHCSWP L | Human PD1; UniProtKB/Sw iss-Prot: Q15116 (PDCD1_HU MAN) |
| 3 | PGWFLDSPDRPWNPPTFSPALLVVTEGDNATFTCSFSNTSES FVLNWYRMSPSNQTDKLAAFPEDRSQPGQDCRFRVTQLPN GRDFHMSVVRARRNDSGTYLCGAISLAPKAQIKESLRAELR VTERRAEVPTAHPSPSPRPAGQFQ | Human PD1 motif (21-167) |
| 4 | PGWFLDSPDRPWNPPTFSPALLVVTEGDNATFTCSFSNTSES FVLNWYRMSPSNQTDKLAAFPEDRSQPGQDCRFRVTQLPN GRDFHMSVVRARRNDSGTYLCGAISLAPKAQIKESLRAELR VTERRAEVPTAHPSPSPRPAGQFQPPCPSCPAPEFLGGPSVFL FPPKPKDTLMISRTPEVTCVVVDVSQEDPEVQFNWYVDGVE VHNAKTKPREEQFNSTYRVVSVLTVLHQDWLNGKEYKCK VSNKGLPSSIEKTISKAKGQPREPQVYTLPPSQEEMTKNQVS LTCLVKGFYPSDIAVEWESNGQPENNYKTTPPVLDSDGSFFL YSRLTVDKSRWQEGNVFSCSVMHEALHNHYTQKSLSLSPG K | Human PD1.IgG4 |
| 5 | CCTGGCTGGTTTCTGGACAGCCCTGACAGACCCTGGAATC CTCCAACATTCAGCCCCGCTCTGCTGGTGGTTACCGAGGG CGATAATGCCACCTTCACCTGTAGCTTCAGCAACACCAGC GAGAGCTTCGTGCTGAACTGGTACAGAATGAGCCCCAGC AACCAGACCGACAAGCTGGCCGCCTTTCCTGAGGATAGA TCTCAGCCCGGCCAGGACTGCCGGTTCAGAGTTACACAG CTGCCCAACGGCCGGGACTTCCACATGTCTGTCGTCCGGG CCAGAAGAAACGACAGCGGCACATATCTGTGCGGCGCCA TTTCTCTGGCCCCTAAGGCTCAGATCAAAGAGAGCCTGA GAGCCGAGCTGAGAGTGACAGAAAGACGGGCCGAAGTG CCCACAGCTCACCCTTCACCTTCTCCAAGACCTGCCGGCC AGTTTCAGCCTCCTTGTCCTAGCTGTCCTGCTCCTGAGTTT CTCGGCGGACCCTCCGTGTTCCTGTTTCCTCCAAAGCCTA AGGACACCCTGATGATCAGCAGAACCCCTGAAGTGACCT GCGTGGTGGTGGACGTGTCCCAAGAGGACCCTGAGGTGC AGTTCAATTGGTACGTGGACGGCGTGGAAGTGCACAACG CCAAGACCAAGCCTAGAGAGGAACAGTTCAACAGCACCT ACAGAGTGGTGTCCGTGCTGACCGTGCTGCACCAGGATT GGCTGAACGGCAAAGAGTACAAGTGCAAGGTGTCCAACA AGGGCCTGCCTAGCAGCATCGAGAAAACCATCAGCAAGG CCAAGGGCCAGCCAAGAGAACCCCAGGTGTACACACTGC CTCCAAGCCAAGAGGAAATGACCAAGAACCAGGTGTCCC TGACCTGCCTGGTCAAGGGCTTCTACCCTTCCGATATCGC CGTGGAATGGGAGAGCAATGGCCAGCCTGAGAACAACTA CAAGACCACACCTCCTGTGCTGGACAGCGACGGCTCATT CTTCCTGTACAGCAGACTGACCGTGGACAAGAGCAGATG GCAAGAGGGCAACGTGTTCAGCTGCAGCGTGATGCACGA GGCCCTGCACAACCACTACACCCAGAAGTCCCTGAGCCT GTCTCCTGGCAAA | Human PD1.IgG4 |
| 6 | MASDSPDRPWNPPTFSPALLVVTEGDNATFTCSFSNTSESFV LNWYRMSPSNQDDRLAAFPEDRSQPGQDARFRVTQLPNGR DFHMSVVRARRNDSGTYFCGAISLAPKAKIKESLRAELRVT ERRALE | PD1_4XMUT _M70 |
| 7 | MASDSPDRPWNPPTFSPALLVVTEGDNATFTCSFSNTSESFV LNWYRMSPSNQDDRLAAFPEDRSQPGQDARFRVTQLPNGR DFHMSVVRARRNDSGTYFCGAISLAPKAKIKESLRAELRVT ERRALEHHHHHH | PD1_4XMUT _M70 (with 6xHis) |
| 8 | DSPDRPWNPPTFSPALLVVTEGDNATFTCSFSNTSESFVLNW YRMSPSNQDDRLAAFPEDRSQPGQDARFRVTQLPNGRDFH MSVVRARRNDSGTYFCGAISLAPKAKIKESLRAELRVTERR AEVPTAHPSPSPRPAGQFQTPPCPSCPAPEFLGGPSVFLFPP KPKDTLMISRTPEVTCVVVDVSQEDPEVQFNWYVDGVE VHNAKTKPREEQFNSTYRVVSVLTVLHQDWLNGKEYKC KVSNKGLPSSIEKTISKAKGQPREPQVYTLPPSQEEMTKN QVSLTCLVKGFYPSDIAVEWESNGQPENNYKTTPPVLDS DGSFFLYSRLTVDKSRWQEGNVFSCSVMHEALHNHYTQ KSLSLSLGK | PD1_4XMUT _M701/IgG4Fc IgG4Fc is bolded and spacing residues are underlined |
| 9 | MASDSPDRPWNPPTFSPALLVVTEGDNATFTCSFSNTSESFV LNWYRMSPSNQVDRLAAFPEDRSQPGQDARFRVTQLPNGR DFHMSVVRARRNDSGTYFCGAISLAPKWKIKESLRAELRVT ERRALE | PD1_6XDM |
| 10 | MASDSPDRPWNPPTFSPALLVVTEGDNATFTCSFSNTSESFV LNWYRMSPSNQVDRLAAFPEDRSQPGQDARFRVTQLPNGR DFHMSVVRARRNDSGTYFCGAISLAPKWKIKESLRAELRVT ERRALEHHHHHH | PD1_6XDM (with 6xHis) |
| 11 | DSPDRPWNPPTFSPALLVVTEGDNATFTCSFSNTSESFVLNW YRMSPSNQVDRLAAFPEDRSQPGQDARFRVTQLPNGRDFH MSVVRARRNDSGTYFCGAISLAPKWKIKESLRAELRVTERR AEVPTAHPSPSPRPAGQFQTPPCPSCPAPEFLGGPSVFLFPP KPKDTLMISRTPEVTCVVVDVSQEDPEVQFNWYVDGVE VHNAKTKPREEQFNSTYRVVSVLTVLHQDWLNGKEYKC KVSNKGLPSSIEKTISKAKGQPREPQVYTLPPSQEEMTKN QVSLTCLVKGFYPSDIAVEWESNGQPENNYKTTPPVLDS DGSFFLYSRLTVDKSRWQEGNVFSCSVMHEALHNHYTQ KSLSLSLGK | PD1_6XDM/I gG4Fc IgG4Fc is bolded and spacing residues are underlined |
| 12 | MASDSPDRPWNPPTFSPALLVVTEGDNATFTCSFSNTSESFV LNWYRESPSNQDDRLAAFPEDRSQPGQDARFRVTQLPNGR DFHMSVVRARRNDSGTYFCGAISLAPKAKIKESLRAELRVT ERRALE | PD1_4XMUT |
| 13 | MASDSPDRPWNPPTFSPALLVVTEGDNATFTCSFSNTSESFV LNWYRESPSNQDDRLAAFPEDRSQPGQDARFRVTQLPNGR DFHMSVVRARRNDSGTYFCGAISLAPKAKIKESLRAELRVT ERRALEHHHHHH | PD1_4XMUT (with 6xHis) |
| 14 | MASDSPDRPWNPPTFSPALLVVTEGDNATFTCSFSNTSESFV LNWYRESPSNQDDRLAAFPEDRSQPGQDARFRVTQLPNGR DFHMSVVRARRNDSGTYFCGAISLAPKAKIKESLRAELRVT ERRAEVPTAHPSPSPRPAGQFQTPPCPSCPAPEFLGGPSVFL FPPKPKDTLMISRTPEVTCVVVDVSQEDPEVQFNWYVDG VEVHNAKTKPREEQFNSTYRVVSVLTVLHQDWLNGKEY KCKVSNKGLPSSIEKTISKAKGQPREPQVYTLPPSQEEMT KNQVSLTCLVKGFYPSDIAVEWESNGQPENNYKTTPPVL DSDGSFFLYSRLTVDKSRWQEGNVFSCSVMHEALHNHY TQKSLSLSLGK | PD1_4XMUT/ IgG4Fc IgG4Fc is bolded and spacing residues are underlined |
| 15 | MASDSPDRPWNPPTFSPALLVVTEGDNATFTCSFSNTSESFV LNWYRESPSNQVDRLAAFPEDRSQPGQDARFRVTQLPNGR DFHMSVVRARRNDSGTYFCGAISLAPKWKIKESLRAELRVT ERRALE | PD1_6XD |
| 16 | MASDSPDRPWNPPTFSPALLVVTEGDNATFTCSFSNTSESFV LNWYRESPSNQVDRLAAFPEDRSQPGQDARFRVTQLPNGR DFHMSVVRARRNDSGTYFCGAISLAPKWKIKESLRAELRVT ERRALEHHHHHH | PD1_6XD (with 6xHis) |
| 17 | MASDSPDRPWNPPTFSPALLVVTEGDNATFTCSFSNTSESFV LNWYRESPSNQVDRLAAFPEDRSQPGQDARFRVTQLPNGR DFHMSVVRARRNDSGTYFCGAISLAPKWKIKESLRAELRVT ERRAEVPTAHPSPSPRPAGQFQTPPCPSCPAPEFLGGPSVFL FPPKPKDTLMISRTPEVTCVVVDVSQEDPEVQFNWYVDG VEVHNAKTKPREEQFNSTYRVVSVLTVLHQDWLNGKEY KCKVSNKGLPSSIEKTISKAKGQPREPQVYTLPPSQEEMT KNQVSLTCLVKGFYPSDIAVEWESNGQPENNYKTTPPVL DSDGSFFLYSRLTVDKSRWQEGNVFSCSVMHEALHNHY TQKSLSLSLGK | PD1_6XD/IgG 4Fc IgG4Fc is bolded and spacing residues are underlined |
| 18 | PPCPSCPAPEFLGGPSVFLFPPKPKDTLMISRTPEVTCVVVDV SQEDPEVQFNWYVDGVEVHNAKTKPREEQFNSTYRVVSVL TVLHQDWLNGKEYKCKVSNKGLPSSIEKTISKAKGQPREPQ VYTLPPSQEEMTKNQVSLTCLVKGFYPSDIAVEWESNGQPE NNYKTTPPVLDSDGSFFLYSRLTVDKSRWQEGNVFSCSVM HEALHNHYTQKSLSLSPGK | Human IgG4 motif (104-327) |
| 19 | ASTKGPSVFPLAPCSRSTSESTAALGCLVKDYFPEPVTVSWN SGALTSGVHTFPAVLQSSGLYSLSSVVTVPSSSLGTKTYTCN VDHKPSNTKVDKRVESKYGPPCPSCPAPEFLGGPSVFLFPPK PKDTLMISRTPEVTCVVVDVSQEDPEVQFNWYVDGVEVHN AKTKPREEQFNSTYRVVSVLTVLHQDWLNGKEYKCKVSNK GLPSSIEKTISKAKGQPREPQVYTLPPSQEEMTKNQVSLTCL VKGFYPSDIAVEWESNGQPENNYKTTPPVLDSDGSFFLYSR LTVDKSRWQEGNVFSCSVMHEALHNHYTQKSLSLSLGK | human IgG4_Fc (Uniprot ID: P01861.1) |
| 20 | DVQMIQSPSSLSASLGDIVTMTCQASQGTSINLNWFQQKPG KAPKLLIYGASILEDGVPSRFSGSRYGTDFTLTISSLEDEDMA TYFCLQHSYLPYTFGGGTKLEIKR | murine IgG Kappa Chain region V-III MOPC 321; Uniprot ID: P01650 |
| 21 | MYRMQLLSCIALSLALVTNSAPTSSSTKKTQLQLEHLLLDLQ MILNGINNYKNPKLTRMLTFKFYMPKKATELKHLQCLEEEL KPLEEVLNLAQSKNFHLRPRDLISNINVIVLELKGSETTFMCE YADETATIVEFLNRWITFCQSIISTLT | Human Interleukin 2 (IL2); UniProtKB/Sw iss-Prot: P60568 (IL2_HUMAN ) |
| 22 | | Truncated IL-2 |
| 23 | APTSSSTKKTQLQLEHLLLDLQMILNGINNYKNPKLTAMLT AKFAMPKKATELKHLQCLEEALKPLEEVLNLAQSKNFHLRP RDLISNINVIVLELKGSETTFMCEYADETATIVEFLNRWITFS QSIISTLT | IL2V (R38A F42A Y45A E62A, C145S) |
| 24 | GCCCCTACCAGCAGCAGCACCAAAAAGACACAGCTGCAA CTGGAACACCTCCTGCTGGACCTGCAGATGATCCTGAAC GGCATCAACAACTACAAGAACCCCAAGCTGACCGCCATG CTGACAGCCAAGTTCGCCATGCCTAAGAAGGCCACCGAG CTGAAGCACCTCCAGTGCCTGGAAGAGGCCCTGAAGCCT CTGGAAGAAGTGCTGAATCTGGCCCAGAGCAAGAACTTC CACCTGAGGCCTAGGGACCTGATCAGCAACATCAACGTG ATCGTGCTGGAACTGAAGGGCAGCGAGACAACCTTCATG TGCGAGTACGCCGACGAGACAGCTACCATCGTGGAATTT CTGAACCGGTGGATCACCTTCAGCCAGAGCATCATCAGC ACCCTGACCTGA | IL2V (R38A F42A Y45A E62A); cDNA |
| 25 | SITGISPITEYLASLSTYNDQSITFALEDESYEIYVEDLKKDEK KDKVLLSYYESQHPSNESGDGVDGKMLMVTLSPTKDFWLH ANNKEHSVELHKCEKPLPDQAFFVLHNMHSNCVSFECKTD PGVFIGVKDNHLALIKVDSSENLCTENILFKLSET | Human Interleukin 33 (IL-33) motif (112-270) |
| 26 | AGCATCACCGGCATCAGCCCCATCACAGAGTATCTGGCC AGCCTGAGCACCTACAACGACCAGAGCATCACATTCGCC CTGGAAGATGAGAGCTACGAGATCTACGTGGAAGATCTG AAGAAGGACGAGAAGAAAGACAAGGTGCTGCTGAGCTA CTACGAGTCTCAGCACCCCAGCAATGAGTCTGGCGACGG CGTGGACGGAAAGATGCTGATGGTTACACTGAGCCCCAC CAAGGATTTCTGGCTGCACGCCAACAACAAAGAGCACAG CGTCGAGCTGCACAAGTGCGAGAAGCCTCTGCCTGACCA GGCCTTCTTCGTGCTGCACAACATGCACAGCAACTGCGTG TCCTTCGAGTGCAAGACCGATCCTGGCGTGTTCATCGGCG TGAAGGACAACCATCTGGCCCTGATCAAGGTGGACAGCA GCGAGAATCTGTGCACCGAGAACATCCTGTTCAAGCTGA GCGAGACA | Human IL-33 motif |
| 27 | SIQGTSLLTQSPASLSTYNDQSVSFVLENGCYVINVDDSGKD QEQDQVLLRYYESPCPASQSGDGVDGKKLMVNMSPIKDTDI WLHANDKDYSVELQRGDVSPPEQAFFVLHKKSSDFVSFEC KNLPGTYIGVKDNQLALVEEKDESCNNIMFKLSKI | murine IL-33 (Uniprot ID:Q8BVZ5.1, residues S109-I266 ) |
| 28 | MEESVVRPSVFVVDGQTDIPFTRLGRSHRRQSCSVARVGLG LLLLLMGAGLAVQGWFLLQLHWRLGEMVTRLPDGPAGSW EQLIQERRSHEVNPAAHLTGANSSLTGSGGPLLWETQLGLA FLRGLSYHDGALVVTKAGYYYIYSKVQLGGVGCPLGLASTI THGLYKRTPRYPEELELLVSQQSPCGRATSSSRVWWDSSFL GGVVHLEAGEKVVVRVLDERLVRLRDGTRSYFGAFMV | Human LIGHT (LIGHT); UniProtKB/Sw iss-Port: O43557 (TNF14_HUM AN) |
| 29 | DGPAGSWEQLIQERRSHEVNPAAHLTGANSSLTGSGGPLLW ETQLGLAFLRGLSYHDGALVVTKAGYYYIYSKVQLGGVGC PLGLASTITHGLYKRTPRYPEELELLVSQQSPCGRATSSSRV WWDSSFLGGVVHLEAGEKVVVRVLDERLVRLRDGTRSYFG AFMV | Human LIGHT motif (74-240) |
| 30 | GATGGACCTGCCGGATCTTGGGAGCAGCTGATCCAAGAG CGGAGAAGCCACGAAGTGAACCCTGCCGCACATCTGACA GGCGCCAATTCTAGCCTGACAGGCTCTGGTGGACCCCTGC TGTGGGAAACTCAACTGGGACTCGCCTTCCTGAGAGGCC TGAGCTATCATGATGGCGCCCTGGTGGTTACCAAGGCCG GCTACTACTACATCTACAGCAAGGTGCAGCTCGGCGGCG TGGGATGTCCTCTTGGACTGGCCTCTACAATCACCCACGG CCTGTACAAGCGGACCCCTAGATACCCCGAGGAACTGGA ACTGCTGGTGTCCCAGCAGAGCCCTTGTGGCAGAGCCAC AAGCAGCAGCAGAGTTTGGTGGGACAGCAGCTTTCTCGG CGGAGTGGTGCATCTGGAAGCCGGCGAAAAGGTGGTCGT CAGAGTGCTGGATGAGAGACTCGTGCGGCTGAGAGATGG CACCAGAAGCTACTTCGGCGCCTTCATGGTT | Human LIGHT motif; cDNA |
| 31 | DGGKGSWEKLIQDQRSHQANPAAHLTGANASLIGIGGPLLW ETRLGLAFLRGLTYHDGALVTMEPGYYYVYSKVQLSGVGC PQGLANGLPITHGLYKRTSRYPKELELLVSRRSPCGRANSSR VWWDSSFLGGVVHLEAGEEVVVRVPGNRLVRPRDGTRSYF GAFMV | murine LIGHT (Uniprot ID:Q9QYH9.1 , residues D72-V239) |
| 32 | | Human CD40 Ligand (CD40L); UniProtKB/Sw iss-Port: P29965 (CD40L_HUM AN) |
| 33 | MQRGDEDPQIAAHVVSEANSNAASVLQWAKKGYYTMKSN LVMLENGKQLTVKREGLYYVYTQVTFCSNREPSSQRPFIVG LWLKPSSGSERILLKAANTHSSSQLCEQQSVHLGGVFELQA GASVFVNVTEASQVIHRVGFSSFGLLKL | murine CD40L (Uniprot ID:P27548, residues M112-L260) |
| 34 | MQKGDQNPQIAAHVISEASSKTTSVLQWAEKGYYTMSNNL VTLENGKQLTVKRQGLYYIYAQVTFCSNREASSQAPFIASLC LKSPGRFERILLRAANTHSSAKPCGQQSIHLGGVFELQPGAS VFVNVTDPSQVSHGTGFTSFGLLKL | Human CD40 Ligand motif (113-261) |
| 35 | ATGCAGAAGGGCGACCAGAATCCTCAGATCGCCGCTCAC GTGATCAGCGAGGCCAGCAGCAAGACAACAAGCGTGCTG CAGTGGGCCGAGAAGGGCTACTACACCATGAGCAACAAC CTGGTCACCCTGGAAAACGGCAAGCAGCTGACCGTGAAG AGACAGGGCCTGTACTACATCTACGCCCAAGTGACCTTCT GCAGCAACAGAGAGGCCAGCTCTCAGGCCCCTTTTATCG CCAGCCTGTGCCTGAAGTCCCCTGGCAGATTCGAGCGGA TTCTGCTGAGAGCCGCCAACACACACAGCAGCGCCAAAC CTTGTGGCCAGCAGTCTATTCACCTCGGCGGAGTGTTTGA GCTGCAGCCTGGCGCAAGCGTGTTCGTGAATGTGACAGA CCCTAGCCAGGTGTCCCACGGCACCGGCTTTACATCTTTC GGCCTGCTGAAGCTG | Human CD40 Ligand motif; cDNA |
| 36 | MGTNKCLLQIALLLCFSTTALSRMKQIEDKIEEILSKIYHI ENEIARIKKLIGEVGGGSGGGSGGGSMQKGDQNPQIAAHV ISEASSKTTSVLQWAEKGYYTMSNNLVTLENGKQLTVKRQ GLYYIYAQVTFCSNREASSQAPFIASLCLKSPGRFERILLRAA NTHSSAKPCGQQSIHLGGVFELQPGASVFVNVTDPSQVSHG TGFTSFGLLKL | Zipper (bolded)-Linker (underlined)-Extracellular region of CD40L |
| 37 | ATGGGAACAAACAAATGCCTGCTGCAGATCGCCCTGCTG CTGTGCTTTAGCACAACAGCCCTGAGCCGGATGAAGCAG ATCGAGGACAAGATCGAAGAGATCCTGAGCAAGATCTAC CACATCGAGAACGAGATCGCCCGGATCAAGAAACTGATC GGCGAAGTCGGCGGAGGCTCTGGTGGTGGATCTGGCGGA GGATCTATGCAGAAAGGCGACCAGAATCCTCAGATCGCC GCTCACGTGATCAGCGAGGCCAGCAGCAAGACAACAAGC GTGCTGCAGTGGGCCGAGAAGGGCTACTACACCATGAGC AACAACCTGGTCACCCTGGAAAACGGCAAGCAGCTGACC GTGAAGAGACAGGGCCTGTACTACATCTACGCCCAAGTG ACCTTCTGCAGCAACAGAGAGGCCAGCTCTCAGGCCCCT TTTATCGCCAGCCTGTGCCTGAAGTCCCCTGGCAGATTCG AGCGGATTCTGCTGAGAGCCGCCAACACACACAGCAGCG CCAAACCTTGTGGCCAGCAGTCTATTCACCTCGGCGGAGT GTTTGAGCTGCAGCCTGGCGCAAGCGTGTTCGTGAATGTG ACAGACCCTAGCCAGGTGTCCCACGGCACCGGCTTTACA TCTTTCGGCCTGCTGAAGCTCTGA | Zipper-Linker-Extracellular region of CD40L |
| 38 | | CD40 ligand [Homo sapiens] Sequence ID: NP_000065.1 |
| 39 | MNNRWILHAAFLLCFSTTALSINYKQLQLQERTNIRKCQELL EQLNGKINLTYRADFKIPMEMTEKMQKSYTAFAIQEMLQN VFLVFRNNFSSTGWNETIVVRLLDELHQQTVFLKTVLEEKQ EERLTWEMSSTALHLKSYYWRVQRYLKLMKYNSYAWMV VRAEIFRNFLIIRRLTRNFQN | murine IFN-beta (Uniprot ID:P01575.1) |
| 40 | MLLLVTSLLLCELPHPAFLLIPRKVCNGIGIGEFKDSLSINAT NIKHFKNCTSISGDLHILPVAFRGDSFTHTPPLDPQELDILKT VKEITGFLLIQAWPENRTDLHAFENLEIIRGRTKQHGQFSLA VVSLNITSLGLRSLKEISDGDVIISGNKNLCYANTINWKKLFG TSGQKTKIISNRGENSCKATGQVCHALCSPEGCWGPEPRDC VSCRNVSRGRECVDKCNLLEGEPREFVENSECIQCHPECLPQ AMNITCTGRGPDNCIQCAHYIDGPHCVKTCPAGVMGENNT LVWKYADAGHVCHLCHPNCTYGCTGPGLEGCPTNGPKIPSI ATGMVGALLLLLVVALGIGLFM | tEGFR (286 amino acids) |
| 41 | ATGCTTCTGCTGGTTACATCTCTGCTGCTGTGCGAGCTGC CCCATCCTGCCTTTCTGCTGATCCCCAGAAAAGTGTGCAA CGGCATCGGCATCGGAGAGTTCAAGGACAGCCTGAGCAT CAACGCCACCAACATCAAGCACTTCAAGAACTGCACCAG CATCAGCGGCGACCTGCACATTCTGCCTGTGGCCTTTAGA GGCGACAGCTTCACCCACACACCTCCACTCGATCCCCAA GAGCTGGACATCCTGAAAACCGTGAAAGAGATCACCGGA TTTCTGTTGATCCAGGCTTGGCCCGAGAACCGGACAGATC TGCACGCCTTCGAGAACCTGGAAATCATCAGAGGCCGGA CCAAGCAGCACGGCCAGTTTTCTCTGGCTGTGGTGTCCCT GAACATCACCAGCCTGGGCCTGAGAAGCCTGAAAGAAAT CAGCGACGGCGACGTGATCATCTCCGGCAACAAGAACCT GTGCTACGCCAACACCATCAACTGGAAGAAGCTGTTCGG CACCAGCGGCCAGAAAACAAAGATCATCAGCAACCGGG GCGAGAACAGCTGCAAGGCTACAGGCCAAGTGTGCCACG CTCTGTGTAGCCCTGAAGGCTGTTGGGGACCCGAGCCTA GAGATTGCGTGTCCTGCAGAAACGTGTCCCGGGGCAGAG AATGCGTGGACAAGTGCAATCTGCTGGAAGGCGAGCCCC GCGAGTTCGTGGAAAACAGCGAGTGCATCCAGTGTCACC CCGAGTGTCTGCCCCAGGCCATGAACATTACCTGTACCGG CAGAGGCCCCGACAACTGTATTCAGTGCGCCCACTACAT CGACGGCCCTCACTGCGTGAAAACATGTCCTGCTGGCGT GATGGGAGAGAACAACACCCTCGTGTGGAAGTATGCCGA CGCCGGACATGTGTGCCACCTGTGTCACCCTAATTGCACC TACGGCTGTACAGGCCCTGGCCTGGAAGGCTGTCCAACA AACGGACCTAAGATCCCCTCTATCGCCACCGGCATGGTTG GAGCCCTGCTGCTGCTTCTGGTTGTGGCCCTTGGCATCGG CCTGTTTATGTAG | tEGFR |
| 42 | DSPDRPWNPPTFSPALLVVTEGDNATFTCSFSNTSESFVLNW YRMSPSNQDDRLAAFPEDRSQPGQDARFRVTQLPNGRDFH MSVVRARRNDSGTYFCGAISLAPKAKIKESLRAELRVTERR AEVPTAHPSPSPRPAGQFQTPPCPSCPAPEFLGGPSVFLFPP KPKDTLMISRTPEVTCVVVDVSQEDPEVQFNWYVDGVE VHNAKTKPREEQFNSTYRVVSVLTVLHQDWLNGKEYKC KVSNKGLPSSIEKTISKAKGQPREPQVYTLPPSQEEMTKN QVSLTCLVKGFYPSDIAVEWESNGQPENNYKTTPPVLDS DGSFFLYSRLTVDKSRWQEGNVFSCSVMHEALHNHYTQ KSLSLSLGKASRKRRSGSGEGRGSLLTCGDVEENPGPMLLL VTSLLLCELPHPAFLLIPRKVCNGIGIGEFKDSLSINATNIKHFKN CTSISGDLHILPVAFRGDSFTHTPPLDPQELDILKTVKEITGFLLI QAWPENRTDLHAFENLEIIRGRTKQHGQFSLAVVSLNITSLGLRS LKEISDGDVIISGNKNLCYANTINWKKLFGTSGQKTKIISNRGEN SCKATGQVCHALCSPEGCWGPEPRDCVSCRNVSRGRECVDKC NLLEGEPREFVENSECIQCHPECLPQAMNITCTGRGPDNCIQC AHYIDGPHCVKTCPAGVMGENNTLVWKYADAGHVCHLCHPN CTYGCTGPGLEGCPTNGPKIPSIATGMVGALLLLLVVALGIGLF M | PD1_4XMUT _M70_IgG4Fc /T2A/tEGFR IgG4Fc is bolded; furin cleavage site is double-underlined; T2A is underlined; and tEGFR is italic |
| 43 | GACAGCCCTGACAGACCCTGGAATCCTCCAACATTCAGC CCCGCTCTGCTGGTGGTTACCGAGGGCGATAATGCCACCT TCACCTGTAGCTTCAGCAACACCAGCGAGAGCTTCGTGCT GAACTGGTACAGAatGAGCCCCAGCAACCAGGACGACAG ACTGGCCGCCTTTCCTGAGGATAGATCTCAGCCCGGCCAG GACGCCCGGTTCAGAGTTACACAGCTGCCCAACGGCCGG GACTTCCACATGTCTGTCGTCCGGGCCAGAAGAAACGAC AGCGGCACATATTTTTGCGGCGCCATTTCTCTGGCCCCTA AGGCTAAAATCAAAGAGAGCCTGAGAGCCGAGCTGAGA GTGACAGAAAGACGGGCCGAAGTGCCCACAGCTCACCCT TCACCTTCTCCAAGACCTGCCGGCCAGTTTCAGACTCCTC CTTGTCCTAGCTGTCCTGCTCCTGAGTTTCTCGGCGG ACCCTCCGTGTTCCTGTTTCCTCCAAAGCCTAAGGACA CCCTGATGATCAGCAGAACCCCTGAAGTGACCTGCGT GGTGGTGGACGTGTCCCAAGAGGACCCTGAGGTGCA GTTCAATTGGTACGTGGACGGCGTGGAAGTGCACAAC GCCAAGACCAAGCCTAGAGAGGAACAGTTCAACAGCA CCTACAGAGTGGTGTCCGTGCTGACCGTGCTGCACCA GGATTGGCTGAACGGCAAAGAGTACAAGTGCAAGGTG TCCAACAAGGGCCTGCCTAGCAGCATCGAGAAAACCA TCAGCAAGGCCAAGGGCCAGCCAAGAGAACCCCAGGT GTACACACTGCCTCCAAGCCAAGAGGAAATGACCAAG AACCAGGTGTCCCTGACCTGCCTGGTCAAGGGCTTCT ACCCTTCCGATATCGCCGTGGAATGGGAGAGCAATGG CCAGCCTGAGAACAACTACAAGACCACACCTCCTGTG CTGGACAGCGACGGCTCATTCTTCCTGTACAGCAGAC TGACCGTGGACAAGAGCAGATGGCAAGAGGGCAACG TGTTCAGCTGCAGCGTGATGCACGAGGCCCTGCACAA CCACTACACCCAGAAGTCCCTGAGCCTGTCTCTGGGC AAAGCCAGCCGGAAGAGAAGATCTGGATCTGGCGAAGG CAGAGGCTCCCTGCTGACTTGCGGAGATGTGGAAGAGAA CCCCGGACCTATGCTTCTGCTGGTTACATCTCTGCTGCTGTG CGAGCTGCCCCATCCTGCCTTTCTGCTGATCCCCAGAAAAGT GTGCAACGGCATCGGCATCGGAGAGTTCAAGGACAGCCTGA GCATCAACGCCACCAACATCAAGCACTTCAAGAACTGCACCA GCATCAGCGGCGACCTGCACATTCTGCCTGTGGCCTTTAGAG GCGACAGCTTCACCCACACACCTCCACTCGATCCCCAAGAGC TGGACATCCTGAAAACCGTGAAAGAGATCACCGGATTTCTGT TGATCCAGGCTTGGCCCGAGAACCGGACAGATCTGCACGCC TTCGAGAACCTGGAAATCATCAGAGGCCGGACCAAGCAGCA CGGCCAGTTTTCTCTGGCTGTGGTGTCCCTGAACATCACCAG CCTGGGCCTGAGAAGCCTGAAAGAAATCAGCGACGGCGACG TGATCATCTCCGGCAACAAGAACCTGTGCTACGCCAACACCA TCAACTGGAAGAAGCTGTTCGGCACCAGCGGCCAGAAAACAA AGATCATCAGCAACCGGGGCGAGAACAGCTGCAAGGCTACA GGCCAAGTGTGCCACGCTCTGTGTAGCCCTGAAGGCTGTTG GGGACCCGAGCCTAGAGATTGCGTGTCCTGCAGAAACGTGT CCCGGGGCAGAGAATGCGTGGACAAGTGCAATCTGCTGGAA GGCGAGCCCCGCGAGTTCGTGGAAAACAGCGAGTGCATCCA GTGTCACCCCGAGTGTCTGCCCCAGGCCATGAACATTACCTG TACCGGCAGAGGCCCCGACAACTGTATTCAGTGCGCCCACT ACATCGACGGCCCTCACTGCGTGAAAACATGTCCTGCTGGC GTGATGGGAGAGAACAACACCCTCGTGTGGAAGTATGCCGA CGCCGGACATGTGTGCCACCTGTGTCACCCTAATTGCACCTA CGGCTGTACAGGCCCTGGCCTGGAAGGCTGTCCAACAAACG GACCTAAGATCCCCTCTATCGCCACCGGCATGGTTGGAGCC CTGCTGCTGCTTCTGGTTGTGGCCCTTGGCATCGGCCTGTTT ATGTAG | PD1_4XMUT _M70_IgG4Fc /T2A/tEGFR IgG4Fc is bolded; furin cleavage site is double-underlined; T2A is underlined; and tEGFR is italic |
| 44 | DSPDRPWNPPTFSPALLVVTEGDNATFTCSFSNTSESFVLNW YRMSPSNQVDRLAAFPEDRSQPGQDARFRVTQLPNGRDFH MSVVRARRNDSGTYFCGAISLAPKWKIKESLRAELRVTERR PPCPSCPAPEFLGGPSVFLFPPKPKDTLMISRTPEVTCVV VDVSQEDPEVQFNWYVDGVEVHNAKTKPREEQFNSTYR VVSVLTVLHQDWLNGKEYKCKVSNKGLPSSIEKTISKAK GQPREPQVYTLPPSQEEMTKNQVSLTCLVKGFYPSDIAV EWESNGQPENNYKTTPPVLDSDGSFFLYSRLTVDKSRWQ EGNVFSCSVMHEALHNHYTQKSLSLSLGKASRKRRSGSG EGRGSLLTCGDVEENPGPMLLLVTSLLLCELPHPAFLLIPRKVC NGIGIGEFKDSLSINATNIKHFKNCTSISGDLHILPVAFRGDSFTH TPPLDPQELDILKTVKEITGFLLIQAWPENRTDLHAFENLEIIRG RTKQHGQFSLAVVSLNITSLGLRSLKEISDGDVIISGNKNLCYAN TINWKKLFGTSGQKTKIISNRGENSCKATGQVCHALCSPEGCW GPEPRDCVSCRNVSRGRECVDKCNLLEGEPREFVENSECIQCH PECLPQAMNITCTGRGPDNCIQCAHYIDGPHCVKTCPAGVMG ENNTLVWKYADAGHVCHLCHPNCTYGCTGPGLEGCPTNGPKI PSIATGMVGALLLLLVVALGIGLFM | PD1_6XDM_I gG4Fc/T2A/tE GFR IgG4Fc is bolded; furin cleavage site is double-underlined; furin cleavage site is double-underlined; T2A is underlined; and tEGFR is italic |
| 45 | MLLLVTSLLLCELPHPAFLLIPTQVCTGTDMKLRLPASPETH LDMLRHLYQGCQVVQGNLELTYLPTNASLSFLQDIQEVQG YVLIAHNQVRQVPLQRLRIVRGTQLFEDNYALAVLDNGDPL NNTTPVTGASPGGLRELQLRSLTEILKGGVLIQRNPQLCYQD TILWKDIFHKNNQLALTLIDTNRSRACHPCSPMCKGSRCWG ESSEDCQSLTRTVCAGGCARCKGPLPTDCCHEQCAAGCTGP KHSDCLACLHFNHSGICELHCPALVTYNTDTFESMPNPEGR YTFGASCVTACPYNYLSTDVGSCTLVCPLHNQEVTAEDGTQ RCEKCSKPCARVCYGLGMEHLREVRAVTSANIQEFAGCKKI FGSLAFLPESFDGDPASNTAPLQPEQLQVFETLEEITGYLYIS AWPDSLPDLSVFQNLQVIRGRILHNGAYSLTLQGLGISWLGL RSLRELGSGLALIHHNTHLCFVHTVPWDQLFRNPHQALLHT ANRPEDECVGEGLACHQLCARGHCWGPGPTQCVNCSQFLR GQECVEECRVLQGLPREYVNARHCLPCHPECQPQNGSVTCF GPEADQCVACAHYKDPPFCVARCPSGVKPDLSYMPIWKFP DEEGACQPCPINCTHSCVDLDDKGCPAEQRASPLTSIISAVV GILLVVVLGVVFGILI | tHER2/ErbB2 (675 amino acids) |
| 46 | ATGCTTCTGCTGGTTACATCTCTGCTGCTGTGCGAGCTGC CCCATCCTGCCTTTCTGCTGATCCCCACACAAGTGTGCAC CGGCACCGACATGAAGCTGAGACTGCCTGCCTCTCCTGA GACACACCTGGACATGCTGAGACACCTGTACCAGGGCTG TCAGGTGGTGCAGGGCAATCTGGAACTGACCTACCTGCC TACCAACGCCAGCCTGAGCTTTCTGCAGGACATCCAAGA GGTGCAGGGATACGTGCTGATTGCCCACAATCAAGTGCG CCAGGTGCCACTGCAGCGGCTGAGAATCGTTAGAGGCAC CCAGCTGTTCGAGGACAACTATGCCCTGGCCGTGCTGGA CAATGGCGACCCTCTGAACAACACCACACCTGTGACAGG CGCTTCTCCTGGCGGACTGAGAGAACTGCAGCTGAGAAG CCTGACCGAGATCCTGAAAGGCGGCGTGCTGATCCAGAG AAACCCTCAGCTGTGCTACCAGGACACCATCCTGTGGAA GGACATCTTCCACAAGAACAACCAGCTGGCCCTGACACT GATCGACACCAACAGAAGCAGAGCCTGCCATCCTTGCAG CCCCATGTGCAAGGGCTCTAGATGTTGGGGCGAGAGCAG CGAGGATTGCCAGAGCCTGACCAGAACAGTGTGTGCCGG CGGATGTGCCAGATGCAAAGGACCTCTGCCTACCGACTG CTGCCACGAGCAATGTGCCGCTGGATGTACAGGCCCCAA GCACTCTGATTGCCTGGCCTGCCTGCACTTCAACCACTCT GGAATCTGCGAACTGCACTGCCCCGCTCTGGTCACCTACA ACACCGATACCTTCGAGAGCATGCCCAATCCTGAGGGCA GATACACCTTCGGCGCCAGCTGTGTGACAGCCTGTCCTTA CAACTACCTGAGCACCGACGTGGGCAGCTGTACCCTTGT GTGCCCTCTGCATAATCAAGAAGTGACCGCCGAGGACGG CACCCAGAGATGCGAGAAGTGTAGCAAGCCTTGCGCCAG AGTGTGTTACGGCCTCGGCATGGAACACCTGAGAGAAGT GCGGGCCGTGACCAGCGCCAATATCCAAGAATTTGCCGG CTGCAAGAAGATCTTTGGCAGCCTCGCCTTCCTGCCTGAG AGCTTCGATGGCGATCCTGCCAGCAATACTGCCCCTCTGC AGCCTGAACAGCTCCAGGTGTTCGAGACACTGGAAGAGA TCACCGGCTACCTGTATATCAGCGCCTGGCCAGACAGCCT GCCTGACCTGTCCGTGTTCCAGAACCTGCAAGTGATCCGG GGCAGAATCCTGCACAACGGCGCCTATTCTCTGACCCTGC AAGGCCTGGGAATCAGCTGGCTGGGACTGAGATCCCTGA GAGAGCTTGGATCTGGCCTGGCTCTGATCCACCACAATAC CCACCTGTGCTTCGTGCACACCGTGCCTTGGGACCAGCTG TTTCGGAATCCTCATCAGGCCCTGCTGCACACCGCCAACA GACCTGAGGATGAGTGTGTTGGCGAAGGCCTGGCTTGTC ACCAGCTCTGTGCTAGAGGACACTGTTGGGGCCCTGGAC CTACACAGTGCGTGAACTGTAGCCAGTTCCTGCGGGGCC AAGAGTGCGTGGAAGAGTGTAGAGTTCTGCAGGGACTGC CCCGGGAATACGTGAACGCCAGACACTGTCTGCCTTGTC ACCCTGAGTGCCAGCCTCAGAATGGCAGCGTGACCTGTTT TGGCCCTGAGGCCGATCAGTGCGTGGCCTGTGCTCACTAC AAGGACCCTCCATTCTGCGTGGCCAGATGTCCTAGCGGC GTGAAGCCTGATCTGAGCTACATGCCCATCTGGAAGTTCC CCGATGAGGAAGGCGCTTGCCAGCCTTGTCCTATCAACTG CACCCACAGCTGCGTGGACCTGGACGATAAGGGATGTCC AGCCGAGCAGAGAGCCTCTCCACTGACCTCTATCATCTCT GCCGTCGTGGGCATCCTGCTGGTGGTGGTTCTGGGCGTTG TGTTCGGCATCCTGATTTGA | tHER2/ErbB2 |
| 47 | DSPDRPWNPPTFSPALLVVTEGDNATFTCSFSNTSESFVLNW YRMSPSNQDDRLAAFPEDRSQPGQDARFRVTQLPNGRDFH MSVVRARRNDSGTYFCGAISLAPKAKIKESLRAELRVTERR AEVPTAHPSPSPRPAGQFQTPPCPSCPAPEFLGGPSVFLFPP KPKDTLMISRTPEVTCVVVDVSQEDPEVQFNWYVDGVE VHNAKTKPREEQFNSTYRVVSVLTVLHQDWLNGKEYKC KVSNKGLPSSIEKTISKAKGQPREPQVYTLPPSQEEMTKN QVSLTCLVKGFYPSDIAVEWESNGQPENNYKTTPPVLDS DGSFFLYSRLTVDKSRWQEGNVFSCSVMHEALHNHYTQ KSLSLSLGKASRKRRSGSGEGRGSLLTCGDVEENPGPMLLL VTSLLLCELPHPAFLLIPTQVCTGTDMKLRLPASPETHLDMLRH LYQGCQVVQGNLELTYLPTNASLSFLQDIQEVQGYVLIAHNQVR QVPLQRLRIVRGTQLFEDNYALAVLDNGDPLNNTTPVTGASPG GLRELQLRSLTEILKGGVLIQRNPQLCYQDTILWKDIFHKNNQL ALTLIDTNRSRACHPCSPMCKGSRCWGESSEDCQSLTRTVCAG GCARCKGPLPTDCCHEQCAAGCTGPKHSDCLACLHFNHSGIC ELHCPALVTYNTDTFESMPNPEGRYTFGASCVTACPYNYLSTDV GSCTLVCPLHNQEVTAEDGTQRCEKCSKPCARVCYGLGMEHL REVRAVTSANIQEFAGCKKIFGSLAFLPESFDGDPASNTAPLQP EQLQVFETLEEITGYLYISAWPDSLPDLSVFQNLQVIRGRILHNG AYSLTLQGLGISWLGLRSLRELGSGLALIHHNTHLCFVHTVPWD QLFRNPHQALLHTANRPEDECVGEGLACHQLCARGHCWGPG PTQCVNCSQFLRGQECVEECRVLQGLPREYVNARHCLPCHPEC QPQNGSVTCFGPEADQCVACAHYKDPPFCVARCPSGVKPDLS YMPIWKFPDEEGACQPCPINCTHSCVDLDDKGCPAEQRASPLT SIISAVVGILLVVVLGVVFGILI | PD 1 4XMUT _M70_IgG4Fc /T2A/tHER2 IgG4Fc is bolded; furin cleavage site is double-underlined; T2A is underlined; and tEGFR is italic |
| 48 | DSPDRPWNPPTFSPALLVVTEGDNATFTCSFSNTSESFVLNW YRMSPSNQVDRLAAFPEDRSQPGQDARFRVTQLPNGRDFH MSVVRARRNDSGTYFCGAISLAPKWKIKESLRAELRVTERR PPCPSCPAPEFLGGPSVFLFPPKPKDTLMISRTPEVTCVV VDVSQEDPEVQFNWYVDGVEVHNAKTKPREEQFNSTYR VVSVLTVLHQDWLNGKEYKCKVSNKGLPSSIEKTISKAK GQPREPQVYTLPPSQEEMTKNQVSLTCLVKGFYPSDIAV EWESNGQPENNYKTTPPVLDSDGSFFLYSRLTVDKSRWQ EGNVFSCSVMHEALHNHYTQKSLSLSLGKASRKRRSGSG EGRGSLLTCGDVEENPGPMLLLVTSLLLCELPHPAFLLIPTQVC TGTDMKLRLPASPETHLDMLRHLYQGCQVVQGNLELTYLPTNA SLSFLQDIQEVQGYVLIAHNQVRQVPLQRLRIVRGTQLFEDNYA LAVLDNGDPLNNTTPVTGASPGGLRELQLRSLTEILKGGVLIQR NPQLCYQDTILWKDIFHKNNQLALTLIDTNRSRACHPCSPMCK GSRCWGESSEDCQSLTRTVCAGGCARCKGPLPTDCCHEQCAA GCTGPKHSDCLACLHFNHSGICELHCPALVTYNTDTFESMPNP EGRYTFGASCVTACPYNYLSTDVGSCTLVCPLHNQEVTAEDGT QRCEKCSKPCARVCYGLGMEHLREVRAVTSANIQEFAGCKKIF GSLAFLPESFDGDPASNTAPLQPEQLQVFETLEEITGYLYISAWP DSLPDLSVFQNLQVIRGRILHNGAYSLTLQGLGISWLGLRSLREL GSGLALIHHNTHLCFVHTVPWDQLFRNPHQALLHTANRPEDEC VGEGLACHQLCARGHCWGPGPTQCVNCSQFLRGQECVEECR VLQGLPREYVNARHCLPCHPECQPQNGSVTCFGPEADQCVAC AHYKDPPFCVARCPSGVKPDLSYMPIWKFPDEEGACQPCPINC THSCVDLDDKGCPAEQRASPLTSIISAVVGILLVVVLGVVFGILI | PD1_6XDM_I gG4Fc/T2A/tH ER2 IgG4Fc is bolded; furin cleavage site is double-underlined; T2A is underlined; and tEGFR is italic |
| 49 | MTTPRNSVNGTFPAEPMKGPIAMQSGPKPLFRRMSSLVGPT QSFFMRESKTLGAVQIMNGLFHIALGGLLMIPAGIYAPICVT VWYPLWGGIMYIISGSLLAATEKNSRKCLVKGKMIMNSLSL FAAISGMILSIMDILNIKISHFLKMESLNFIRAHTPYINIYNCEP ANPSEKNSPSTQYCYSIQSLFLGILSVMLIFAFFQELVIAGIVE NEWKRTCSRPKSNIVLLSAEEKKEQTIEIKEEVVGLTETSSQP KNEEDIEIIPIQEEEEEETETNFPEPPQDQESSPIENDSSP | CD20 (297 amino acids) |
| 50 | ATGACAACACCTAGAAATAGCGTGAACGGCACATTCCCC GCCGAGCCTATGAAGGGACCTATCGCCATGCAGAGCGGC CCCAAGCCTCTGTTTAGACGGATGTCTAGCCTCGTGGGCC CCACACAGAGCTTTTTCATGAGAGAGAGCAAGACCCTGG GCGCCGTGCAGATCATGAACGGCCTGTTTCACATTGCCCT CGGCGGCCTGCTGATGATCCCTGCCGGAATCTATGCCCCT ATCTGCGTGACCGTGTGGTATCCTCTGTGGGGCGGCATCA TGTACATCATCTCTGGATCTCTGCTGGCCGCCACCGAGAA GAACAGCAGAAAGTGTCTGGTCAAGGGCAAGATGATCAT GAATAGCCTGAGCCTGTTCGCCGCCATCAGCGGCATGAT CCTGAGCATCATGGATATCCTGAATATCAAGATCAGCCA CTTCCTGAAGATGGAAAGCCTGAACTTCATCAGGGCCCA CACACCTTACATCAACATCTACAACTGCGAGCCCGCCAAT CCTAGCGAGAAGAATAGCCCCAGCACACAGTACTGCTAC TCTATCCAGAGCCTGTTTCTGGGCATCCTGAGCGTGATGC TGATCTTCGCATTCTTCCAAGAGCTGGTTATCGCCGGCAT CGTGGAAAACGAGTGGAAGCGGACCTGCAGCAGACCCA AGAGCAACATCGTGCTGCTGAGCGCCGAGGAAAAGAAA GAGCAGACCATCGAGATCAAAGAGGAAGTCGTCGGCCTG ACCGAGACAAGCAGCCAGCCTAAGAACGAAGAGGACAT TGAGATCATCCCCATCCAAGAAGAGGAAGAAGAAGAGA CTGAGACAAACTTCCCCGAGCCTCCTCAGGACCAAGAGA GCAGCCCCATTGAGAACGACAGCAGCCCTTGA | CD20 |
| 51 | DSPDRPWNPPTFSPALLVVTEGDNATFTCSFSNTSESFVLNW YRMSPSNQDDRLAAFPEDRSQPGQDARFRVTQLPNGRDFH MSVVRARRNDSGTYFCGAISLAPKAKIKESLRAELRVTERR AEVPTAHPSPSPRPAGQFQTPPCPSCPAPEFLGGPSVFLFPP KPKDTLMISRTPEVTCVVVDVSQEDPEVQFNWYVDGVE VHNAKTKPREEQFNSTYRVVSVLTVLHQDWLNGKEYKC KVSNKGLPSSIEKTISKAKGQPREPQVYTLPPSQEEMTKN QVSLTCLVKGFYPSDIAVEWESNGQPENNYKTTPPVLDS DGSFFLYSRLTVDKSRWQEGNVFSCSVMHEALHNHYTQ KSLSLSLGKASRKRRSGSGEGRGSLLTCGDVEENPGPMTTP RNSVNGTFPAEPMKGPIAMQSGPKPLFRRMSSLVGPTQSFFMR ESKTLGAVQIMNGLFHIALGGLLMIPAGIYAPICVTVWYPLWGGI MYIISGSLLAATEKNSRKCLVKGKMIMNSLSLFAAISGMILSIMDI LNIKISHFLKMESLNFIRAHTPYINIYNCEPANPSEKNSPSTQYCY SIQSLFLGILSVMLIFAFFQELVIAGIVENEWKRTCSRPKSNIVLLS AEEKKEQTIEIKEEVVGLTETSSQPKNEEDIEIIPIQEEEEEETET NFPEPPQDQESSPIENDSSP | PD1_4XMUT _M70_IgG4Fc /T2A/CD20 IgG4Fc is bolded; furin cleavage site is double-underlined; T2A is underlined; and tEGFR is italic |
| 52 | DSPDRPWNPPTFSPALLVVTEGDNATFTCSFSNTSESFVLNW YRMSPSNQDDRLAAFPEDRSQPGQDARFRVTQLPNGRDFH MSVVRARRNDSGTYFCGAISLAPKAKIKESLRAELRVTERR AEVPTAHPSPSPRPAGQFQTPPCPSCPAPEFLGGPSVFLFPP KPKDTLMISRTPEVTCVVVDVSQEDPEVQFNWYVDGVE VHNAKTKPREEQFNSTYRVVSVLTVLHQDWLNGKEYKC KVSNKGLPSSIEKTISKAKGQPREPQVYTLPPSQEEMTKN QVSLTCLVKGFYPSDIAVEWESNGQPENNYKTTPPVLDS DGSFFLYSRLTVDKSRWQEGNVFSCSVMHEALHNHYTQ KSLSLSLGKASRKRRSGSGEGRGSLLTCGDVEENPGPMTTP RNSVNGTFPAEPMKGPIAMQSGPKPLFRRMSSLVGPTQSFFMR ESKTLGAVQIMNGLFHIALGGLLMIPAGIYAPICVTVWYPLWGGI MYIISGSLLAATEKNSRKCLVKGKMIMNSLSLFAAISGMILSIMDI LNIKISHFLKMESLNFIRAHTPYINIYNCEPANPSEKNSPSTQYCY SIQSLFLGILSVMLIFAFFQELVIAGIVENEWKRTCSRPKSNIVLLS AEEKKEQTIEIKEEVVGLTETSSQPKNEEDIEIIPIQEEEEEETET NFPEPPQDQESSPIENDSSP | PD1_6XDM_I gG4Fc/T2A/C D20 IgG4Fc is bolded; furin cleavage site is double-underlined; T2A is underlined; and tEGFR is italic |

## Claims

1. A composition comprising a plurality of genetically-modified lymphocytes for targeting tumor cells in a subject, wherein the plurality of lymphocytes express at least three transgenes for modulating the immune system of the subject, and wherein the at least three transgenes comprise a PD-1-Fc decoy, an IL-2 variant having an amino acid sequence of any one of SEQ ID NOs: 21-23, and a CD40L having an amino acid of any one of SEQ ID NOs: 32-34, 36, and 38.

2. The composition of claim 1, wherein the PD-1-Fc decoy is a PD-1.IgG4 decoy.

3. The composition of claim 1 or 2, wherein the at least three transgenes further comprise a cellular elimination tag selected from the group consisting of truncated EGFR (tEGFR), HER2, a truncated HER2 (tHER2), CD20, and CD19.

4. The composition of claim 2 or 3, wherein the PD-1-Fc decoy comprises an amino acid sequence of any one of SEQ ID NOs: 1-4, 6-17, 42, 44, 47-48.

5. The composition of claim 3, wherein the tEGFR comprises the amino acid sequence of SEQ ID NO: 40; the HER2 or the tHER2 comprises the amino acid sequence of SEQ ID NO: 45; and the CD20 comprises the amino acid sequence of SEQ ID NO: 49.

6. The composition of any one of the preceding claims, wherein the plurality of lymphocytes comprises at least two subsets of lymphocytes.

7. The composition of claim 6, wherein the plurality of lymphocytes comprises: (i) a first subset expressing at least two transgenes; and (ii) a second subset expressing at least two transgenes, wherein at least one of the transgenes of the first subset is different from the transgenes of the second subset or wherein at least one of the transgenes of the first subset is in common with the transgenes of the second subset.

8. The composition of claim 7, wherein the first subset or the second subset further expresses tEGFR, tHER2, CD20, or CD19.

9. The composition of claim 7 or 8, wherein the two subsets are combined at a ratio from about 1:1 to about 1:100, preferably wherein the two subsets are combined at the ratio of about 1:1.

10. The composition of any one of the preceding claims, wherein the lymphocytes are autologous, preferably wherein the lymphocytes are tumor-infiltrating lymphocytes, preferably wherein the lymphocytes express a chimer antigen receptor (CAR), preferably wherein the lymphocytes express a recombinant T cell receptor (TCR), and preferably wherein the recombinant T cell receptor (TCR) shows reactivity against NY-ESO1, MAGE-A1, MAGE-A3, MAGE A-10, MAGE-C2, SSX2, MAGE-A12, or a combination thereof.

11. A pharmaceutical composition comprising an effective amount of the composition of any one of the preceding claims and a pharmaceutically acceptable carrier.

12. A composition according to any one of claims 1-9 for use in treating a cancer/tumor or chronic infection in a subject, preferably wherein the cancer is selected from the group consisting of melanoma, sarcoma, ovarian cancer, prostate cancer, lung cancer, bladder cancer, MSI-high tumors, head and neck tumors, kidney cancer, and breast cancer, preferably wherein the composition is administered by intravenous infusion, preferably wherein the composition further comprises a second therapeutic agent, and preferably wherein the second therapeutic agent is an anti-cancer or anti-tumor agent.

## Patentansprüche

1. Zusammensetzung, umfassend eine Vielzahl von genetisch modifizierten Lymphozyten zum Abzielen auf Tumorzellen in einem Subjekt, wobei die Vielzahl von Lymphozyten mindestens drei Transgene zum Modulieren des Immunsystems des Subjekts exprimiert und wobei die mindestens drei Transgene einen PD-1-Fc-Köder, eine IL-2-Variante mit einer Aminosäuresequenz einer beliebigen von SEQ ID NO: 21-23 und ein CD40L mit einer Aminosäure einer beliebigen von SEQ ID NO: 32-34, 36 und 38 umfassen.

2. Zusammensetzung nach Anspruch 1, wobei der PD-1-Fc-Köder ein PD-1.IgG4-Köder ist.

3. Zusammensetzung nach Anspruch 1 oder 2, wobei die mindestens drei Transgene ferner eine zelluläre Eliminierungsmarkierung umfassen, die aus der Gruppe ausgewählt ist, die aus verkürztem EGFR (tEGFR), HER2, einem verkürzten HER2 (tHER2), CD20 und CD19 besteht.

4. Zusammensetzung nach Anspruch 2 oder 3, wobei der PD-1-Fc-Köder eine Aminosäuresequenz einer beliebigen der SEQ ID NO: 1-4, 6-17, 42, 44, 47-48 umfasst.

5. Zusammensetzung nach Anspruch 3, wobei das tEGFR die Aminosäuresequenz von SEQ ID NO: 40 umfasst; das HER2 oder das tHER2 die Aminosäuresequenz von SEQ ID NO: 45 umfasst; und das CD20 die Aminosäuresequenz von SEQ ID NO: 49 umfasst.

6. Zusammensetzung nach einem der vorhergehenden Ansprüche, wobei die Vielzahl von Lymphozyten mindestens zwei Untergruppen von Lymphozyten umfasst.

7. Zusammensetzung nach Anspruch 6, wobei die Vielzahl von Lymphozyten Folgendes umfasst: (i) eine erste Untergruppe, die mindestens zwei Transgene exprimiert; und (ii) eine zweite Untergruppe, die mindestens zwei Transgene exprimiert, wobei mindestens eines der Transgene der ersten Untergruppe sich von den Transgenen der zweiten Untergruppe unterscheidet oder wobei mindestens eines der Transgene der ersten Untergruppe wie die Transgene der zweiten Untergruppe ist.

8. Zusammensetzung nach Anspruch 7, wobei die erste Untergruppe oder die zweite Untergruppe ferner tEGFR, tHER2, CD20 oder CD19 exprimiert.

9. Zusammensetzung nach Anspruch 7 oder 8, wobei die zwei Untergruppen in einem Verhältnis von etwa 1:1 bis etwa 1:100 kombiniert sind, wobei die zwei Untergruppen vorzugsweise im Verhältnis von etwa 1:1 kombiniert sind.

10. Zusammensetzung nach einem der vorhergehenden Ansprüche, wobei die Lymphozyten autolog sind, wobei vorzugsweise die Lymphozyten tumorinfiltrierende Lymphozyten sind, wobei vorzugsweise die Lymphozyten einen Chimerantigenrezeptor (CAR) exprimieren, wobei vorzugsweise die Lymphozyten einen rekombinanten T-Zellrezeptor (TCR) exprimieren und wobei vorzugsweise der rekombinante T-Zellrezeptor (TCR) Reaktivität gegen NY-ESO1, MAGE-A1, MAGE-A3, MAGE A-10, MAGE-C2, SSX2, MAGE-A12 oder eine Kombination davon zeigt.

11. Pharmazeutische Zusammensetzung, umfassend eine wirksame Menge der Zusammensetzung nach einem der vorhergehenden Ansprüche und einen pharmazeutisch unbedenklichen Träger.

12. Zusammensetzung gemäß einem der Ansprüche 1-9 zur Verwendung bei der Behandlung eines Krebses/Tumors oder einer chronischen Infektion bei einem Subjekt, wobei der Krebs vorzugsweise aus der Gruppe ausgewählt ist, die aus Melanom, Sarkom, Eierstockkrebs, Prostatakrebs, Lungenkrebs, Blasenkrebs, Tumoren mit hochgradiger MSI, Kopf- und Halstumoren, Nierenkrebs und Brustkrebs besteht, wobei die Zusammensetzung vorzugsweise durch intravenöse Infusion verabreicht wird, wobei die Zusammensetzung vorzugsweise ferner ein zweites therapeutisches Mittel umfasst und wobei das zweite therapeutische Mittel vorzugsweise ein Antikrebs- oder Antitumormittel ist.

## Revendications

1. Composition comprenant une pluralité de lymphocytes génétiquement modifiés pour cibler des cellules tumorales chez un sujet, dans laquelle la pluralité de lymphocytes exprime au moins trois transgènes pour moduler le système immunitaire du sujet, et dans laquelle les au moins trois transgènes comprennent un leurre PD-1-Fc, un variant d'IL-2 ayant une séquence d'acides aminés de l'une quelconque des SEQ ID N° : 21-23, et un CD40L ayant un acide aminé de l'une quelconque des SEQ ID N° : 32-34, 36 et 38.

2. Composition de la revendication 1, dans laquelle le leurre PD-1-Fc est un leurre PD-1.IgG4.

3. Composition de l'une des revendications 1 ou 2, dans laquelle les au moins trois transgènes comprennent en outre une étiquette d'élimination cellulaire choisie dans le groupe constitué d'EGFR tronqué (tEGFR), de HER2, d'un HER2 tronqué (tHER2), de CD20 et de CD19.

4. Composition de l'une des revendications 2 ou 3, dans laquelle le leurre PD-1-Fc comprend une séquence d'acides aminés de l'une quelconque des SEQ ID N° : 1-4, 6-17, 42, 44, 47-48.

5. Composition de la revendication 3, dans laquelle le tEGFR comprend la séquence d'acides aminés de SEQ ID N° : 40 ; le HER2 ou le tHER2 comprend la séquence d'acides aminés de SEQ ID N° : 45 ; et le CD20 comprend la séquence d'acides aminés de SEQ ID N° : 49.

6. Composition de l'une quelconque des revendications précédentes, dans laquelle la pluralité de lymphocytes comprend au moins deux sous-ensembles de lymphocytes.

7. Composition de la revendication 6, dans laquelle la pluralité de lymphocytes comprend : (i) un premier sous-ensemble exprimant au moins deux transgènes ; et (ii) un second sous-ensemble exprimant au moins deux transgènes, dans laquelle au moins l'un des transgènes du premier sous-ensemble est différent des transgènes du second sous-ensemble ou dans laquelle au moins l'un des transgènes du premier sous-ensemble est en commun avec les transgènes du second sous-ensemble.

8. Composition de la revendication 7, dans laquelle le premier sous-ensemble ou le second sous-ensemble exprime en outre tEGFR, tHER2, CD20 ou CD19.

9. Composition de l'une des revendications 7 ou 8, dans laquelle les deux sous-ensembles sont combinés à un rapport d'environ 1:1 à environ 1:100, de préférence dans laquelle les deux sous-ensembles sont combinés à un rapport d'environ 1:1.

10. Composition de l'une quelconque des revendications précédentes, dans laquelle les lymphocytes sont autologues, de préférence dans laquelle les lymphocytes sont des lymphocytes infiltrant une tumeur, de préférence dans laquelle les lymphocytes expriment un récepteur d'antigène chimérique (CAR), de préférence dans laquelle les lymphocytes expriment un récepteur des cellules T recombinant (TCR), et de préférence dans laquelle le récepteur des cellules T recombinant (TCR) présente une réactivité contre NY-ESO1, MAGE-A1, MAGE-A3, MAGE A-10, MAGE-C2, SSX2, MAGE-A12, ou une combinaison de ceux-ci.

11. Composition pharmaceutique comprenant une quantité efficace de la composition de l'une quelconque des revendications précédentes et un support pharmaceutiquement acceptable.

12. Composition selon l'une quelconque des revendications 1 à 9, destinée à être utilisée dans le traitement d'un cancer/d'une tumeur ou d'une infection chronique chez un sujet, de préférence dans laquelle le cancer est choisi dans le groupe constitué par le mélanome, le sarcome, le cancer de l'ovaire, le cancer de la prostate, le cancer du poumon, le cancer de la vessie, des tumeurs présentant une MSI élevée, des tumeurs de la tête et du cou, le cancer du rein et le cancer du sein, de préférence dans laquelle la composition est administrée par perfusion intraveineuse, de préférence dans laquelle la composition comprend en outre un second agent thérapeutique, et de préférence dans laquelle le second agent thérapeutique est un agent anticancéreux ou antitumoral.
